# EUROPEAN PATENT APPLICATION

(11) **EP 1 964 837 A1**
(43) Date of publication of application: **03.09.2008**
(21) Application number: 06833681.7
(22) Date of filing: 22.11.2006
(51) Int. Cl.: C07D 215/48, A61K 31/47, A61P 35/00, A61P 43/00, C12Q 1/02, G01N 33/68

(54) **ANTI-TUMOR AGENT FOR MULTIPLE MYELOMA**

(30) Priority: 22.11.2005 JP 2005337772; 30.05.2006 US 803450 P
(71) Applicant: Eisai R&D Management Co., Ltd., Tokyo 112-8088 (JP)
(72) Inventor: KAMATA, Junichi, Ibaraki 300-2635 (JP)
(74) Representative: Woods, Geoffrey Corlett
(86) International application number: PCT/JP2006/323878
(87) International publication number: WO 2007/061127

(57) **Abstract**

The object of the invention is to provide a pharmaceutical composition and a therapeutic method which can exert their effects with higher efficiency on a living organism having at least one cell selected from the group consisting of a cell overexpressing FGFR3, a cell that has a t(4;14) translocation and a cell expressing mutant FGFR3. The compound represented by General Formula (I), a pharmacologically acceptable salt thereof or a solvate thereof can exert their effects with higher efficiency on a living organism having at least one cell selected from the group consisting of a cell overexpressing FGFR3, a cell that has a t(4;14) translocation and a cell expressing mutant FGFR3.

## Description

### FIELD OF THE INVENTION

The present invention relates to a pharmaceutical composition comprising a compound represented by General Formula (I), a pharmacologically acceptable salt thereof or a solvate thereof (hereinafter, also referred to as a "compound of the invention") which are to be administered to a living organism having at least one cell selected from the group consisting of a cell overexpressing fibroblast growth factor receptor 3 (hereinafter, also referred to as "FGFR3"), a cell that has a t(4;14) translocation and a cell expressing mutant FGFR3, to a method for treating a disease comprising administering an effective amount of the compound of the invention to the living organism, to use of the compound of the invention for producing the pharmaceutical composition and to the compound of the invention for the pharmaceutical composition.

Moreover, the present invention relates to a therapeutic drug and a method comprising a compound of the invention for treating at least one disease selected from the group consisting of multiple myeloma, bladder cancer, cervical cancer, hypochondroplasia, achondroplasia, thanatophoric dysplasia (TD) and skeletal dysplasia, to use of the compound of the invention for producing the therapeutic drug and to the compound of the invention for the therapeutic drug.

Furthermore, the present invention relates to a FGFR3 inhibitor.

In addition, the present invention relates to a method for predicting the effect of the compound of the invention on a patient using at least one index selected from the group consisting of the FGFR3 expression level, the presence or the absence of a t(4; 14) translocation and the presence or the absence of FGFR3 mutation in the cell.

### BACKGROUND OF THE INVENTION

FGFR3 has three glycosylated domains, namely, an extracellular immunoglobulin-like domain, a transmembrane domain and an intracellular tyrosine kinase domain. Due to ligand stimulation, FGFR3 causes dimerization and autophosphorylation of tyrosine. FGFR3 is hardly expressed in B-cell line.

FGFR3 overexpression in a cell is known to play an important role in malignant alteration of multiple myeloma, bladder cancer, cervical cancer and the like⁽¹⁾.

In addition, a t(4;14) translocation has been found in about 10-20% of multiple myeloma⁽²⁾. The t(4;14) translocation has been reported to cause FGFR3 overexpression and activating mutation of FGFR3 at a constant frequency⁽²⁾.

On the other hand, FGFR3 mutations (Y373C, F384L, K650E and K650M) have been identified in a multiple myeloma patient. Activating mutations of FGFR3 have been reported to enhance malignant alteration of cancer⁽²⁻⁴⁾.

FGFR3 mutation has also been reported to play a central role in the early development of bladder cancer⁽¹¹⁾, and FGFR3 mutation has been reported in about 50% of papillary bladder cancer⁽¹²⁾.

FGFR3 mutation (S249C) has been found in cervical cancer⁽¹⁾.

Furthermore, FGFR3 mutation is known to cause hypochondroplasia, achondroplasia, thanatophoric dysplasia and skeletal dysplasia^{(5, 13)}.

Achondroplasia is considered to result from FGFR3 mutation (G380R)⁽¹⁵⁾.

SU5402 and PD173074, i.e., substances that inhibit a FGFR3 kinase activity (hereinafter, also referred to as "FGFR3 inhibitors") have been reported to cause cell growth inhibition and apoptosis in multiple myeloma cells overexpressing mutant FGFR3 (6, 7).

A FGFR3 inhibitor CHIR-258 has been reported to cause *in vitro* and *in vivo* cell growth inhibition in a multiple myeloma cell overexpressing wild-type FGFR3 and a multiple myeloma cell overexpressing mutant FGFR3⁽⁸⁾. CHIR-258 has also been reported to inhibit cellular viability stronger for a multiple myeloma cell overexpressing mutant FGFR3 than for a multiple myeloma cell that is not expressing FGFR3 or a multiple myeloma cell overexpressing wild-type FGFR3⁽⁸⁾.

A FGFR3 inhibitor PKC412 has been reported to inhibit viability of multiple myeloma cell strains (OPM-1, LP1, and KMS-11) in a cell viability assay⁽⁹⁾.

RNAi of FGFR3 has been reported to cause apoptosis of a multiple myeloma cell overexpressing mutant FGFR3⁽¹⁰⁾.

Thus, FGFR3 inhibitors are suggested to cause cell growth inhibition and apoptosis of at least one cell selected from the group consisting of a cell overexpressing FGFR3, a cell that has a t(4;14) translocation and a cell expressing mutant FGFR3, and to show an anti-tumor effect on a tumor comprising such cell.

FGFR3 inhibitors are also suggested to be effective against multiple myeloma⁽¹⁴⁾.

Moreover, FGFR3 inhibitors seem to be effective against hypochondroplasia, achondroplasia, thanatophoric dysplasia and skeletal dysplasia.

As antiangiogenic agents, 4-(3-chloro-4-(cyclopropylaminocarbonyl)aminophenoxy)-7-methoxy-6-quinolinecarbox amide and its analogous compounds are known⁽¹⁶⁻¹⁸⁾. However, it has never been reported that 4-(3-chloro-4-(cyclopropylaminocarbonyl)aminophenoxy)-7-methoxy-6-quinolinecarbox amide and its analogous compounds have a FGFR3-inhibiting activity.

### References

(1) Nature Genetics. 1999, 23, 18-20.
(2) Nature Genetics. 1997, 16, 260-264.
(3) Cell. 1994, 78, 335-342.
(4) Blood. 2001, 97, 729-736.
(5) Nature Genetics. 1996, 13, 233-237.
(6) British Journal ofHaematology. 2004, 124, 595-603.
(7) Blood. 2004, 103, 3521-3528.
(8) Blood. 2005, 105, 2941-2948.
(9) Oncogene. 2005, 24, 8259-8267.
(10) Molecular Cancer Therapeutics. 2005, 4, 787-798.
(11) Clinical Cancer Research. 2005, 11, 7709-7719.
(12) Clinical Cancer Research. 2005, 11, 7743-7748.
(13) Human Molecular Genetics. 2005, 14, 1153-1160.
(14) Blood. 2000, 95, 992-998.
(15) Nature. 1994, 371, 252-254.
(16) International publication No. 02/32872 (pamphlet)
(17) International publication No. 2004/080462 (pamphlet)
(18) International publication No. 2005/063713 (pamphlet)

### DISCLOSURE OF THE INVENTION

The present invention was achieved regarding the circumstances described above. The problem to be solved by the invention is to provide a pharmaceutical composition and a therapeutic method which can exert their effects with higher efficiency on a living organism having at least one cell selected from the group consisting of a cell overexpressing FGFR3, a cell that has a t(4;14) translocation and a cell expressing mutant FGFR3, and to provide a therapeutic drug and a method for treating at least one disease selected from the group consisting of multiple myeloma, bladder cancer, cervical cancer, hypochondroplasia, achondroplasia, thanatophoric dysplasia and skeletal dysplasia. In addition, the problem to be solved by the present invention is to provide a method for predicting an effect of a compound represented by General Formula (I), a pharmacologically acceptable salt thereof or a solvate thereof.

In order to solve the above problem, the present inventors have gone through keen examination, as a result of which they found that a compound represented by General Formula (I), a pharmacologically acceptable salt thereof or a solvate thereof has a FGFR3 kinase-inhibiting activity and found that the compound represented by General Formula (I), a pharmacologically acceptable salt thereof or a solvate thereof can exert their effects with higher efficiency on a living organism having at least one cell selected from the group consisting of a cell overexpressing FGFR3, a cell that has a t(4;14) translocation and a cell expressing mutant FGFR3. Furthermore, the compound represented by General Formula (I), a pharmacologically acceptable salt thereof or a solvate thereof was found to exert their effects with higher efficiency on at least one disease selected from the group consisting of multiple myeloma, bladder cancer, cervical cancer, hypochondroplasia, achondroplasia, thanatophoric dysplasia and skeletal dysplasia. The present inventors also found that the effect of the compound represented by General Formula (I), a pharmacologically acceptable salt thereof or a solvate thereof can be predicted by using at least one index selected from the group consisting of the FGFR3 expression level, the presence or the absence of a t(4;14) translocation and the presence or the absence of FGFR3 mutation in the cell.

Thus, the present invention relates to:
(1) A pharmaceutical composition comprising a compound represented by General Formula (I), a pharmacologically acceptable salt thereof or a solvate thereof, which is to be administered to a living organism having at least one cell selected from the group consisting of a cell overexpressing FGFR3, a cell that has a t(4;14) translocation and a cell expressing mutant FGFR3.
(2) A therapeutic drug for treating multiple myeloma comprising a compound represented by General Formula (I), a pharmacologically acceptable salt thereof or a solvate thereof.
(3) A therapeutic drug for treating at least one disease selected from the group consisting of bladder cancer, cervical cancer, hypochondroplasia, achondroplasia, thanatophoric dysplasia and skeletal dysplasia, the therapeutic drug comprising a compound represented by General Formula (I), a pharmacologically acceptable salt thereof or a solvate thereof.
(4) A method for treating a disease, comprising administering an effective amount of a compound represented by General Formula (I), a pharmacologically acceptable salt thereof or a solvate thereof to a living organism having at least one cell selected from the group consisting of a cell overexpressing FGFR3, a cell that has a t(4;14) translocation and a cell expressing mutant FGFR3.
(5) A method for treating multiple myeloma, comprising administering an effective amount of a compound represented by General Formula (I), a pharmacologically acceptable salt thereof or a solvate thereof to a patient.
(6) A method for treating at least one disease selected from the group consisting of bladder cancer, cervical cancer, hypochondroplasia, achondroplasia, thanatophoric dysplasia and skeletal dysplasia, the method comprising administering an effective amount of a compound represented by General Formula (I), a pharmacologically acceptable salt thereof or a solvate thereof to a patient.
(7) Use of a compound represented by General Formula (I), a pharmacologically acceptable salt thereof or a solvate thereof for producing a pharmaceutical composition which is to be administered to a living organism having at least one cell selected from the group consisting of a cell overexpressing FGFR3, a cell that has a t(4;14) translocation and a cell expressing mutant FGFR3.
(8) Use of a compound represented by General Formula (I), a pharmacologically acceptable salt thereof or a solvate thereof for producing a therapeutic drug for treating multiple myeloma.
(9) Use of a compound represented by General Formula (I), a pharmacologically acceptable salt thereof or a solvate thereof for producing a therapeutic drug for treating at least one disease selected from the group consisting of bladder cancer, cervical cancer, hypochondroplasia, achondroplasia, thanatophoric dysplasia and skeletal dysplasia.
(10) A compound represented by General Formula (I), a pharmacologically acceptable salt thereof or a solvate thereof for a pharmaceutical composition which is to be administered to a living organism having at least one cell selected from the group consisting of a cell overexpressing FGFR3, a cell that has a t(4;14) translocation and a cell expressing mutant FGFR3.
(11) A compound represented by General Formula (I), a pharmacologically acceptable salt thereof or a solvate thereof for a therapeutic drug for treating multiple myeloma.
(12) A compound represented by General Formula (I), a pharmacologically acceptable salt thereof or a solvate thereof for a therapeutic drug for treating at least one disease selected from the group consisting of bladder cancer, cervical cancer, hypochondroplasia, achondroplasia, thanatophoric dysplasia and skeletal dysplasia.
(13) A method for predicting whether or not a patient is highly sensitive to a compound represented by General Formula (I), a pharmacologically acceptable salt thereof or a solvate thereof, the method comprising using at least one index selected from the group consisting of the FGFR3 expression level, the presence or the absence of a t(4;14) translocation and the presence or the absence of FGFR3 mutation in the cell.
(14) A method for analyzing sensitivity of a cell to a compound represented by General Formula (I), a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell.
(15) A method for selecting a cell highly sensitive to a compound represented by General Formula (I), a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell.
(16) A method for selecting a patient highly sensitive to a compound represented by General Formula (I), a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell.
(17) A method for classifying a patient comprising determining at least one selected from the group consisting of the FGFR3 expression level, the presence or the absence of a t(4;14) translocation and the presence or the absence of FGFR3 mutation in the cell to analyze sensitivity to a compound represented by General Formula (I), a pharmacologically acceptable salt thereof or a solvate thereof, and classifying the patient according to the result.
(18) A method for selecting a patient for administering a compound represented by General Formula (I), a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell, and selecting a patient having at least one cell selected from the group consisting of a cell overexpressing FGFR3, a cell that has a t(4;14) translocation and a cell expressing mutant FGFR3 based on the determination results.
(19) A method for predicting a therapeutic effect of a compound represented by General Formula (I), a pharmacologically acceptable salt thereof or a solvate thereof on a patient, the method comprising determining at least one selected from the group consisting of the FGFR3 expression level, the presence or the absence of a t(4;14) translocation and the presence or the absence of FGFR3 mutation in the cell.
(20) A method for determining at least one selected from the group consisting of the FGFR3 expression level, the presence or the absence of a t(4;14) translocation and the presence or the absence of FGFR3 mutation in the cell from a patient for predicting a sensitivity level of the patient to a compound represented by General Formula (I), a pharmacologically acceptable salt thereof or a solvate thereof.
   The compound represented by General Formula (I), a pharmacologically acceptable salt thereof or a solvate thereof is as follows: 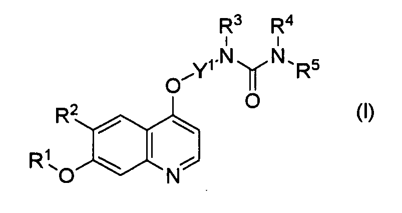 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
   R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
   Y¹ represents group represented by Formula 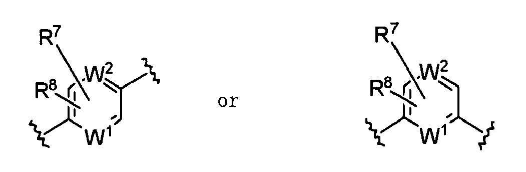 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
   W¹ and W² each independently represent a carbon atom or a nitrogen atom that may have a substituent);
   R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
   R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent],
   a pharmacologically acceptable salt thereof, or a solvate thereof.
   The present invention also relates to the followings.
(21) A FGFR3 inhibitor comprising the compound represented by General Formula (I), a pharmacologically acceptable salt thereof or a solvate thereof.
   Preferably, the present invention also relates to the followings.
(22) A pharmaceutical composition which is to be administered to a living organism having at least one cell selected from the group consisting of a cell overexpressing FGFR3, a cell that has a t(4;14) translocation and a cell expressing mutant FGFR3, the composition comprising 4-(3-chloro-4-(cyclopropylaminocarbonyl)aminophenoxy)-7-methoxy-6-quinolinecarbox amide, a pharmacologically acceptable salt thereof or a solvate thereof.
(23) A therapeutic drug for treating multiple myeloma, comprising 4-(3-chloro-4-(cyclopropylaminocarbonyl)aminophenoxy)-7-methoxy-6-quinolinecarbox amide, a pharmacologically acceptable salt thereof or a solvate thereof.
(24) A therapeutic drug for treating at least one disease selected from the group consisting of bladder cancer, cervical cancer, hypochondroplasia, achondroplasia, thanatophoric dysplasia and skeletal dysplasia, the therapeutic drug comprising 4-(3-chloro-4-(cyclopropylaminocarbonyl)aminophenoxy)-7-methoxy-6-quinolinecarbox amide, a pharmacologically acceptable salt thereof or a solvate thereof.
(25) A method for treating a disease, comprising administering an effective amount of 4-(3-chloro-4-(cyclopropylaminocarbonyl)aminophenoxy)-7-methoxy-6-quinolinecarbox amide, a pharmacologically acceptable salt thereof or a solvate thereof to a living organism having at least one cell selected from the group consisting of a cell overexpressing FGFR3, a cell that has a t(4;14) translocation and a cell expressing mutant FGFR3.
(26) A method for treating multiple myeloma comprising administering an effective amount of 4-(3-chloro-4-(cyclopropylaminocarbonyl)aminophenoxy)-7-methoxy-6-quinolinecarbox amide, a pharmacologically acceptable salt thereof or a solvate thereof to a patient.
(27) A method for treating at least one disease selected from the group consisting of bladder cancer, cervical cancer, hypochondroplasia, achondroplasia, thanatophoric dysplasia and skeletal dysplasia, the method comprising administering an effective amount of 4-(3-chloro-4-(cyclopropylaminocarbonyl)aminophenoxy)-7-methoxy-6-quinolinecarbox amide, a pharmacologically acceptable salt thereof or a solvate thereof to a patient.
(28) Use of 4-(3-chloro-4-(cyclopropylaminocarbonyl)aminophenoxy)-7-methoxy-6-quinolinecarbox amide, a pharmacologically acceptable salt thereof or a solvate thereof for producing a pharmaceutical composition which is to be administered to a living organism having at least one cell selected from the group consisting of a cell overexpressing FGFR3, a cell that has a t(4;14) translocation and a cell expressing mutant FGFR3.
(29) Use of 4-(3-chloro-4-(cyclopropylaminocarbonyl)aminophenoxy)-7-methoxy-6-quinolinecarbox amide, a pharmacologically acceptable salt thereof or a solvate thereof for producing a therapeutic drug for treating multiple myeloma.
(30) Use of 4-(3-chloro-4-(cyclopropylaminocarbonyl)aminophenoxy)-7-methoxy-6-quinolinecarbox amide, a pharmacologically acceptable salt thereof or a solvate thereof for producing a therapeutic drug for treating at least one disease selected from the group consisting of bladder cancer, cervical cancer, hypochondroplasia, achondroplasia, thanatophoric dysplasia and skeletal dysplasia.
(31) 4-(3-chloro-4-(cyclopropylaminocarbonyl)aminophenoxy)-7-methoxy-6-quinolinecarbox amide, a pharmacologically acceptable salt thereof or a solvate thereof for a pharmaceutical composition which is to be administered to a living organism having at least one cell selected from the group consisting of a cell overexpressing FGFR3, a cell that has a t(4;14) translocation and a cell expressing mutant FGFR3.
(32) 4-(3-chloro-4-(cyclopropylaminocarbonyl)aminophenoxy)-7-methoxy-6-quinolinecarbox amide, a pharmacologically acceptable salt thereof or a solvate thereof for a therapeutic drug for treating multiple myeloma.
(33) 4-(3-chloro-4-(cyclopropylaminocarbonyl)aminophenoxy)-7-methoxy-6-quinolinecarbox amide, a pharmacologically acceptable salt thereof or a solvate thereof for a therapeutic drug for treating at least one disease selected from the group consisting of bladder cancer, cervical cancer, hypochondroplasia, achondroplasia, thanatophoric dysplasia and skeletal dysplasia.
(34) A method for predicting whether or not a patient is highly sensitive to 4-(3-chloro-4-(cyclopropylaminocarbonyl)aminophenoxy)-7-methoxy-6-quinolinecarbox amide, a pharmacologically acceptable salt thereof or a solvate thereof, the method comprising using at least one index selected from the group consisting of the FGFR3 expression level, the presence or the absence of a t(4;14) translocation and the presence or the absence of FGFR3 mutation in the cell.
(35) A method for analyzing sensitivity of a cell to 4-(3-chloro-4-(cyclopropylaminocarbonyl)aminophenoxy)-7-methoxy-6-quinolinecarbox amide, a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell.
(36) A method for selecting a cell highly sensitive to 4-(3-chloro-4-(cyclopropylaminocarbonyl)aminophenoxy)-7-methoxy-6-quinolinecarbox amide, a pharmacologically acceptable salt thereof or a solvate thereof, the method comprising determining at least one selected from the group consisting of the FGFR3 expression level, the presence or the absence of a t(4; 14) translocation and the presence or the absence of FGFR3 mutation in the cell.
(37) A method for selecting a patient highly sensitive to 4-(3-chloro-4-(cyclopropylaminocarbonyl)aminophenoxy)-7-methoxy-6-quinolinecarbox amide, a pharmacologically acceptable salt thereof or a solvate thereof, the method comprising determining at least one selected from the group consisting of the FGFR3 expression level, the presence or the absence of a t(4; 14) translocation and the presence or the absence of FGFR3 mutation in the cell.
(38) A method for classifying a patient, comprising determining at least one selected from the group consisting of the FGFR3 expression level, the presence or the absence of a t(4;14) translocation and the presence or the absence of FGFR3 mutation in the cell to analyze its sensitivity to 4-(3-chloro-4-(cyclopropylaminocarbonyl)aminophenoxy)-7-methoxy-6-quinolinecarbox amide, a pharmacologically acceptable salt thereof or a solvate thereof according to the obtained results.
(39) A method for selecting a patient for administering 4-(3-chloro-4-(cyclopropylaminocarbonyl)aminophenoxy)-7-methoxy-6-quinolinecarbox amide, a pharmacologically acceptable salt thereof or a solvate thereof, the method comprising determining at least one selected from the group consisting of the FGFR3 expression level, the presence or the absence of a t(4;14) translocation and the presence or the absence of FGFR3 mutation in the cell, and selecting a patient having at least one cell selected from the group consisting of a cell overexpressing FGFR3, a cell that has a t(4;14) translocation and a cell expressing mutant FGFR3 from the obtained determination results.
(40) A method for predicting a therapeutic effect of 4-(3-chloro-4-(cyclopropylaminocarbonyl)aminophenoxy)-7-methoxy-6-quinolinecarbox amide, a pharmacologically acceptable salt thereof or a solvate thereof on a patient, the method comprising determining at least one selected from the group consisting of the FGFR3 expression level, the presence or the absence of a t(4;14) translocation and the presence or the absence of FGFR3 mutation in the cell.
(41) A method for determining at least one selected from the group consisting of the FGFR3 expression level, the presence or the absence of a t(4;14) translocation and the presence or the absence of FGFR3 mutation in the cell from a patient for predicting a sensitivity level of the patient to 4-(3-chloro-4-(cyclopropylaminocarbonyl)aminophenoxy)-7-methoxy-6-quinolinecarbox amide, a pharmacologically acceptable salt thereof or a solvate thereof.
(42) A FGFR3 inhibitor comprising 4-(3-chloro-4-(cyclopropylaminocarbonyl)aminophenoxy)-7-methoxy-6-quinolinecarbox amide, a pharmacologically acceptable salt thereof or a solvate thereof.

According to the present invention, a pharmaceutical composition and a therapeutic method are provided which can exert their effects with higher efficiency on a living organism having at least one cell selected from the group consisting of a cell overexpressing FGFR3, a cell that has a t(4;14) translocation and a cell expressing mutant FGFR3.

Specifically, the present invention provides: a pharmaceutical composition comprising a compound of the invention which is to be administered to a living organism having at least one cell selected from the group consisting of a cell overexpressing FGFR3, a cell that has a t(4;14) translocation and a cell expressing mutant FGFR3; a method for treating a disease comprising administering an effective amount of the compound of the invention to the living organism; use of the compound of the invention for producing the pharmaceutical composition; and the compound of the invention for the pharmaceutical composition.

The present invention also provides: a therapeutic drug comprising a compound of the invention and a method for treating at least one disease selected from the group consisting of multiple myeloma, bladder cancer, cervical cancer, hypochondroplasia, achondroplasia, thanatophoric dysplasia and skeletal dysplasia; use of the compound of the invention for producing the therapeutic drug; and the compound of the invention for the therapeutic drug.

The present invention also provides a FGFR3 inhibitor.

Furthermore, the present invention provides a method for predicting an effect of the compound of the invention.

More specifically, the effect of the compound of the invention can be predicted by using at least one index selected from the group consisting of the FGFR3 expression level, the presence or the absence of a t(4; 14) translocation and the presence or the absence of FGFR3 mutation in the cell.

According to the method of the invention, an effect of a compound can be predicted by selecting a patient who is expected to be more sensitive to the compound without administering the compound to the patient, thereby contributing to the QOL of the patient.

### BEST MODES FOR CARRYING OUT THE INVENTION

Hereinafter, embodiments of the present invention will be described. The following embodiments illustrate the present invention, which are not intended to limit the present invention. The present invention may be carried out in various embodiments without departing from the spirits of the invention.

The documents, laid-open patent applications, patent publications and other patent documents cited herein are incorporated herein by reference. The present specification also incorporates the disclosures of Japanese Patent Application No. 2005-337772 and US provisional application US60/803,450 based on which the present application claims priority.

### 1. Pharmaceutical composition, therapeutic drug and therapeutic method of the invention

### (1) FGFR3

According to the present invention, FGFR3 comprises a polypeptide having an amino acid sequence identical or substantially identical to the amino acids 23-806 (SEQ ID NO: 3) of the amino acid sequence represented by SEQ ID NO: 2 (GenBank Accession No: NM_000142). The polypeptide having the amino acid sequence represented by SEQ ID NO: 3 is generally processed and produced from a polypeptide having the amino acid sequence represented by SEQ ID NO: 2.

An example of the polypeptide having an amino acid sequence identical to the amino acid sequence represented by SEQ ID NO: 3 includes a polypeptide coded by polynucleotides having nucleotides 106-2460 of the nucleotide sequence represented by SEQ ID NO: 1 (GenBank Accession No: NM_000142).

An example of the polypeptide having an amino acid sequence substantially identical to the amino acid sequence represented by SEQ ID NO: 3 includes one selected from the group consisting of (a)-(d) below:
(a) a polypeptide including the amino acid sequence represented by SEQ ID NO: 3;
(b) a polypeptide that includes an amino acid sequence in which one or more (e.g., one or several) amino acids have been deleted, substituted, added or varied by any combination thereof in the amino acid sequence represented by SEQ ID NO: 3, and that has substantially the same activity as FGFR3;
(c) a polypeptide that is coded by a polynucleotide that hybridizes with a polynucleotide having a nucleotide sequence complementary to the nucleotide sequence represented by SEQ ID NO: 1 (nucleotides 106-2460) under stringent conditions, and that has substantially the same activity as FGFR3; and
(d) a polypeptide that has an amino acid sequence having 90% or higher, preferably about 95% or higher, more preferably about 98% or higher identity to (also phrased as "homology with") the amino acid sequence represented by SEQ ID NO: 3, and that has substantially the same activity as FGFR3.

Herein, the phrase "having substantially the same activity as FGFR3" means that at least one intracellular signal resulting from ligand (e.g., FGF, etc.) binding is identical to a signal of a protein having the amino acid sequence represented by SEQ ID NO: 3, and that the activation level of the intracellular signal is comparable with that of the protein having the amino acid sequence represented by SEQ ID NO: 3. Furthermore, the phrase "comparable with" means, for example, that the activation level of an intracellular signal resulting from ligand (e.g., FGF, etc.) binding has an activation level of 10% or higher, preferably 30% or higher of the activation level of an intracellular signal of a protein having the amino acid sequence represented by SEQ ID NO: 3. In this case, they are considered to have substantially the same activities. Examples of intracellular signals resulting from ligand binding include FGFR3 phosphorylation, Raf, MEK, ERK1 and ERK2 phosphorylations resulting from FGFR3 phosphorylation (Blood. 2001, 97, 729-736.), phosphatidylinositol 3 kinase phosphorylation, Akt phosphorylation, phospholipase C-γ phosphorylation, increase in inositol 1,4,5-trisphosphate (IP3) and increase in diacylglycerol (DAG).

Activity of an intracellular signal resulting from ligand binding can be determined by a conventional method such as immunoprecipitation and western blotting.

Examples of the polypeptide having an amino acid sequence where one or more (e.g., one or several) amino acids are deleted, substituted, added or varied by any combination thereof in the amino acid sequence represented by SEQ ID NO: 3 include polypeptides having:
(i) an amino acid sequence having 1-9 (e.g., 1-5, preferably 1-3, more preferably 1-2, still more preferably one) amino acids deleted from the amino acid sequence represented by SEQ ID NO: 3;
(ii) an amino acid sequence having 1-9 (e.g., 1-5, preferably 1-3, more preferably 1-2, still more preferably one) amino acids added to the amino acid sequence represented by SEQ ID NO: 3;
(iii) an amino acid sequence having 1-9 (e.g., 1-5, preferably 1-3, more preferably1-2, still more preferably one) amino acids in the amino acid sequence represented by SEQ ID NO: 3 substituted with other amino acids; or
(iv) an amino acid sequence varied by any combination of (i)-(iii) above.

Herein, "deletion" of an amino acid refers to mutation where one or more amino acid residues are deleted from the sequence, which includes the case where the amino acid residues are deleted from the end of the amino acid sequence and the case where the amino acid residues are deleted in the middle of the amino acid sequence.

Herein, "addition" of an amino acid refers to mutation where one or more amino acid residues are added to the sequence, which include the case where the amino acid residues are added to the end of the amino acid sequence and the case where the amino acid residues are added to the middle of the amino acid sequence. The latter case may also be referred to as "insertion".

Herein, "substitution" of an amino acid refers to mutation where one or more amino acid residues in the sequence are substituted with different types of amino acid residues. When the amino acid sequence of FGFR3 is to be modified by such substitution, it is preferably a conservative substitution in order to maintain the function of the protein. Conservative substitution means to modify the sequence such that the modified sequence codes for amino acids having similar nature to the unsubstituted amino acids. The natures of amino acids may be classified, for example, into non-polar amino acids (Ala, Ile, Leu, Met, Phe, Pro, Trp, Val), uncharged amino acids (Asn, Cys, Gln, Gly, Ser, Thr, Tyr), acidic amino acids (Asp, Glu), basic amino acids (Arg, His, Lys), neutral amino acids (Ala, Asn, Cys, Gln, Gly, Ile, Leu, Met, Phe, Pro, Ser, Thr, Trp, Tyr, Val), aliphatic amino acids (Ala, Gly), branched amino acids (Ile, Leu, Val), hydroxyamino acids (Ser, Thr), amide type amino acids (Gln, Asn), sulfur-containing amino acids (Cys, Met), aromatic amino acids (His, Phe, Trp, Tyr), heterocyclic amino acids (His, Trp), imino acids (Pro, 4Hyp) or the like.

Accordingly, it is favorable to substitute, for example, a non-polar amino acid for a non-polar amino acid and an uncharged amino acid for an uncharged amino acid. Above all, substitutions between Ala, Val, Leu and Ile, between Ser and Thr, between Asp and Glu, between Asn and Gln, between Lys and Arg and between Phe and Tyr are favorable as substitutions that maintain the nature of the protein. The numbers of amino acids and sites to be varied are not particularly limited.

A polypeptide having an amino acid sequence that is substantially identical to the amino acid sequence represented by SEQ ID NO: 3 comprises a polypeptide that is coded by a polynucleotide that hybridizes with a polynucleotide having a nucleotide sequence complementary to the nucleotide sequence represented by SEQ ID NO: 1 (nucleotides 106-2460) under stringent conditions and that has substantially the same activity as FGFR3 as described above.

Herein, polynucleotides that hybridize under stringent conditions specifically include polynucleotides that have, for example, at least 90% or higher, preferably 95% or higher, more preferably 97% or higher, still more preferably 98% or higher, still yet preferably 99% or higher identity to the nucleotide sequence represented by SEQ ID NO: 1 (nucleotides 106-2460) as calculated by a homology search software such as FASTA, BLAST, Smith-Waterman [Meth. Enzym., 164, 765 (1988)] or the like using default (initial setting) parameters. Examples of stringent conditions include "2 x SSC, 0.1% SDS, 50°C", "2 x SSC, 0.1% SDS, 42°C" and "1 x SSC, 0.1% SDS, 37°C". Examples of more stringent conditions include "2 x SSC, 0.1% SDS, 65°C", "0.5 x SSC, 0.1% SDS, 42°C" and "0.2 x SSC, 0.1% SDS, 65°C".

Hybridization may be carried out according to a known method. Alternatively, when a commercially available library is used, hybridization can be carried out according to the method described in the attached instruction.

Examples of a polynucleotide that hybridizes with a polynucleotide having a nucleotide sequence complementary to the nucleotide sequence represented by SEQ ID NO: 1 (nucleotides 106-2460) under stringent conditions include polynucleotides including a nucleotide sequence that has 90% or higher, preferably 95% or higher, more preferably 98% or higher identity to the nucleotide sequence represented by SEQ ID NO: 1 (nucleotides 106-2460).

Examples of a polynucleotide that hybridizes with a polynucleotide having a nucleotide sequence complementary to the nucleotide sequence represented by SEQ ID NO: 1 (nucleotides 106-2460) under stringent conditions include polynucleotides including a nucleotide sequence having one or more (e.g., one or several) nucleic acids varied, for example, deleted, substituted, added or the like in the nucleotide sequence represented by SEQ ID NO: 1 (nucleotides 106-2460).

Examples of a polynucleotide that hybridizes with a polynucleotide having a nucleotide sequence complementary to the nucleotide sequence represented by SEQ ID NO: 1 (nucleotides 106-2460) under stringent conditions include polynucleotides including:
(i) a nucleotide sequence having 1-9 (e.g., 1-5, preferably 1-3, more preferably 1-2, still more preferably one) nucleic acids deleted from the nucleotide sequence represented by SEQ ID NO: 1 (nucleotides 106-2460);
(ii) a nucleotide sequence having 1-9 (e.g., 1-5, preferably 1-3, more preferably 1-2, still more preferably one) nucleic acids added to the nucleotide sequence represented by SEQ ID NO: 1 (nucleotides 106-2460);
(iii) a nucleotide sequence having 1-9 (e.g., 1-5, preferably 1-3, more preferably 1-2, still more preferably one) nucleic acids substituted with other nucleic acids in the nucleotide sequence represented by SEQ ID NO: 1 (nucleotides 106-2460); or
(iv) a nucleotide sequence varied by any combination of (i)-(iii) above.

Herein, the term "identity" (also referred to as "homology") of an amino acid sequence is used to indicate the degree of consistency of amino acid residues forming the sequences to be compared. In order to calculate identity of a given amino acid sequence to an amino acid sequence to be compared, the presence of gaps and the nature of the amino acids are considered (Wilbur, Natl. Acad. Sci. U.S.A. 80:726-730 (1983)). For the calculation of identity, a commercially available software BLAST (Altschul: J. Mol. Biol. 215:403-410 (1990)), FASTA(Peasron: Methods in Enzymology 183:63-69 (1990)) or the like can be used.

The "identity" value may be any value as long as it is obtained using a homology search program known to those skilled in the art. For example, the default (initial setting) parameters can be used in homology algorithm BLAST (Basic local alignment search tool) http://www.ncbi.nlm.nih.gov/BLAST/ of the National Center for Biotechnology Information (NCBI) for the calculation.

According to the present invention, FGFR3 comprises mutant FGFR3 described below.

### (2) Cell overexpressing FGFR3

According to the present invention, a cell overexpressing FGFR3 comprises, for example, a cell expressing a significant amount of FGFR3 as compared to a normal cell. In addition, according to the present invention, a cell overexpressing FGFR3 comprises, for example, a cell expressing FGFR3 more than 1.5 times higher, preferably more than 2 times higher, more preferably more than 3 times higher, still more preferably more than 4 times higher than a normal cell.

Since a normal bone marrow cell barely expresses FGFR3 (Nature Genetics., 1997, 16, 260-264.), detection of FGFR3 in a bone marrow cell can be considered to indicate overexpression.

Overexpression of FGFR3 is frequently observed in a cell that has a t(4;14) translocation described below (Nature Genetics., 1997, 16, 260-264).

According to the present invention, a cell overexpressing FGFR3 is preferably a multiple myeloma cell.

An expression level of FGFR3 may be analyzed, for example, by determining a protein and/or mRNA of FGFR3 expressed in the cell.

An expression level of a protein can be determined, for example, by an immunochemical method (e.g., immunohistochemistry method, immunoprecipitation, western blotting, flow cytometry, ELISA, RIA, etc.), mass spectrometry or the like, preferably an immunochemical technique, particularly preferably flow cytometry. These methods may be carried out according to conventional techniques.

On the other hand, an expression level of mRNA can be determined, for example, by a method such as *in situ* hybridization, northern blot analysis, DNA microarray, RT-PCR or the like, preferably RT-PCR. These methods may be carried out according to conventional techniques.

### (3) Cell with t(4;14) translocation

According to the present invention, a cell that has a t(4;14) translocation refers to a cell associated with a translocation between the immunoglobulin heavy chain gene (IgH) at 14q32 and FGFR3 gene at 4p16 (Nature Genetics., 1997, 16, 260-264.).

The presence or the absence of a t(4; 14) translocation can be analyzed, for example, by a method such as PCR, RT-PCR and fluorescence *in situ* hybridization (FISH). These methods may be carried out according to conventional techniques.

In addition, the presence or the absence of a t(4;14) translocation can also be analyzed, for example, by immunochemical methods (e.g., immunohistochemistry method, immunoprecipitation, western blotting, flow cytometry, ELISA, RIA, etc.). These methods may be carried out according to conventional techniques.

According to the present invention, a cell that has a t(4;14) translocation is preferably a multiple myeloma cell.

### (4) Cell expressing mutant FGFR3

According to the present invention, mutant FGFR3 may be a polypeptide that includes an amino acid sequence having one or several amino acids deleted, substituted, added or varied by any combination thereof in the amino acid sequence of wild-type FGFR3 such as the amino acid sequence represented by SEQ ID NO: 3, and that has substantially the same activity as FGFR3. Preferably, mutant FGFR3 is a polypeptide that includes an amino acid sequence having one amino acid substituted in the amino acid sequence of wild-type FGFR3 such as the amino acid sequence represented by SEQ ID NO: 3, and that has substantially the same activity as FGFR3. According to the present invention, an example of a cell expressing mutant FGFR3 includes cell expressing the polypeptide above.

Examples of mutant FGFR3 include polypeptides including the sequences indicated in (i)-(ix) below.
(i) An amino acid sequence having arginine at position 248 substituted with other amino acid, preferably cysteine (R248C), in the amino acid sequence represented by SEQ ID NO: 2 (Nature Genetics., 1996, 13, 233-237., British Journal of Haematology., 2001, 114, 362-364).
(ii) An amino acid sequence having serine at position 249 substituted with other amino acid, preferably cysteine (S249C), in the amino acid sequence represented by SEQ ID NO: 2 (Clinical Cancer Research., 2005, 11, 7743-7748., Human Molecular Genetics., 2005, 14, 1153-1160).
(iii) An amino acid sequence having glycine at position 370 substituted with other amino acid, preferably cysteine (G370C), in the amino acid sequence represented by SEQ ID NO: 2 (Clinical Cancer Research., 2005, 11, 7743-7748., Human Molecular Genetics., 2005, 14, 1153-1160).
(iv) An amino acid sequence having serine at position 371 substituted with other amino acid, preferably cysteine (S371C), in the amino acid sequence represented by SEQ ID NO: 2 (Human Molecular Genetics., 2005, 14, 1153-1160).
(v) An amino acid sequence having tyrosine at position 373 substituted with other amino acid, preferably cysteine (Y373C), in the amino acid sequence represented by SEQ ID NO: 2 (Nature Genetics., 1997, 16, 260-264).
(vi) An amino acid sequence having glycine at position 380 substituted with other amino acid, preferably arginine (G380R), in the amino acid sequence represented by SEQ ID NO: 2 (Nature., 1994, 371, 252-254).
(vii) An amino acid sequence having phenylalanine at position 384 substituted with other amino acid, preferably leucine (F384L), in the amino acid sequence represented by SEQ ID NO: 2 (Blood. 2001, 97, 729-736).
(viii) An amino acid sequence having alanine at position 391 substituted with other amino acid, preferably glutamic acid (A391E), in the amino acid sequence represented by SEQ ID NO: 2 (Clinical Cancer Research., 2005, 11, 7743-7748).
(ix) An amino acid sequence having lysine at position 650 substituted with other amino acid, preferably glutamic acid, methionine, glutamine or threonine (K650E, K650M, K650Q or K650T), in the amino acid sequence represented by SEQ ID NO: 2 (Nature Genetics. 1997, 16, 260-264., Human Molecular Genetics., 2005, 14, 1153-1160).

Moreover, examples of mutant FGFR3 include those containing at least one of the substitutions indicated in (i)-(ix) above, specifically those containing mutation sites where at least one amino acid selected from the group consisting of amino acids of codons 248, 249, 370, 371, 373, 380, 384, 391 and 650 is substituted with other amino acid in the amino acid sequence represented by SEQ ID NO: 2. For example, a polypeptide including an amino acid sequence containing a mutation site where arginine at position 248 is substituted with cysteine and a mutation site where tyrosine at position 373 is substituted with cysteine in the amino acid sequence represented by SEQ ID NO: 2 is comprised in mutant FGFR3.

Herein, alphabetical notation of amino acids is expressed in generally used three-letter or single-letter codes. The alphabet preceding the number indicates single-letter code of the unsubstituted amino acid, the alphabet following the number indicates single-letter code of the amino acid that has replaced the original amino acid, and the number indicates the position of the amino acid in the amino acid sequence. For example, as indicated in (i) above, when arginine at position 248 is substituted with cysteine, it may be indicated as "R248C". This applies to other substitutions and, for example, serine at position 249 substituted with cysteine in (ii) may be indicated as "S249C", tyrosine at position 373 substituted with cysteine in (v) may be indicated as "Y373C", phenylalanine at position 384 substituted with leucine in (vii) may be indicated as "F384L", lysine at position 650 substituted with glutamic acid in (ix) may be indicated as "K650E", and lysine at position 650 substituted with methionine may be indicated as "K650M".

The number following the codon may indicate the position of the amino acid in the amino acid sequence. For example, "an amino acid of codon 248" refers to 248th amino acid in the amino acid sequence.

Preferably, mutant FGFR3 is activating-mutation-type FGFR3. Activating-mutation-type FGFR3 refers to mutant FGFR3 that causes ligand-independent autophosphorylation and that activates an intracellular signal.

Examples of the activating-mutation-type FGFR3 include polypeptides including the sequences of (a)-(c) below.
(a) An amino acid sequence where arginine at position 248 is substituted with cysteine (R248C) in the amino acids represented by SEQ ID NO: 2.
(b) An amino acid sequence where tyrosine at position 373 is substituted with cysteine (Y373C) in the amino acids represented by SEQ ID NO: 2.
(c) An amino acid sequence where lysine at position 650 is substituted with glutamic acid (K650E) in the amino acids represented by SEQ ID NO: 2.

These sequences are provided only for illustration and activating-mutation-type FGFR3 is not limited thereto. Activating-mutation-type FGFR3 may be mutant FGFR3 other than those of (a)-(c).

The presence or the absence of FGFR3 mutation can be determined by analyzing the gene sequence of FGFR3 or the transcript of FGFR3, i.e., the mRNA sequence. An example of a sequence analysis method includes dideoxynucleotide chain termination method (Sanger et al. (1977) Proc. Natl. Acad. Sci. USA 74: 5463). The sequence may be analyzed by employing an appropriate DNA sequencer.

The presence or the absence of FGFR3 mutation can also be analyzed by methods such as *in situ* hybridization, northern blot analysis, DNA microarray, RT-PCR, SSCP-PCR (Single-Strand Conformation Polymorphism-PCR) or the like. These methods may be carried out according to conventional techniques.

In addition, the presence or the absence of FGFR3 mutation can also be analyzed by immunochemical methods (e.g., immunohistochemistry method, immunoprecipitation, western blotting, flow cytometry, ELISA, RIA, etc.). These methods may be carried out according to conventional techniques.

According to the present invention, a cell expressing mutant FGFR3 is preferably a multiple myeloma cell.

### (5) Compound of the invention

Herein, "a halogen atom" refers to a fluorine atom, a chlorine atom, a bromine atom or an iodine atom.

Preferable examples of "a halogen atom" include a fluorine atom and a chlorine atom.

Herein, "C₁₋₆ alkyl group" refers to linear or branched alkyl group with a carbon number of 1-6, and specific examples include methyl group, ethyl group, 1-propyl group (n-propyl group), 2-propyl group (i-propyl group), 2-methyl-1-propyl group (i-butyl group), 2-methyl-2-propyl group (t-butyl group), 1-butyl group (n-butyl group), 2-butyl group (s-butyl group), 1-pentyl group, 2-pentyl group, 3-pentyl group, 2-methyl-1-butyl group, 3-methyl-1-butyl group, 2-methyl-2-butyl group, 3-methyl-2-butyl group, 2,2-dimethyl-1-propyl group, 1-hexyl group, 2- hexyl group, 3- hexyl group, 2-methyl-1-pentyl group, 3-methyl-1-pentyl group, 4-methyl-1-pentyl group, 2-methyl-2-pentyl group, 3-methyl-2-pentyl group, 4-methyl-2-pentyl group, 2-methyl-3-pentyl group, 3-methyl-3-pentyl group, 2,3-dimethyl-1-butyl group, 3,3-dimethyl-1-butyl group, 2,2-dimethyl-1-butyl group, 2-ethyl-1-butyl group, 3,3-dimethyl-2-butyl group and 2,3-dimethyl-2-butyl group.

Preferable examples of "C₁₋₆ alkyl group" include methyl group, ethyl group, 1-propyl group, 2-propyl group, 2-methyl-1-propyl group, 2-methyl-2-propyl group, 1-butyl group and 2-butyl group.

Herein, "C₁₋₆ alkylene group" refers to divalent group derived from the "C₁₋₆ alkyl group" defined above by removing any one hydrogen atom therefrom, and specific examples include methylene group, 1,2-ethylene group, 1,1-ethylene group, 1,3-propylene group, tetramethylene group, pentamethylene group and hexamethylene group.

Herein, "C₂₋₆ alkenyl group" refers to linear or branched alkenyl group having one double bond and a carbon number of 2-6, and specific examples include ethenyl group (vinyl group), 1-propenyl group, 2-propenyl group (allyl group), 1-butenyl group, 2-butenyl group, 3-butenyl group, pentenyl group and hexenyl group.

Herein, "C₂₋₆ alkynyl group" refers to linear or branched alkynyl group having one triple bond and a carbon number of 2-6, and specific examples include ethinyl group, 1-propynyl group, 2-propynyl group, 1-butynyl group, 2-butynyl group, 3-butynyl group, pentynyl group and hexynyl group.

Herein, "C₃₋₈ cycloalkyl group" refers to monocyclic or bicyclic saturated aliphatic hydrocarbon group with a carbon number of 3-8, and specific examples include cyclopropyl group, cyclobutyl group, cyclopentyl group, cyclohexyl group, cycloheptyl group, cyclooctyl group, bicyclo[2. 1. 0]pentyl group, bicyclo[3. 1. 0]hexyl group, bicyclo[2. 1. 1]hexyl group, bicyclo[4. 1. 0]heptyl group, bicyclo[2. 2. 1]heptyl group (norbornyl group), bicyclo[3. 3. 0]octyl group, bicyclo[3. 2. 1]octyl group and bicyclo[2. 2. 2]octyl group.

Preferable examples of "C₃₋₈ cycloalkyl group" include cyclopropyl group, cyclobutyl group and cyclopentyl group.

Herein, "C₆₋₁₀ aryl group" refers to aromatic hydrocarbon cyclic group with a carbon number of 6-10, and specific examples include phenyl group, 1-naphthyl group, 2-naphthyl group, indenyl group and azulenyl group.

A preferable example of "C₆₋₁₀ aryl group" includes phenyl group.

Herein, "a heteroatom" refers to a nitrogen atom, an oxygen atom or a sulfur atom.

Herein, "5-10-membered heteroaryl group" refers to aromatic cyclic group having 5-10 atoms forming the ring and 1-5 heteroatoms included in the atom forming the ring, and specific examples include furyl group, thienyl group, pyrrolyl group, imidazolyl group, triazolyl group, tetrazolyl group, thiazolyl group, pyrazolyl group, oxazolyl group, isoxazolyl group, isothiazolyl group, furazanyl group, thiadiazolyl group, oxadiazolyl group, pyridyl group, pyrazinyl group, pyridazinyl group, pyrimidinyl group, triazinyl group, purinyl group, pteridinyl group, quinolyl group, isoquinolyl group, naphthyridinyl group, quinoxalinyl group, cinnolinyl group, quinazolinyl group, phthalazinyl group, imidazopyridyl group, imidazothiazolyl group, imidazoxazolyl group, benzothiazolyl group, benzoxazolyl group, benzimidazolyl group, indolyl group, isoindolyl group, indazolyl group, pyrrolopyridyl group, thienopyridyl group, furo-pyridyl group, benzothiadiazolyl group, benzoxadiazolyl group, pyridopyrimidinyl group, benzofuryl group, benzothienyl group and thienofuryl group.

Preferable examples of "5-10-membered heteroaryl group" include furyl group, thienyl group, pyrrolyl group, imidazolyl group, thiazolyl group, pyrazolyl group, oxazolyl group, isoxazolyl group, isothiazolyl group, pyridyl group and pyrimidinyl group.

Herein, "3-10-membered nonaromatic heterocyclic group":
(a) has 3-10 atoms forming the ring;
(b) has 1-2 heteroatoms included in the atoms forming the ring;
(c) may include 1-2 double bonds in the ring;
(d) may have 1-3 carbonyl group, sulfinyl group or sulfonyl group in the ring; and
(e) is nonaromatic monocyclic or bicyclic group. When a nitrogen atom is included in the atoms forming the ring, the nitrogen atom may have a chemical bond. Specific examples include aziridinyl group, azetidinyl group, pyrrolidinyl group, piperidinyl group, azepanyl group, azocanyl group, piperazinyl group, diazepanyl group, diazocanyl group, diazabicyclo[2. 2. 1]heptyl group, morpholinyl group, thiomorpholinyl group, 1,1-dioxothiomorpholinyl group, oxiranyl group, oxetanyl group, tetrahydrofuryl group, dioxoranyl group, tetrahydropyranyl group, dioxanyl group, tetrahydrothienyl group, tetrahydrothiopyranyl group, oxazolidinyl group and thiazolidinyl group.

Preferable examples of "3-10-membered nonaromatic heterocyclic group" include aziridinyl group, azetidinyl group, pyrrolidinyl group, piperidinyl group, azepanyl group, piperazinyl group, diazepanyl group, morpholinyl group, thiomorpholinyl group, 1,1-dioxothiomorpholinyl group, tetrahydrofuryl group and tetrahydropyranyl group.

Herein, "C₁₋₆ alkoxy group" refers to group in which an oxygen atom is bound to the terminal of "C₁₋₆ alkyl group" defined above, and specific examples include methoxy group, ethoxy group, 1-propoxy group (n-propoxy group), 2-propoxy group (i-propoxy group), 2-methyl-1-propoxy group (i-butoxy group), 2-methyl-2-propoxy group (t-butoxy group), 1-butoxy group (n-butoxy group), 2-butoxy group (s-butoxy group), 1-pentyloxy group, 2-pentyloxy group, 3-pentyloxy group, 2-methyl-1-butoxy group, 3-methyl-1-butoxy group, 2-methyl-2-butoxy group, 3-methyl-2-butoxy group, 2,2-dimethyl-1-propoxy group, 1-hexyloxy group, 2-hexyloxy group, 3-hexyloxy group, 2-methyl-1-pentyloxy group, 3-methyl-1-pentyloxy group, 4-methyl-1-pentyloxy group, 2-methyl-2-pentyloxy group, 3-methyl-2-pentyloxy group, 4-methyl-2-pentyloxy group, 2-methyl-3-pentyloxy group, 3-methyl-3-pentyloxy group, 2,3-dimethyl-1-butoxy group, 3,3-dimethyl-1-butoxy group, 2,2-dimethyl-1-butoxy group, 2-ethyl-1-butoxy group, 3,3-dimethyl-2-butoxy group and 2,3-dimethyl-2-butoxy group.

Preferable examples of "C₁₋₆ alkoxy group" include methoxy group, ethoxy group, 1-propoxy group, 2-propoxy group, 2-methyl-1-propoxy group, 2-methyl-2-propoxy group, 1-butoxy group and 2-butoxy group.

Herein, "C₁₋₆ alkylthio group" refers to group in which a sulfur atom is bound to the terminal of "C₁₋₆ alkyl group" defined above, and specific examples include methylthio group, ethylthio group, 1-propylthio group (n-propylthio group), 2-propylthio group (i-propylthio group), 2-methyl-1-propylthio group (i-butylthio group), 2-methyl-2-propylthio group (t-butylthio group), 1-butylthio group (n-butylthio group), 2-butylthio group (s-butylthio group), 1-pentylthio group, 2-pentylthio group, 3-pentylthio group, 2-methyl-1-butylthio group, 3-methyl-1-butylthio group, 2-methyl-2-butylthio group, 3-methyl-2-butylthio group, 2,2-dimethyl-1-propylthio group, 1-hexylthio group, 2-hexylthio group, 3-hexylthio group, 2-methyl-1-pentylthio group, 3-methyl-1-pentylthio group, 4-methyl-1-pentylthio group, 2-methyl-2-pentylthio group, 3-methyl-2-pentylthio group, 4-methyl-2-pentylthio group, 2-methyl-3-pentylthio group, 3-methyl-3-pentylthio group, 2,3-dimethyl-1-butylthio group, 3,3-dimethyl-1-butylthio group, 2,2-dimethyl-1-butylthio group, 2-ethyl-1-butylthio group, 3,3-dimethyl-2-butylthio group and 2,3-dimethyl-2-butylthio group.

Preferable examples of "C₁₋₆ alkylthio group" include methylthio group, ethylthio group, 1-propylthio group (n-propylthio group), 2-propylthio group (i-propylthio group), 2-methyl-1-propylthio group (i-butylthio group), 2-methyl-2-propylthio group (t-butylthio group), 1-butylthio group (n-butylthio group) and 2-butylthio group (s-butylthio group).

Herein, "C₃₋₈ cycloalkoxy group" refers to group in which an oxygen atom is bound to the terminal of "C₃₋₈ cycloalkyl group" defined above, and specific examples include cyclopropoxy group, cyclobutoxy group, cyclopentyloxy group, cyclohexyloxy group, cycloheptyloxy group, cyclooctyloxy group, bicyclo[2. 1. 0]pentyloxy group, bicyclo[3. 1. 0]hexyloxy group, bicyclo[2. 1. 1]hexyloxy group, bicyclo[4. 1. 0]heptyloxy group, bicyclo[2. 2. 1]heptyloxy group (norbornyloxy group), bicyclo[3. 3. 0]octyloxy group, bicyclo[3. 2. 1]octyloxy group and bicyclo[2. 2. 2]octyloxy group.

Preferable examples of "C₃₋₈ cycloalkoxy group" include cyclopropoxy group, cyclobutoxy group and cyclopentyloxy group.

Herein, "mono-C₁₋₆ alkylamino group" refers to group in which a hydrogen atom in amino group is substituted with "C₁₋₆ alkyl group" defined above, and specific examples include methylamino group, ethylamino group, 1-propylamino group (n-propylamino group), 2-propylamino group (i-propylamino group), 2-methyl-1-propylamino group (i-butylamino group), 2-methyl-2-propylamino group (t-butylamino group), 1-butylamino group (n-butylamino group), 2-butylamino group (s-butylamino group), 1-pentylamino group, 2-pentylamino group, 3-pentylamino group, 2-methyl-1-butylamino group, 3-methyl-1-butylamino group, 2-methyl-2-butylamino group, 3-methyl-2-butylamino group, 2,2-dimethyl-1-propylamino group, 1-hexylamino group, 2-hexylamino group, 3-hexylamino group, 2-methyl-1-pentylamino group, 3-methyl-1-pentylamino group, 4-methyl-1-pentylamino group, 2-methyl-2-pentylamino group, 3-methyl-2-pentylamino group, 4-methyl-2-pentylamino group, 2-methyl-3-pentylamino group, 3-methyl-3-pentylamino group, 2,3-dimethyl-1-butylamino group, 3,3-dimethyl-1-butylamino group, 2,2-dimethyl-1-butylamino group, 2-ethyl-1-butylamino group, 3,3-dimethyl-2-butylamino group and 2,3-dimethyl-2-butylamino group.

Herein, "di-C₁₋₆ alkylamino group" refers to group in which two hydrogen atoms in amino group are substituted with identical or different "C₁₋₆ alkyl group" defined above, and specific examples include N,N-dimethylamino group, N,N-diethylamino group, N,N-di-n-propylamino group, N,N-di-i-propylamino group, N,N-di-n-butylamino group, N,N-di-i-butylamino group, N,N-di-s-butylamino group, N,N-di-t-butylamino group, N-ethyl-N-methylamino group, N-n-propyl-N-methylamino group, N-i-propyl-N-methylamino group, N-n-butyl-N-methylamino group, N-i-butyl-N-methylamino group, N-s-butyl-N-methylamino group and N-t-butyl-N-methylamino group.

Herein, "C₂₋₇ acyl group" refers to carbonyl group bound with "C₁₋₆ alkyl group" defined above, and specific examples include acetyl group, propionyl group, isopropionyl group, butyryl group, isobutyryl group, valeryl group, isovaleryl group and pivaloyl group.

Herein, "C₂₋₇ alkoxycarbonyl group" refers to carbonyl group bound with "C₁₋₆ alkoxy group" defined above, and specific examples include methoxycarbonyl group, ethoxycarbonyl group, 1-propyloxycarbonyl group, 2-propyloxycarbonyl group and 2-methyl-2-propoxycarbonyl group.

Herein, "that may have a substituent" means "that may have one or more substituents in any combination at substitutable positions", and specific examples of substituents include a halogen atom, hydroxyl group, thiol group, nitro group, cyano group, formyl group, carboxyl group, amino group, silyl group, methanesulfonyl group, C₁₋₆ alkyl group, C₂₋₆ alkenyl group, C₂₋₆ alkynyl group, C₃₋₈ cycloalkyl group, C₆₋₁₀ aryl group, 5-10-membered heteroaryl group, 3-10-membered nonaromatic heterocyclic group, C₁₋₆ alkoxy group, C₁₋₆ alkylthio group, C₃₋₈ cycloalkoxy group, mono-C₁₋₆ alkylamino group, di-C₁₋₆ alkylamino group, C₂₋₇ acyl group and C₂₋₇ alkoxycarbonyl group. In this case, C₁₋₆ alkyl group, C₂₋₆ alkenyl group, C₂₋₆ alkynyl group, C₃₋₈ cycloalkyl group, C₆₋₁₀ aryl group, 5-10-membered heteroaryl group, 3-10-membered nonaromatic heterocyclic group, C₁₋₆ alkoxy group, C₁₋₆ alkylthio group, C₃₋₈ cycloalkoxy group, mono-C₁₋₆ alkylamino group, di-C₁₋₆ alkylamino group, C₂₋₇ acyl group and C₂₋₇ alkoxycarbonyl group may each independently have 1-3 groups selected from the group consisting of the following substituent groups.

### <Substituent groups>

A halogen atom, hydroxyl group, thiol group, nitro group, cyano group, C₁₋₆ alkyl group, C₃₋₈ cycloalkyl group, C₂₋₆ alkenyl group, C₂₋₆ alkynyl group, C₆₋₁₀ aryl group, 5-10-membered heteroaryl group, 3-10-membered nonaromatic heterocyclic group, C₁₋₆ alkoxy group and C₁₋₆ alkylthio group.

According to the present invention, a compound represented by General Formula (I) is as follows.

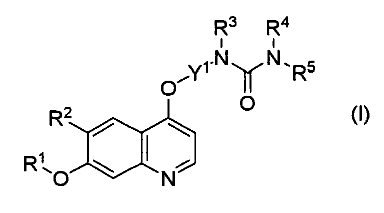

### (i) R¹

R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent).

A preferable example of R¹ includes C₁₋₆ alkyl group. In this case, R¹ may have a substituent selected from 3-10-membered nonaromatic heterocyclic group, hydroxyl group, C₁₋₆ alkoxy group, amino group, mono-C₁₋₆ alkylamino group and di-C₁₋₆ alkylamino group which may have C₁₋₆ alkyl group.

More preferable examples of R¹ include methyl group and group represented by any one of the following Formulae 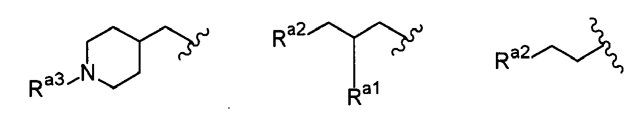 (wherein, R^{a3} represents methyl group; R^{a1} represents a hydrogen atom or hydroxyl group; R^{a2} represents methoxy group, ethoxy group, 1-pyrrolidinyl group, 1-piperidinyl group, 4-morpholinyl group, dimethylamino group or diethylamino group).

Still more preferable examples of R¹ include methyl group and 2-methoxyethyl group.

### (ii) R²

R² represents cyano group, C₁₋₆ alkoxy group that may have a substituent, carboxyl group, C₂₋₇ alkoxycarbonyl group that may have a substituent or group represented by Formula -CONV^{a11}V¹² (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent).

Preferable examples of R² include cyano group or group represented by Formula -CONV^{a11}V^{a12} (wherein, V^{a11} and V^{a12} have the same meaning as defined above).

More preferable examples of R² include cyano group or group represented by Formula -CONHV^{a16} (wherein, V^{a16} represents a hydrogen atom, C₁₋₆ alkyl group, C₃₋₈ cycloalkyl group, C₁₋₆ alkoxy group or C₃₋₈ cycloalkoxy group, where V^{a16} may have a substituent selected from a halogen atom, cyano group, hydroxyl group and C₁₋₆ alkoxy group).

Still more preferable example of R² includes group represented by Formula -CONHV^{a17} (wherein, V^{a17} represents a hydrogen atom, C₁₋₆ alkyl group or C₁₋₆ alkoxy group).

The most preferable example of R² include group represented by Formula -CONHV^{a18} (wherein, V^{a18} represents a hydrogen atom, methyl group or methoxy group).

### (iii) Y¹

Y¹ represents group represented by Formula 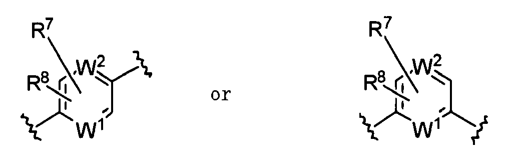 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent); and W¹ and W² each independently represent a carbon atom or a nitrogen atom that may have a substituent).

A preferable example of Y¹ includes group represented by Formula 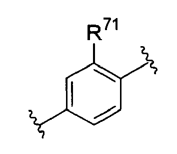 (wherein, R⁷¹ represents a hydrogen atom or a halogen atom).

### (iv) R³ and R⁴

R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent.

A preferable example of R³ and R⁴ includes a hydrogen atom.

### (v) R⁵

R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent.

Preferable examples of R⁵ include a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent or 3-10-membered nonaromatic heterocyclic group that may have a substituent.

More preferable examples of R⁵ include a hydrogen atom, C₁₋₆ alkyl group, C₃₋₈ cycloalkyl group and C₆₋₁₀ aryl group (where R⁵ may have at least one substituent selected from the group consisting of a halogen atom and methanesulfonyl group).

More preferable examples of R⁵ include methyl group, ethyl group or cyclopropyl group.

Moreover, preferable examples of the compound represented by General Formula (I) include:
N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl)-N'-(4-fluorophenyl)urea;
N-(4-((6-cyano-7-(((2R)-2-hydroxy-3-(1-pyrrolidino)propyl)oxy)-4-quinolyl)oxy)phenyl) -N'-(4-fluorophenyl)urea;
4-(3-chloro-4-(cyclopropylaminocarbonyl)aminophenoxy)-7-methoxy-6-quinolinecarbox amide;
4-(3-chloro-4-(cyclopropylaminocarbonyl)aminophenoxy)-7-(2-methoxyethoxy)-6-quino linecarboxamide;
N6-cyclopropyl-4-(3-chloro-4-(((cyclopropylamino)carbonyl)amino)phenoxy)-7-methox y-6-quinolinecarboxamide;
N6-(2-methoxyethyl)-4-(3-chloro-4-(((cyclopropylamino)carbonyl)amino)phenoxy)-7-m ethoxy-6-quinolinecarboxamide;
N6-(2-fluoroethyl)-4-(3-chloro-4-(((cyclopropylamino)carbonyl)amino)phenoxy)-7-meth oxy-6-quinolinecarboxamide;
N6-methoxy-4-(3-chloio-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-q uinolinecarboxamide;
N6-ethyl-4-(3-chloro-4-(((cyclopropylamino)carbonyl)amino)phenoxy)-7-methoxy-6-qui nolinecarboxamide;
4-(3-fluoro-4-(cyclopropylaminocarbonyl)aminophenoxy)-7-(2-methoxyethoxy)-6-quinol inecarboxamide;
4-(3-chloro-4-(cyclopropylaminocarbonyl)aminophenoxy)-7-(2-hydroxyethoxy)-6-quinol inecarboxamide;
4-(3-chloro-4-(cyclopropylaminocarbonyl)aminophenoxy)-7-((2S)-2,3-dihydroxypropyl) oxy-6-quinolinecarboxamide;
4-(3-chloro-4-(methylaminocarbonyl)aminophenoxy)-7-methoxy-6-quinolinecarboxamid e;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinol inecarboxamide;
4-(3-chloro-4-(cyclopropylaminocarbonyl)aminophenoxy)-7-(2-ethoxyethoxy)-6-quinoli necarboxamide;
4-(4-((cyclopropylamino)carbonyl)aminophenoxy)-7-(2-methoxyethoxy)-6-quinolinecarb oxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-me thoxy-6-quinolinecarboxamide;
4-(3-chloro-4-(1-propylaminocarbonyl)aminophenoxy)-7-methoxy-6-quinolinecarboxami de;
4-(3-chloro-4-(cis-2-fluoro-cyclopropylaminocarbonyl)aminophenoxy)-7-methoxy-6-qui nolinecarboxamide;
N6-methyl-4-(3-chloro-4-(((cyclopropylamino)carbonyl)amino)phenoxy)-7-(2-methoxyet hoxy)-6-quinolinecarboxamide;
N6-methyl-4-(3-chloro-4-(((ethylamino)carbonyl)amino)phenoxy)-7-methoxy-6-quinolin ecarboxamide;
4-(3-chloro-4-(cyclopropylaminocarbonyl)aminophenoxy)-7-(2-(4-morpholino)ethoxy)-6 -quinolinecarboxamide;
4-(3-chloro-4-(2-fluoroethylaminocarbonyl)aminophenoxy)-7-methoxy-6-quinolinecarbo xamide;
N6-((2R)tetrahydro-2-furanylmethyl)-4-(3-chloro-4-(((methylamino)carbonyl)amino)phe noxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-py rrolidino)propoxy)-6-quinolinecarboxamide;
N6-methyl-4-(3-chloro-4-(((methylamino)carbonyl)amino)phenoxy)-7-((2R)-3-diethylam ino-2-hydroxypropoxy)-6-quinolinecarboxamide;
N6-methyl-4-(3-chloro-4-(((ethylamino)carbonyl)amino)phenoxy)-7-((2R)-3-diethylamin o-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-( 1-pyrrolidino)propoxy)-6-quinolinecarboxamide;
N6-methyl-4-(3-chloro-4-(((methylamino)carbonyl)amino)phenoxy)-7-((1-methyl-4-pipe ridyl)methoxy)-6-quinolinecarboxamide;
N6-methyl-4-(3-chloro-4-(((ethylamino)carbonyl)amino)phenoxy)-7-((1-methyl-4-piperi dyl)methoxy)-6-quinolinecarboxamide;
N-(4-(6-cyano-7-(2-methoxyethoxy)-4-quinolyl)oxy-2-fluorophenyl)-N'-cyclopropylurea ;
N-(4-(6-cyano-7-(3-(4-morpholino)propoxy)-4-quinolyl)oxyphenyl)-N'-(3-(methylsulfon yl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarb oxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopr opylurea.

More preferable examples of the compound represented by General Formula (I) further include:
4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamid e; and
N6-methoxy-4-(3-chloro-4-(((ethylamino)carbonyl)amino)phenoxy)-7-methoxy-6-quinol inecarboxamide.

A still more preferable example of the compound represented by General Formula (I) further includes
4-(3-chloro-4-(cyclopropylaminocarbonyl)aminophenoxy)-7-methoxy-6-quinolinecarbox amide (see Formula (II)).

The most preferable example of the compound of the invention represented by General Formula (I) includes methanesulfonate of 4-(3-chloro-4-(cyclopropylaminocarbonyl)aminophenoxy)-7-methoxy-6-quinolinecarbox amide.

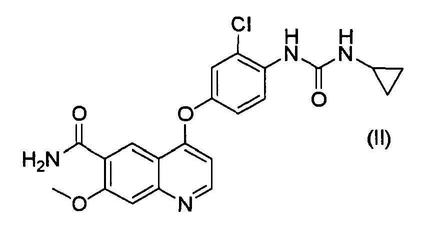

The compound represented by General Formula (I) can be produced by a known method, for example, by methods described in International publication No. 02/32872 pamphlet (WO02/32872) and International publication No. 2005/063713 pamphlet (WO2005/063713)

According to the present invention, the compound represented by General Formula (I) may form a pharmacologically acceptable salt with acid or base. According to the present invention, the compound of the invention also comprises such pharmacologically acceptable salts. Examples of salts formed with acid include inorganic acid salts such as hydrochloride, hydrobromate, sulfate and phosphate, and organic acid salts such as formate, acetate, lactate, succinate, fumarate, maleate, citrate, tartarate, stearate, benzoate, methanesulfonate, benzenesulfonate, p-toluenesulfonate and trifluoroacetate. Examples of salts formed with base include alkali metal salts such as sodium salt and potassium salt, alkaline earth metal salts such as calcium salt and magnesium salt, organic base salts such as trimethylamine, triethylamine, pyridine, picoline, dicyclohexylamine, N, N'-dibenzyl ethylenediamine, arginine and lysine and ammonium salt.

Furthermore, according to the present invention, the compound represented by General Formula (I) also comprises, if any, solvates and enantiomers thereof. According to the present invention, the compound of the invention comprises these solvates and enantiomers. Examples of solvates include hydrates and nonhydrates, preferably hydrates. Examples of solvents include water, alcohols (for example, methanol, ethanol, n-propanol) and dimethylformamide.

Moreover, according to the present invention, the compound represented by General Formula (I) may be crystalline or amorphous. If a crystalline polymorph is present, it may be single crystalline or a polymorph mixture of any crystalline shape

According to the present invention, the compound of the invention also comprises compounds that generate the compound represented by General Formula (I) by undergoing metabolism such as oxidation, reduction and hydrolysis *in vivo*.

Preferably, the compound of the invention is a substance (a FGFR3 inhibitor) that has an activity of inhibiting a kinase activity of FGFR3 (hereinafter, also referred to as a "FGFR3-inhibiting activity"). Herein, a "kinase activity of FGFR3" refers to an activity of FGFR3 to phosphorylate a tyrosine residue of its or other protein.

Examples of methods for determining the FGFR3-inhibiting activity of the compound of the invention include cell free kinase assay, western blotting, cell growth assay and viability assay. Examples of the cell growth assay include tritium thymidine uptake method, MTT method, XTT method (cell counting kit-8 (Dojindo Laboratories)), AlamarBlue technique, Neutral Red technique, BrdU technique, Ki67 staining and PCNA staining. Examples of the viability assay include TUNNEL staining, Caspase-3 cleavage detection and PARP cleavage detection. These methods may be carried out according to conventional techniques (Blood. 2005, 105, 2941-2948., Molecular Cancer Therapeutics. 2005, 4, 787-798).

Hereinafter, an example of a method for determining a FGFR3-inhibiting activity will be described.

The FGFR3-inhibiting activity can be determined by cell free kinase assay.

FGFR3 can be prepared by gene-engineering means according to a conventional method. For example, according to the method of Baculovirus Expression System, human recombinant GST (glutathione S-transferase) fusion protein, human recombinant histidine-tag fusion protein or the like may be expressed in an insect cell (*Spondopteα frugiperda* 9 (Sf9)). Furthermore, the expressed recombinant protein can be purified by affinity chromatography (e.g., GSH-agarose (from Sigma) or Ni-NTH-agarose (from Qiagen)). The purity and identification of the protein can be confirmed by SDS-PAGE, silver staining and western blotting using an antibody specific to FGFR3.

The cell free kinase assay can be carried out as follows.

First, to each well of a plate (e.g., 96-well, 384-well, etc.), a mixed solution containing 20 µl of standard reaction solution, 5 µl of ATP solution, 5 µl of the test substance, 10 µl of solution containing 100 ng of FGFR3 recombinant protein and 10 µl of solution containing 125 ng ofbiotinylated Poly(Glu, Tyr)_{4:1} can be added sequentially.

This kinase reaction solution (50 µl) may contain 60 mM HEPES-NaOH (pH7.5), 3 mM MgCl₂, 3 mM MnCl₂, 3 µM Na-orthovanadate, 1.2 mM DTT (dithiothreitol), 50 µg/ml PEG (polyethylene glycol) ₂₀₀₀₀ and 1 µM ATP. In this case, the ATP may be labeled with a radioactive isotope such as [γ-³²P]-ATP and [γ-³³P]-ATP.

The reaction solution may be incubated for a certain period of time, and then 50 µl of 2% (v/v) H₃PO₄ solution may be added to terminate the reaction.

Each well may be subjected to an appropriate washing procedure.

A FGFR3-inhibiting activity can be assessed by determining the amount of ATP incorporation. When ATP labeled with a radioactive isotope as mentioned above is used, the amount of ATP incorporation can be assessed by determining radioactivity captured on the plate with a scintillation counter.

According to this method, the FGFR3-inhibiting activity of the compound of the invention can be assessed.

### (6) Pharmaceutical composition, therapeutic drug and therapeutic method

The pharmaceutical composition of the invention is a pharmaceutical composition comprising a compound of the invention, which is to be administered to a living organism having at least one cell selected from the group consisting of a cell overexpressing FGFR3, a cell that has a t(4;14) translocation and a cell expressing mutant FGFR3.

The pharmaceutical composition of the invention may be used as a therapeutic drug for treating a disease comprising at least one cell selected from the group consisting of a cell overexpressing FGFR3, a cell that has a t(4;14) translocation and a cell expressing mutant FGFR3. Examples of such disease include multiple myeloma, bladder cancer, cervical cancer, hypochondroplasia, achondroplasia, thanatophoric dysplasia and skeletal dysplasia.

Furthermore, the pharmaceutical composition of the invention is effective as a pharmaceutical composition for treating cancer comprising at least one cell selected from the group consisting of a cell overexpressing FGFR3, a cell that has a t(4;14) translocation and a cell expressing mutant FGFR3, and thus may be used as a therapeutic drug for treating cancer.

According to the present invention, a therapeutic drug for treating cancer comprises an antitumor drug, a drug for improving prognosis of cancer, a drug for preventing cancer recurrence, a drug for suppressing cancer metastasis and the like.

The effect of cancer treatment may be confirmed by observation of an x-ray picture, CT or the like, by histopathological diagnosis of biopsy, or from a tumor marker value.

The types of cancer treated by the pharmaceutical composition for treating the cancer comprising at least one cell selected from the group consisting of a cell overexpressing FGFR3, a cell that has a t(4;14) translocation and a cell expressing mutant FGFR3 may include, for example, multiple myeloma, bladder cancer and cervical cancer and the like, and more preferably multiple myeloma.

The pharmaceutical composition of the invention may be administered to a living organism, i.e., a mammal (e.g., human, rat, rabbit, sheep, pig, bovine, cat, dog, monkey, etc.). According to the present invention, the living organism may have any one, two or all of a cell overexpressing FGFR3, a cell that has a t(4;14) translocation and a cell expressing mutant FGFR3.

The therapeutic drug of the invention comprises the compound of the invention and is a drug for treating at least one disease selected from the group consisting of multiple myeloma, bladder cancer, cervical cancer, hypochondroplasia, achondroplasia, thanatophoric dysplasia and skeletal dysplasia. Preferably, the therapeutic drug of the invention is used for a disease comprising at least one cell selected from the group consisting of a cell overexpressing FGFR3, a cell that has a t(4;14) translocation and a cell expressing mutant FGFR3.

The therapeutic drug of the invention may be administered to a living organism, i.e., a mammal (e.g., human, rat, rabbit, sheep, pig, bovine, cat, dog, monkey, etc.).

Where a pharmaceutical composition or a therapeutic drug of the invention is used, the given dose of the compound of the invention differs depending on the degree of the symptom, age, sex, weight and sensitivity difference of the patient, administration mode, administration period, administration interval, nature, prescription and the type of the pharmaceutical formulation, and the type of the active ingredient. Usually, but without limitation, the dose of the compound is 0.1-1000 mg/day, preferably 0.5-100 mg/day, more preferably 1-30 mg/day for an adult (weight 60 kg), which may be administered usually once to three times a day.

Although the pharmaceutical composition or the therapeutic drug comprising the compound of the invention as an active ingredient may be used alone, it is usually mixed with appropriate additives and made into a formulation.

Examples of such additive include excipients, binders, lubricants, disintegrants, colorants, flavoring agents, emulsifiers, surfactants, solubilizing agents, suspending agents, tonicity agents, buffers, antiseptic agents, antioxidant agents, stabilizers, absorption promoters and the like that are generally used for medicine. If required, they may be used in combination. Examples of such additive are as follows.

Excipients: lactose, sucrose, glucose, cornstarch, mannitol, sorbitol, starch, alpha-starch, dextrin, crystalline cellulose, light anhydrous silicic acid, aluminum silicate, calcium silicate, magnesium aluminometasilicate and calcium hydrogen phosphate.

Binders: for example, polyvinyl alcohol, methyl cellulose, ethyl cellulose, gum arabic, tragacanth, gelatin, shellack, hydroxypropyl methylcellulose, hydroxypropylcellulose, carboxymethylcellulose sodium, polyvinylpyrrolidone and macrogol.

Lubricants: magnesium stearate, calcium stearate, sodium stearyl fumarate, talc, polyethyleneglycol and colloid silica.

Disintegrants: crystalline cellulose, agar, gelatin, calcium carbonate, sodium hydrogen carbonate, calcium citrate, dextrin, pectin, low substituted hydroxypropylcellulose, carboxymethylcellulose, carboxymethylcellulose calcium, croscarmellose sodium, carboxymethyl starch and carboxymethyl starch sodium.

Colorants: ferric oxide, yellow ferric oxide, carmine, caramel, beta-carotene, titanium oxide, talc, riboflavin sodium phosphate, yellow aluminum lake and the like that are approved as additives in drugs.

Flavoring agents: cocoa powder, menthol, aromatic powder, peppermint oil, camphor and cinnamon powder.

Emulsifiers and surfactants: stearyltriethanolamine, sodium lauryl sulfate, laurylaminopropionate, lecithin, glycerine monostearate, sucrose fatty acid ester and glycerine fatty acid ester.

Solubilizing agents: polyethyleneglycol, propylene glycol, benzyl benzoate, ethanol, cholesterol, triethanolamine, sodium carbonate, sodium citrate, Polysorbate 80 and nicotine acid amide.

Suspending agents: for example, in addition to the surfactants mentioned above, hydrophilic polymers such as polyvinyl alcohol, polyvinylpyrrolidone, methylcellulose, hydroxymethylcellulose, hydroxyethylcellulose and hydroxypropylcellulose.

Tonicity agents: glucose, sodium chloride, mannitol and sorbitol.

Buffers: buffers such as phosphate, acetate, carbonate, citrate and the like.

Antiseptic agents: methylparaben, propylparaben, chlorobutanol, benzyl alcohol, phenethyl alcohol, dehydroacetic acid and sorbic acid.

Antioxidant agents: hydrosulfate, ascorbic acid and alpha-tocopherol.

Stabilizers: those generally used for medicine.

Absorption promoters: those generally used for medicine.

If required, components such as vitamins and amino acids may be blended.

Examples of formulations include oral formulations such as tablets, powder, granule, fine granule, capsule, syrup, lozenge and inhaler; external formulations such as suppository, ointment, eye ointment, poultice strip, eye-drops, nasal drops, eardrops, skin patch and lotion; and injectable formulations.

The oral formulations mentioned above may be formulated by appropriately combining the additives mentioned above. If necessary, surface of these formulations may be coated.

The external formulations mentioned above may be formulated by appropriately combining the additives mentioned above, particularly excipients, binders, flavoring agents, emulsifiers, surfactants, solubilizing agents, suspending agent, tonicity agents, antiseptic agents, antioxidant agents, stabilizers and absorption promoters.

The injectable formulations mentioned above may be formulated by appropriately combining the additives mentioned above, particularly emulsifiers, surfactants, solubilizing agents, suspending agents, tonicity agents, buffers, antiseptic agents, antioxidant agents, stabilizers and absorption promoters. The injectable formulations may be used through means such as infusion, intramuscular injection, subcutaneous injection, intradermal injection and intravenous injection.

The present invention relates to a method for treating a disease, comprising administering an effective amount of a compound of the invention to a living organism having at least one cell selected from the group consisting of a cell overexpressing FGFR3, a cell that has a t(4;14) translocation and a cell expressing mutant FGFR3. According to the present invention, the disease is preferably at least one disease selected from the group consisting of multiple myeloma, bladder cancer, cervical cancer, hypochondroplasia, achondroplasia, thanatophoric dysplasia and skeletal dysplasia.

Moreover, the present invention relates to a method for treating at least one disease selected from the group consisting of multiple myeloma, bladder cancer, cervical cancer, hypochondroplasia, achondroplasia, thanatophoric dysplasia and skeletal dysplasia, the method comprising administering an effective amount of a compound of the invention to a patient.

According to the therapeutic method of the invention, the route and the method for administering the compound of the invention are not particularly limited and reference may be made to the description of the pharmaceutical composition of the invention or the description of therapeutic drug above.

The present invention relates to use of a compound of the invention for producing a pharmaceutical composition which is to be administered to a living organism having at least one cell selected from the group consisting of a cell overexpressing FGFR3, a cell that has a t(4;14) translocation and a cell expressing mutant FGFR3. According to the use of the invention, the pharmaceutical composition is effective as a therapeutic drug for treating at least one disease selected from multiple myeloma, bladder cancer, cervical cancer, hypochondroplasia, achondroplasia, thanatophoric dysplasia and skeletal dysplasia.

Moreover, the present invention relates to use of a compound of the invention for producing a therapeutic drug for treating at least one disease selected from the group consisting of multiple myeloma, bladder cancer, cervical cancer, hypochondroplasia, achondroplasia, thanatophoric dysplasia and skeletal dysplasia.

The present invention relates to a compound of the invention for a pharmaceutical composition which is to be administered to a living organism having at least one cell selected from the group consisting of a cell overexpressing FGFR3, a cell that has a t(4;14) translocation and a cell expressing mutant FGFR3. According to the present invention, the pharmaceutical composition is effective as a therapeutic drug for treating at least one disease selected from multiple myeloma, bladder cancer, cervical cancer, hypochondroplasia, achondroplasia, thanatophoric dysplasia and skeletal dysplasia.

Moreover, the present invention relates to a compound of the invention for a therapeutic drug for treating at least one disease selected from the group consisting of multiple myeloma, bladder cancer, cervical cancer, hypochondroplasia, achondroplasia, thanatophoric dysplasia and skeletal dysplasia.

The present invention further provides a FGFR3 inhibitor comprising a compound of the invention. The FGFR3 inhibitor has an effect of inhibiting a kinase activity of FGFR3.

Although the compound of the invention is as described above, it is preferably 4-(3-chloro-4-(cyclopropylaminocarbonyl)aminophenoxy)-7-methoxy-6-quinolinecarbox amide, a pharmacologically acceptable salt thereof or a solvate thereof.

The FGFR3-inhibiting activity of the FGFR3 inhibitor of the invention can be determined as described above.

As the FGFR3 inhibitor of the invention, the compound of the invention may be used alone, or it may be formulated with appropriate additives mentioned above.

As to the usage and the dosage of the FGFR3 inhibitor, reference may be made to the description of the pharmaceutical composition above.

The present invention also relates to use of a compound represented by General Formula (I), a pharmacologically acceptable salt thereof or a solvate thereof for producing a FGFR3 inhibitor.

The present invention further relates to a compound represented by General Formula (I), a pharmacologically acceptable salt thereof or a solvate thereof for a FGFR3 inhibitor.

The present invention yet further relates to a method for inhibiting FGFR3, preferably a method for inhibiting FGFR3 kinase with a compound represented by General Formula (I), a pharmacologically acceptable salt thereof or a solvate thereof. According to the method of the invention, the usage and the dosage of the compound are not particularly limited and reference may be made to the description of the pharmaceutical composition above.

### 2. Method for Predicting Sensitivity

The present invention provides a method for predicting whether or not a patient is highly sensitive to a compound of the invention using at least one index selected from the group consisting of the FGFR3 expression level, the presence or the absence of a t(4;14) translocation and the presence or the absence of FGFR3 mutation in the cell.

According to the method of the invention, a patient is preferably a patient suffering from at least one disease selected from the group consisting of multiple myeloma, bladder cancer, cervical cancer, hypochondroplasia, achondroplasia, thanatophoric dysplasia and skeletal dysplasia. The patient is preferably a cancer patient, more preferably a patient suffering from multiple myeloma, bladder cancer or cervical cancer, particularly preferably a patient suffering from multiple myeloma.

### (1) Step of determining at least one selected from the group consisting of FGFR3 expression level, presence or absence of t(4;14) translocation and presence or absence of FGFR3 mutation in cell

In this step, the cell is preferably a cell taken from the patient. The cell may be obtained, for example, by removing it from a patient by a surgical procedure (e.g., biopsy, marrow puncture, etc.).

Preferably, the cell is a tumor cell. In the case of multiple myeloma, bladder cancer, cervical cancer, hypochondroplasia, achondroplasia, thanatophoric dysplasia or skeletal dysplasia that results from genetic variation, a blood cell is preferably used as the cell.

Examples of the types of tumor include multiple myeloma, bladder cancer and cervical cancer and the like, and more preferably multiple myeloma.

The FGFR3 expression level, the presence or the absence of a t(4;14) translocation and the presence or the absence of FGFR3 mutation can be determined by the method described in "1. Pharmaceutical composition, therapeutic drug and therapeutic method of the invention".

In this step, any one, a combination of two or more or all of the FGFR3 expression level, the presence or the absence of a t(4;14) translocation and the presence or the absence of FGFR3 mutation in the cell may be determined.

### (2) Step of predicting whether or not a patient is highly sensitive to compound of the invention

In this step, at least one index selected from the group consisting of the FGFR3 expression level, the presence or the absence of a t(4; 14) translocation and the presence or the absence of FGFR3 mutation in the cell that has been determined in (1) can be preferably used to predict whether or not a patient is highly sensitive to the compound of the invention. Specifically, when the cell determined corresponds to at least one of a cell overexpressing FGFR3, a cell that has a t(4;14) translocation and a cell expressing mutant FGFR3, the patient can be judged to be highly sensitive to the compound of the invention. Herein, the meanings of the cell overexpressing FGFR3, the cell that has a t(4;14) translocation and the cell expressing mutant FGFR3 are as described in "1. Pharmaceutical composition, therapeutic drug and therapeutic method of the invention".

Another aspect of the invention is a method for analyzing sensitivity of a cell to the compound of the invention using the results from determination in (1) as an index. When the cell corresponds to at least one of a cell overexpressing FGFR3, a cell that has a t(4;14) translocation and a cell expressing mutant FGFR3 based on the results from determination in (1), this cell can be judged to be highly sensitive to the compound of the invention as compared to a cell that does not correspond to any of these cells.

Yet another aspect of the invention is a method for selecting a cell or a patient that is highly sensitive to the compound of the invention using the results from the determination in (1) as an index. When the cell corresponds to at least one of a cell overexpressing FGFR3, a cell that has a t(4;14) translocation and a cell expressing mutant FGFR3 based on the results from determination in (1), this cell or the patient having this cell can be judged to be highly sensitive to the compound of the invention as described above. Thus, such cell or such patient can be selected as a cell or a patient highly sensitive to the compound of the invention.

Still yet another aspect of the invention is a method comprising analyzing the sensitivity of a patient to the compound of the invention using the results from the determination in (1) as an index, and classifying the patient according to the results from the analysis. Specifically, according to the method of the invention, the sensitivity to the compound of the invention is analyzed as described above based on the results from the determination in (1), and the cell can be classified based on the analysis results. For example, a cell can be classified into a group of cells that correspond to at least one of a cell overexpressing FGFR3, a cell that has a t(4;14) translocation and a cell expressing mutant FGFR3, or a group of cells that do not correspond to any of the above cells. Alternatively, the cell can be classified into a group of cells highly sensitive to the compound of the invention or a group of cells other than these cells.

Still yet another aspect of the invention is a method for selecting a patient for administering the compound of the invention, the method comprising selecting a patient having at least one cellselected from a cell overexpressing FGFR3, a cell that has a t(4;14) translocation and a cell expressing mutant FGFR3, based on the results from the determination in (1). Patients having at least one cell selected from a cell overexpressing FGFR3, a cell that has a t(4;14) translocation and a cell expressing mutant FGFR3 can be a target intended for administering the compound of the invention.

Still yet another aspect of the invention is a method for predicting the therapeutic effect of the compound of the invention on a patient based on the results from the determination in (1). According to the method of the invention, when the cell determined corresponds to at least one of a cell overexpressing FGFR3, a cell that has a t(4;14) translocation and a cell expressing mutant FGFR3 based on the results from the determination in (1), the cell is judged to be highly sensitive to the compound of the invention, and thus the therapeutic effect of this compound can be predicted be high on the cell or a patient having this cell.

The present invention also relates to a method for determining at least one selected from the group consisting of the FGFR3 expression level, the presence or the absence of a t(4;14) translocation and the presence or the absence of FGFR3 mutation in the cell derived from a patient for predicting the sensitivity level of the patient to the compound of the invention. This determination method is as described in (1) above.

Determination of any one or two or more of the FGFR3 expression level, the presence or the absence of a t(4;14) translocation and the presence or the absence of FGFR3 mutation in the cell enables prediction of the sensitivity level of a patient to the compound of the invention.

In this step, although the compound of the invention is as described above, it is preferably 4-(3-chloro-4-(cyclopropylaminocarbonyl)aminophenoxy)-7-methoxy-6-quinolinecarbox amide, a pharmacologically acceptable salt thereof or a solvate thereof.

The method of the invention can be employed to predict the degree of the efficacy of the compound of the invention on a patient before administering the compound of the invention to the patient. Therefore, patients who are more susceptible to the effect of the compound of the invention can be selected for carrying out the treatment of the disease. Thus, the present invention is clinically highly effective.

The present invention provides a test kit for determining at least one selected from the group consisting of the FGFR3 expression level, the presence or the absence of a t(4;14) translocation and the presence or the absence of FGFR3 mutation for the method of the invention. The test kit of the invention comprises the reagents mentioned above used for the determination. The test kit of the invention allows prediction of whether or not a patient is highly sensitive to the compound of the invention.

The present invention also relates to use of a test kit for the prediction described above.

Hereinafter, the present invention will be illustrated by way of specific examples, although the invention should not be limited thereto.

### [EXAMPLE 1]

A FGFR3-inhibiting activity of 4-(3-chloro-4-(cyclopropylaminocarbonyl)aminophenoxy)-7-methoxy-6-quinolinecarbox amide was examined by ProQinase (Freiburg, GmbH) on our request. Specifically, the FGFR3-inhibiting activity was determined as follows.

FGFR3 kinase was expressed in an insect cell (*Spondoptea frugiperda* 9 (Sf9)) as human recombinant GST fusion protein by the method of Baculovirus Expression System. The expressed recombinant protein was purified by affinity chromatography using GSH-agarose (from Sigma) or Ni-NTH-agarose (from Qiagen). The purity and identification of the protein were confirmed by SDS-PAGE, silver staining and western blotting using an antibody specific to FGFR3 kinase.

Kinase assay was carried out as follows.

First, to each well of 96-well FlashPlate (from Perkin Elmer/NEM), a mixed solution containing 20 µl of standard reaction solution, 5 µl of ATP solution (diluted with H₂O), 5 µl of the test substance (10% aqueous dimethylsulfoxide solution), 10 µl of solution containing 100 ng of FGFR3 recombinant protein and 10 µl of solution containing 125 ng of biotinylated Poly(Glu, Tyr)_{4:1} was added sequentially.

Herein, methanesulfonate of 4-(3-chloro-4-(cyclopropylaminocarbonyl)aminophenoxy)-7-methoxy-6-quinolinecarbo xamide was used as the test substance. The methanesulfonate of 4-(3-chloro-4-(cyclopropylaminocarbonyl)aminophenoxy)-7-methoxy-6-quinolinecarbo xamide was produced based on the description in International publication No. 02/32872 (pamphlet) (WO02/32872) and International publication No. 2005/063713 (pamphlet) (WO2005/063713)

This kinase reaction solution (50 µl) contained 60 mM HEPES-NaOH (pH7.5), 3 mM MgCl₂, 3 mM MnCl₂, 3 µM Na-orthovanadate, 1.2 mM DTT, 50 µg/ml PEG₂₀₀₀₀ and 1 µM [γ-³³P]-ATP.

The reaction solution was incubated at 30°C for 80 minutes, after which 50 µl of 2% (v/v) H₃PO₄ solution was added to terminate the reaction.

The 96-well plate was washed and suctioned twice with 200 µl of 0.9% (w/v) NaCl solution.

The amount of ³³Pᵢ incorporation was assessed by determining the radioactivity on the plate with a microplate scintillation counter (from Microbeta, Wallac).

The manipulation was performed with a BeckmanCoulter/Sagian robotic system.

The concentration (IC₅₀) of the test substance required for inhibiting a FGFR3 kinase activity by 50% was calculated using radioactivity with respect to ³³P at various concentrations (10 points ranging from 10 µM to 0.0003 µM) using Prism 3.03 (Windows (Registered Trademark), Graphpad, San Diego, California, USA).

In this case, the value obtained for the case where only substrate Poly(Glu, Tyr)_{4:1} (without the addition of FGFR3 protein) was added was assumed 0% while the value obtained for the case where FGFR3 protein and substrate Poly(Glu, Tyr)_{4:1} were added (without the addition of the test substance) was assumed 100%.

The kinase activity in the presence of the test substance at each concentration was assessed as percentage of the value obtained by subtracting the 0% value from the radioactivity value to the value obtained by subtracting the 0% value from the 100% value.

As a result, 4-(3-chloro-4-(cyclopropylaminocarbonyl)aminophenoxy)-7-methoxy-6-quinolinecarbox amide was found to have a FGFR3 kinase-inhibiting activity (IC₅₀ = 140 nM).

As described above, the FGFR3 inhibitor causes cell growth inhibition and apoptosis of at least one cell selected from the group consisting of a cell overexpressing FGFR3, a cell that has a t(4;14) translocation and a cell expressing mutant FGFR3, and thus shows effects (e.g., anti-tumor effect, etc.) on a living organism having these cells.

As described above, the FGFR3 inhibitor appears to be effective against multiple myeloma, bladder cancer, cervical cancer, hypochondroplasia, achondroplasia, thanatophoric dysplasia and skeletal dysplasia.

From these results and findings, the compound of the invention was shown to exert their effects with higher efficiency on a living organism having at least one cell selected from the group consisting of a cell overexpressing FGFR3, a cell that has a t(4;14) translocation and a cell expressing mutant FGFR3.

The compound of the invention was also expected to be more effective against at least one disease selected from the group consisting of multiple myeloma, bladder cancer, cervical cancer, hypochondroplasia, achondroplasia, thanatophoric dysplasia and skeletal dysplasia.

Furthermore, since an effect of the compound of the invention can be predicted without administering the compound to a patient by determining at least one selected from the group consisting of the FGFR3 expression level, the presence or the absence of a t(4;14) translocation and the presence or the absence of FGFR3 mutation in the cell and using at least one or a combination of the FGFR3 expression level, the presence or the absence of a t(4; 14) translocation and the presence or the absence of FGFR3 mutation determined in the cell as an index, patients who are expected to be more susceptible to the compound can be selected, thereby contributing to the QOL of the patient.

### [Reference Example]

Hereinafter, a method for producing a formulation of one of the compounds of the invention,
4-(3-chloro-4-(cyclopropylaminocarbonyl)aminophenoxy)-7-methoxy-6-quinolinecarbox amide will be described as a reference example.

### (Production of pharmaceutical composition)

### (1) 1 mg tablet

24g of crystal (C) of methanesulfonate of 4-(3-chloro-4-(cyclopropylaminocarbonyl)aminophenoxy)-7-methoxy-6-quinolinecarbo xamide (hereinafter, also referred to as "crystal (C)", which was produced according to the method described in Example 7 of W02005/063713) and 192g of light anhydrous silicic acid (antigelling agent sold under the product name of AEROSIL (Registered Trademark) 200, Nippon Aerosil) were mixed with 20L Super Mixer, and then 1236g of D-mannitol (excipient, Towa-Kasei Co., Ltd.), 720g of crystalline cellulose (excipient sold under the product name of Avicel PH101, Asahi Kasei Corporation) and 72g of hydroxypropylcellulose (binder sold under the product name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 120g of croscarmellose sodium (disintegrant sold under the product name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 100 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 105 mg per tablet.

### (2) 10 mg tablet

60g of crystal (C) and 192g of light anhydrous silicic acid (antigelling agent sold under the product name of AEROSIL (Registered Trademark) 200, Nippon Aerosil) were mixed with 20L Super Mixer, and then 1200g of D-mannitol (excipient, Towa-Kasei Co., Ltd.), 720g of crystalline cellulose (excipient sold under the product name of Avicel PH101, Asahi Kasei Corporation) and 72g of hydroxypropylcellulose (binder sold under the product name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 120g of croscarmellose sodium (disintegrant sold under the product name of Ac-Di-Sol, FMC International Inc.) and 36g of sodium stearyl fumarate (lubricant, JRS Pharma LP) were placed and mixed together in a 20L tumbler mixer, and molded with a tablet machine to obtain tablets with a total mass of 400 mg per tablet. Furthermore, the tablets were coated using aqueous 10% Opadry yellow (OPADRY 03F42069 YELLOW, Colorcon Japan) solution as a coating solution with a tablet coating machine, thereby obtaining coated tablets with a total mass of 411 mg per tablet.

### (3) 100 mg tablet

31.4g of crystal (C) and 4g of light anhydrous silicic acid (antigelling agent sold under the product name of AEROSIL (Registered Trademark) 200, Nippon Aerosil) were mixed with 1L Super Mixer, and then 40.1g of anhydrous calcium hydrogen phosphate (excipient, Kyowa Chemical Industry Co., Ltd.), 10g of low substituted hydroxypropylcellulose (binder sold under the product name ofL-HPC (LH-21), Shin-Etsu Chemical Co., Ltd.) and 3g of hydroxypropylcellulose (binder sold under the product name of HPC-L, Nippon Soda Co., Ltd.) were further added and mixed together. Subsequently, a suitable amount of anhydrous ethanol was added to obtain a granulated body containing crystal (C). This granulated body was dried in a rack dryer (60°C), and then size-regulated using PowerMILL to obtain granules. Together with the granules, 10g of croscarmellose sodium (disintegrant sold under the product name of Ac-Di-Sol, FMC International Inc.) and 1.5g of sodium stearyl fumarate (lubricant, JRS Pharma LP) were mixed and molded with a tablet machine to obtain tablets with a total mass of 400 mg per tablet.

### INDUSTRIAL APPLICABILITY

According to the present invention, there is provided a pharmaceutical composition and a therapeutic method which can exert their effects with higher efficiency on a living organism having at least one cell selected from the group consisting of a cell overexpressing FGFR3, a cell that has a t(4;14) translocation and a cell expressing mutant FGFR3.

Specifically, the present invention provides: a pharmaceutical composition comprising a compound of the invention, which is to be administered to a living organism having at least one cell selected from the group consisting of a cell overexpressing FGFR3, a cell that has a t(4;14) translocation and a cell expressing mutant FGFR3; a method for treating a disease comprising administering an effective amount of a compound of the invention to the living organism; use of a compound of the invention for producing the pharmaceutical composition; and a compound of the invention for the pharmaceutical composition.

In addition, the present invention provides: a therapeutic drug and a method for treating at least one disease selected from the group consisting of multiple myeloma, bladder cancer, cervical cancer, hypochondroplasia, achondroplasia, thanatophoric dysplasia and skeletal dysplasia, the therapeutic drug and the method comprising a compound of the invention; use of a compound of the invention for producing the therapeutic drug; and a compound of the invention for the therapeutic drug.

Furthermore, the present invention provides a FGFR3 inhibitor.

Moreover, the present invention provides a method for predicting the effect of a compound of the invention.

More specifically, the effect of the compound of the invention can be predicted by using at least one index selected from the group consisting of the FGFR3 expression level, the presence or the absence of a t(4;14) translocation and the presence or the absence of FGFR3 mutation in the cell.

According to the method of the invention, an effect of a compound can be predicted without administering the compound to a patient by selecting a patient who is expected to be more susceptible to the compound, thereby contributing to the QOL of the patient.

## Claims

1. A pharmaceutical composition comprising a compound represented by General Formula (I) below, a pharmacologically acceptable salt thereof or a solvate thereof which is to be administered to a living organism having at least one cell selected from the group consisting of a cell overexpressing FGFR3, a cell that has a t(4;14) translocation and a cell expressing mutant FGFR3,
General Formula (I) 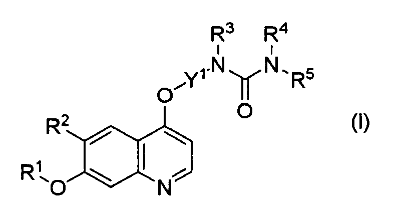 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 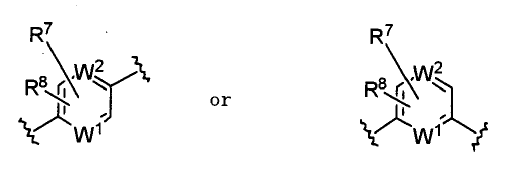 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{a1} and V^{a2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

2. The pharmaceutical composition according to Claim 1, wherein R¹ is C₁₋₆ alkyl group (wherein, R¹ may have at least one substituent selected from the group consisting of 3-10-membered nonaromatic heterocyclic group, hydroxyl group, C₁₋₆ alkoxy group, amino group, mono-C₁₋₆ alkylamino group and di-C₁₋₆ alkylamino group which may have C₁₋₆ alkyl group).

3. The pharmaceutical composition according to Claim 1, wherein R¹ is methyl group or group represented by any one of the following Formulae 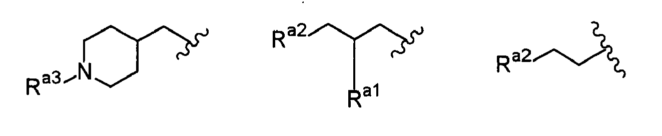 (wherein, R^{a3} represents methyl group; R^{a1} represents a hydrogen atom or hydroxyl group; R^{a2} represents methoxy group, ethoxy group, 1-pyrrolidinyl group, 1-piperidinyl group, 4-morpholinyl group, dimethylamino group or diethylamino group).

4. The pharmaceutical composition according to Claim 1, wherein R¹ is methyl group or 2-methoxyethyl group.

5. The pharmaceutical composition according to Claim 1, wherein R² is cyano group or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent).

6. The pharmaceutical composition according to Claim 1, wherein R² is cyano group or group represented by Formula -CONHV^{a16} (wherein, V^{a16} represents a hydrogen atom, C₁₋₆ alkyl group, C₃₋₈ cycloalkyl group, C₁₋₆ alkoxy group or C₃₋₈ cycloalkoxy group, where V^{a16} may have at least one substituent selected from the group consisting of a halogen atom, cyano group, hydroxyl group and C₁₋₆ alkoxy group).

7. The pharmaceutical composition according to Claim 1, wherein R² is group represented by Formula -CONHV^{a17} (wherein, V^{a17} represents a hydrogen atom, C₁₋₆ alkyl group or C₁₋₆ alkoxy group).

8. The pharmaceutical composition according to Claim 1, wherein R² is group represented by Formula -CONHV^{a18} (wherein, V^{a18} represents a hydrogen atom, methyl group or methoxy group).

9. The pharmaceutical composition according to Claim 1, wherein Y¹ is group represented by Formula 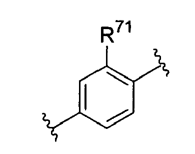 (wherein, R⁷¹ represents a hydrogen atom or a halogen atom).

10. The pharmaceutical composition according to Claim 1, wherein R³ and R⁴ represent a hydrogen atom.

11. The pharmaceutical composition according to Claim 1, wherein R⁵ is a hydrogen atom, C₁₋₆ alkyl group, C₃₋₈ cycloalkyl group or C₆₋₁₀ aryl group (where R⁵ may have at least one substituent selected from the group consisting of a halogen atom and methanesulfonyl group).

12. The pharmaceutical composition according to Claim 1, wherein R⁵ is methyl group, ethyl group or cyclopropyl group.

13. The pharmaceutical composition according to Claim 1, wherein the compound represented by General Formula (I), a pharmacologically acceptable salt thereof or a solvate thereof is at least one compound selected from the group consisting of:
N-(4-(6-cyano-7-(2-methoxyethoxy)-4-quinolyl)oxy-2-fluorophenyl)-N'-(4-fluorophenyl)urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl) -N'-(4-fluorophenyl)urea;
N-(4-((6-cyano-7-(((2R)-2-hydroxy-3-(1-pyrrolidino)propyl)oxy)-4-quinolyl)oxy)phenyl) -N'-(4-fluorophenyl)urea;
4-(3-chloro-4-(cyclopropylaminocarbonyl)aminophenoxy)-7-methoxy-6-quinolinecarbox amide;
4-(3-chloro-4-(cyclopropylaminocrbonyl)aminophenoxy)-7-(2-methoxyethoxy)-6-quino linecarboxamide;
N6-cyclopropyl-4-(3-chloro-4-(((cyclopropylamino)carbonyl)amino)phenoxy)-7-methox y-6-quinolinecarboxamide;
N6-(2-methoxyethyl)-4-(3-chloro-4-(((cyclopropylamino)carbonyl)amino)phenoxy)-7-m ethoxy-6-quinolinecarboxamide;
N6-(2-fluoroethyl)-4-(3-chloro-4-(((cyclopropylamino)carbonyl)amino)phenoxy)-7-meth oxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-q uinolinecarboxamide;
N6-ethyl-4-(3-chloro-4-(((cyclopropylamino)carbonyl)amino)phenoxy)-7-methoxy-6-qui nolinecarboxamide;
4-(3-fluoro-4-(cyclopropylaminocarbonyl)aminophenoxy)-7-(2-methoxyethoxy)-6-quinol inecarboxamide;
4-(3-chloro-4-(cyclopropylaminocarbonyl)aminophenoxy)-7-(2-hydroxyethoxy)-6-quinol inecarboxamide;
4-(3-chloro-4-(cyclopropylaminocarbonyl)aminophenoxy)-7-((2S)-2,3-dihydroxypropyl) oxy-6-quinolinecarboxamide;
4-(3-chloro-4-(methylaminocarbonyl)aminophenoxy)-7-methoxy-6-quinolinecarboxamid e;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinol inecarboxamide;
4-(3-chloro-4-(cyclopropylaminocarbonyl)aminophenoxy)-7-(2-ethoxyethoxy)-6-quinoli necarboxamide;
4-(4-((cyclopropylamino)carbonyl)aminophenoxy)-7-(2-methoxyethoxy)-6-quinolinecarb oxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-me thoxy-6-quinolinecarboxamide;
4-(3-chloro-4-(1-propylaminocarbonyl)aminophenoxy)-7-methoxy-6-quinolinecarboxami de;
4-(3-chloro-4-(cis-2-fluoro-cyclopropylaminocarbonyl)aminophenoxy)-7-methoxy-6-qui nolinecarboxamide;
N6-methyl-4-(3-chloro-4-(((cyclopropylamino)carbonyl)amino)phenoxy)-7-(2-methoxyet hoxy)-6-quinolinecarboxamide;
N6-methyl-4-(3-chloro-4-(((ethylamino)carbonyl)amino)phenoxy)-7-methoxy-6-quinolin ecarboxamide;
4-(3-chloro-4-(cyclopropylaminocarbonyl)aminophenoxy)-7-(2-(4-morpholino)ethoxy)-6 -quinolinecarboxamide;
4-(3-chloro-4-(2-fluoroethylaminocarbonyl)aminophenoxy)-7-methoxy-6-quinolinecarbo xamide;
N6-((2R)tetrahydro-2-furanylmethyl)-4-(3-chloro-4-(((methylamino)carbonyl)amino)phe noxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-py rrolidino)propoxy)-6-quinolinecarboxamide;
N6-methyl-4-(3-chloro-4-(((methylamino)carbonyl)amino)phenoxy)-7-((2R)-3-diethylam ino-2-hydroxypropoxy)-6-quinolinecarboxamide;
N6-methyl-4-(3-chloro-4-(((ethylamino)carbonyl)amino)phenoxy)-7-((2R)-3-diethylamin o-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-( 1-pyrrolidino)propoxy)-6-quinolinecarboxamide;
N6-methyl-4-(3-chloro-4-(((methylamino)carbonyl)amino)phenoxy)-7-((1-methyl-4-pipe ridyl)methoxy)-6-quinolinecarboxamide;
N6-methyl-4-(3-chloro-4-(((ethylamino)carbonyl)amino)phenoxy)-7-((1-methyl-4-piperi dyl)methoxy)-6-quinolinecarboxamide;
N-(4-(6-cyano-7-(2-methoxyethoxy)-4-quinolyl)oxy-2-fluorophenyl)-N'-cyclopropylurea ;
N-(4-(6-cyano-7-(3-(4-morpholino)propoxy)-4-quinolyl)oxyphenyl)-N'-(3-(methylsulfon yl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarb oxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopr opylurea,
a pharmacologically acceptable salt thereof or a solvate thereof.

14. The pharmaceutical composition according to Claim 1, wherein the compound represented by General Formula (I), a pharmacologically acceptable salt thereof or a solvate thereof is at least one compound selected from the group consisting of:
4-(3-chloro-4-(cyclopropylaminocarbonyl)aminophenoxy)-7-methoxy-6-quinolinecarboxamide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamid e; and
N6-methoxy-4-(3-chloro-4-(((ethylamino)carbonyl)amino)phenoxy)-7-methoxy-6-quinol inecarboxamide,
a pharmacologically acceptable salt thereof or a solvate thereof.

15. The pharmaceutical composition according to Claim 1, wherein the compound represented by General Formula (I), a pharmacologically acceptable salt thereof or a solvate thereof is
4-(3-chloro-4-(cyclopropylaminocarbonyl)aminophenoxy)-7-methoxy-6-quinolinecarbox amide), a pharmacologically acceptable salt thereof or a solvate thereof.

16. The pharmaceutical composition according to Claim 1, wherein the compound represented by General Formula (I), a pharmacologically acceptable salt thereof or a solvate thereof is methanesulfonate of
4-(3-chloro-4-(cyclopropylaminocarbonyl)aminophenoxy)-7-methoxy-6-quinolinecarbox amide.

17. The pharmaceutical composition according to any one of Claims 1-16, wherein mutant FGFR3 comprises a mutation site where at least one amino acid selected from the group consisting of codons 248, 249, 370, 371, 373, 380, 384, 391 and 650 in the amino acid sequence represented by SEQ ID NO: 2 is substituted with other amino acid.

18. The pharmaceutical composition according to any one of Claims 1-16, wherein mutant FGFR3 is a polypeptide comprising at least one mutation selected from the group consisting of R248C, S249C, G370C, S371C, Y373C, G380R, F384L, A391E, K650E, K650M, K650Q and K650T in the amino acid sequence represented by SEQ ID NO: 2.

19. The pharmaceutical composition according to any one of Claims 1-16, wherein the cell is a multiple myeloma cell.

20. The pharmaceutical composition according to any one of Claims 1-16, wherein the living organism is a patient suffering from at least one disease selected from the group consisting of multiple myeloma, bladder cancer, cervical cancer, hypochondroplasia, achondroplasia, thanatophoric dysplasia and skeletal dysplasia.

21. A therapeutic drug for treating multiple myeloma, comprising a compound represented by General Formula (I) below, a pharmacologically acceptable salt thereof or a solvate thereof,
General Formula (I) 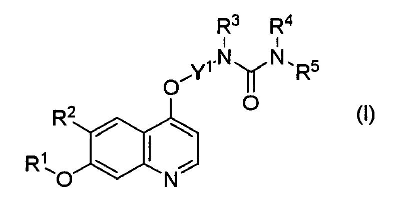 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 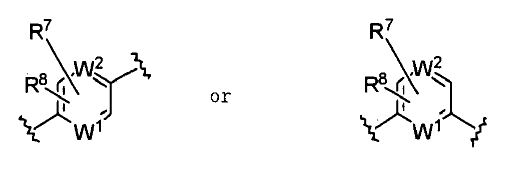 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

22. The therapeutic drug according to Claim 21, wherein R¹ is C₁₋₆ alkyl group (wherein, R¹ may have at least one substituent selected from the group consisting of 3-10-membered nonaromatic heterocyclic group, hydroxyl group, C₁₋₆ alkoxy group, amino group, mono-C₁₋₆ alkylamino group and di-C₁₋₆ alkylamino group which may have C₁₋₆ alkyl group).

23. The therapeutic drug according to Claim 21, wherein R¹ is methyl group or group represented by any one of the following Formulae 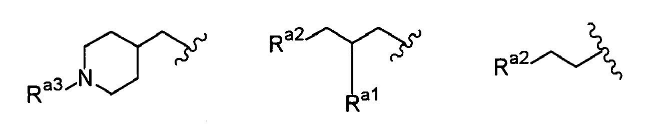 (wherein, R^{a3} represents methyl group; R^{a1} represents a hydrogen atom or hydroxyl group; R^{a2} represents methoxy group, ethoxy group, 1-pyrrolidinyl group, 1-piperidinyl group, 4-morpholinyl group, dimethylamino group or diethylamino group).

24. The therapeutic drug according to Claim 21, wherein R¹ is methyl group or 2-methoxyethyl group.

25. The therapeutic drug according to Claim 21, wherein R² is cyano group or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent).

26. The therapeutic drug according to Claim 21, wherein R² is cyano group or group represented by Formula -CONHV^{a16} (wherein, V^{a16} represents a hydrogen atom, C₁₋₆ alkyl group, C₃₋₈ cycloalkyl group, C₁₋₆ alkoxy group or C₃₋₈ cycloalkoxy group, where V^{a16} may have at least one substituent selected from the group consisting of a halogen atom, cyano group, hydroxyl group and C₁₋₆ alkoxy group).

27. The therapeutic drug according to Claim 21, wherein R² is group represented by Formula -CONHV^{a17} (wherein, V^{a17} represents a hydrogen atom, C₁₋₆ alkyl group or C₁₋₆ alkoxy group).

28. The therapeutic drug according to Claim 21, wherein R² is group represented by Formula -CONHV^{a18} (wherein, V^{a18} represents a hydrogen atom, methyl group or methoxy group).

29. The therapeutic drug according to Claim 21, wherein Y¹ is group represented by Formula 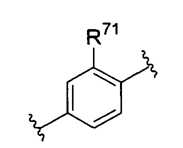 (wherein, R⁷¹ represents a hydrogen atom or a halogen atom).

30. The therapeutic drug according to Claim 21, wherein R³ and R⁴ represent a hydrogen atom.

31. The therapeutic drug according to Claim 21, wherein R⁵ is a hydrogen atom, C₁₋₆ alkyl group, C₃₋₈ cycloalkyl group or C₆₋₁₀ aryl group (where R⁵ may have at least one substituent selected from the group consisting of a halogen atom and methanesulfonyl group).

32. A therapeutic drug according to Claim 21, wherein R⁵ is methyl group, ethyl group or cyclopropyl group.

33. The therapeutic drug according to Claim 21, wherein the compound represented by General Formula (I), a pharmacologically acceptable salt thereof or a solvate thereof is at least one compound selected from the group consisting of:
N-(4-(6-cyano-7-(2-methoxyethoxy)-4-quinolyl)oxy-2-fluorophenyl)-N'-(4-fluorophenyl )urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl) -N'-(4-fluorophenyl)urea;
N-(4-((6-cyano-7-(((2R)-2-hydroxy-3-(1-pyrrolidino)propyl)oxy)-4-quinolyl)oxy)phenyl) -N'-(4-fluorophenyl)urea;
4-(3-chloro-4-(cyclopropylaminocarbonyl)aminophenoxy)-7-methoxy-6-quinolinecarbox amide;
4-(3-chloro-4-(cyclopropylaminocarbonyl)aminophenoxy)-7-(2-methoxyethoxy)-6-quino linecarboxamide;
N6-cyclopropyl-4-(3-chloro-4-(((cyclopropylamino)carbonyl)amino)phenoxy)-7-methox y-6-quinolinecarboxamide;
N6-(2-methoxyethyl)-4-(3-chloro-4-(((cyclopropylamino)carbonyl)amino)phenoxy)-7-m ethoxy-6-quinolinecarboxamide;
N6-(2-fluoroethyl)-4-(3-chloro-4-(((cyclopropylamino)carbonyl)amino)phenoxy)-7-meth oxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-q uinolinecarboxamide;
N6-ethyl-4-(3-chloro-4-(((cyclopropylamino)carbonyl)amino)phenoxy)-7-methoxy-6-qui nolinecarboxamide;
4-(3-fluoro-4-(cyclopropylaminocarbonyl)aminophenoxy)-7-(2-methoxyethoxy)-6-quinol inecarboxamide;
4-(3-chloro-4-(cyclopropylaminocarbonyl)aminophenoxy)-7-(2-hydroxyethoxy)-6-quinol inecarboxamide;
4-(3-chloro-4-(cyclopropylaminocarbonyl)aminophenoxy)-7-((2S)-2,3-dihydroxypropyl) oxy-6-quinolinecarboxamide;
4-(3-chloro-4-(methylaminocarbonyl)aminophenoxy)-7-methoxy-6-quinolinecarboxamid e;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinol inecarboxamide;
4-(3-chloro-4-(cyclopropylaminocarbonyl)aminophenoxy)-7-(2-ethoxyethoxy)-6-quinoli necarboxamide;
4-(4-((cyclopropylamino)carbonyl)aminophenoxy)-7-(2-methoxyethoxy)-6-quinolinecarb oxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-me thoxy-6-quinolinecarboxamide;
4-(3-chloro-4-(1-propylaminocarbonyl)aminophenoxy)-7-methoxy-6-quinolinecarboxami de;
4-(3-chloro-4-(cis-2-fluoro-cyclopropylaminocarbonyl)aminophenoxy)-7-methoxy-6-qui nolinecarboxamide;
N6-methyl-4-(3-chloro-4-(((cyclopropylamino)carbonyl)amino)phenoxy)-7-(2-methoxyet hoxy)-6-quinolinecarboxamide;
N6-methyl-4-(3-chloro-4-(((ethylamino)carbonyl)amino)phenoxy)-7-methoxy-6-quinolin ecarboxamide;
4-(3-chloro-4-(cyclopropylaminocarbonyl)aminophenoxy)-7-(2-(4-morpholino)ethoxy)-6 -quinolinecarboxamide;
4-(3-chloro-4-(2-fluoroethylaminocarbonyl)aminophenoxy)-7-methoxy-6-quinolinecarbo xamide;
N6-((2R)tetrahydro-2-furanylmethyl)-4-(3-chloro-4-(((methylamino)carbonyl)amino)phe noxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-py rrolidino)propoxy)-6-quinolinecarboxamide;
N6-methyl-4-(3-chloro-4-(((methylamino)carbonyl)amino)phenoxy)-7-((2R)-3-diethylam ino-2-hydroxypropoxy)-6-quinolinecarboxamide; N6-methyl-4-(3-chloro-4-(((ethylamino)carbonyl)amino)phenoxy)-7-((2R)-3-diethylamin o-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-( 1-pyrrolidino)propoxy)-6-quinolinecarboxamide;
N6-methyl-4-(3-chloro-4-(((methylamino)carbonyl)amino)phenoxy)-7-((1-methyl-4-pipe ridyl)methoxy)-6-quinolinecarboxamide;
N6-methyl-4-(3-chloro-4-(((ethylamino)carbonyl)amino)phenoxy)-7-((1-methyl-4-piperi dyl)methoxy)-6-quinolinecarboxamide;
N-(4-(6-cyano-7-(2-methoxyethoxy)-4-quinolyl)oxy-2-fluorophenyl)-N'-cyclopropylurea ;
N-(4-(6-cyano-7-(3-(4-morpholino)propoxy)-4-quinolyl)oxyphenyl)-N'-(3-(methylsulfon yl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarb oxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopr opylurea,
a pharmacologically acceptable salt thereof or a solvate thereof.

34. The therapeutic drug according to Claim 21, wherein the compound represented by General Formula (I), a pharmacologically acceptable salt thereof or a solvate thereof is at least one compound selected from the group consisting of:
4-(3-chloro-4-(cyclopropylaminocarbonyl)aminophenoxy)-7-methoxy-6-quinolinecarbox amide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamid e; and
N6-methoxy-4-(3-chloro-4-(((ethylamino)carbonyl)amino)phenoxy)-7-methoxy-6-quinol inecarboxamide,
a pharmacologically acceptable salt thereof or a solvate thereof.

35. The therapeutic drug according to Claim 21, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarbox amide), a pharmacologically acceptable salt thereof or a solvate thereof.

36. The therapeutic drug according to Claim 21, wherein the compound represented by General Formula (I), a pharmacologically acceptable salt thereof or a solvate thereof is methanesulfonate of
4-(3-chloro-4-(cyclopropylaminocarbonyl)aminophenoxy)-7-methoxy-6-quinolinecarbox amide.

37. The therapeutic drug according to any one of Claims 21-36, wherein multiple myeloma comprises at least one cell selected from the group consisting of a cell overexpressing FGFR3, a cell that has a t(4;14) translocation and a cell expressing mutant FGFR3.

38. The therapeutic drug according to Claim 37, wherein mutant FGFR3 comprises a mutation site where at least one amino acid selected from the group consisting of codons 248, 249, 370, 371, 373, 380, 384, 391 and 650 in the amino acid sequence represented by SEQ ID NO: 2 is substituted with other amino acid.

39. The therapeutic drug according to Claim 37, wherein mutant FGFR3 is a polypeptide comprising at least one mutation selected from the group consisting of R248C, S249C, G370C, S371C, Y373C, G380R, F384L, A391E, K650E, K650M, K650Q and K650T in the amino acid sequence represented by SEQ ID NO: 2.

40. A therapeutic drug for treating at least one disease selected from the group consisting of bladder cancer, cervical cancer, hypochondroplasia, achondroplasia, thanatophoric dysplasia and skeletal dysplasia, the drug comprising a compound represented by General Formula (I) below, a pharmacologically acceptable salt thereof or a solvate thereof,
General Formula (I) 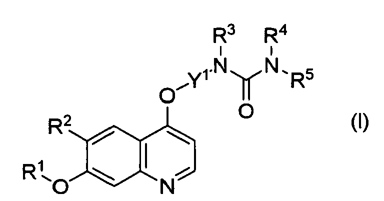 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 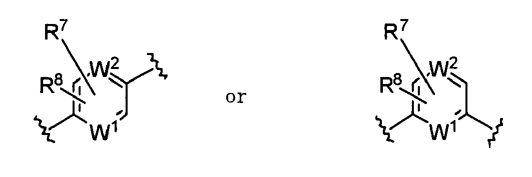 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

41. The therapeutic drug according to Claim 40, wherein R¹ is C₁₋₆ alkyl group (wherein, R¹ may have at least one substituent selected from the group consisting of 3-10-membered nonaromatic heterocyclic group, hydroxyl group, C₁₋₆ alkoxy group, amino group, mono-C₁₋₆ alkylamino group and di-C₁₋₆ alkylamino group which may have C₁₋₆ alkyl group).

42. The therapeutic drug according to Claim 40, wherein R¹ is methyl group or group represented by any one of the following Formulae 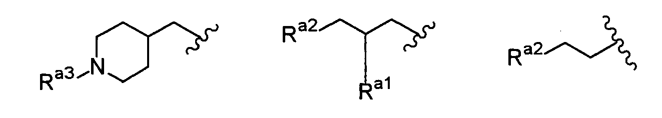 (wherein, R^{a3} represents methyl group; R^{a1} represents a hydrogen atom or hydroxyl group; R^{a2} represents methoxy group, ethoxy group, 1-pyrrolidinyl group, 1-piperidinyl group, 4-morpholinyl group, dimethylamino group or diethylamino group).

43. The therapeutic drug according to Claim 40, wherein R¹ is methyl group or 2-methoxyethyl group.

44. The therapeutic drug according to Claim 40, wherein R² is cyano group or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent).

45. The therapeutic drug according to Claim 40, wherein R² is cyano group or group represented by Formula -CONHV^{a16} (wherein, V^{a16} represents a hydrogen atom, C₁₋₆ alkyl group, C₃₋₈ cycloalkyl group, C₁₋₆ alkoxy group or C₃₋₈ cycloalkoxy group, where V^{a16} may have at least one substituent selected from the group consisting of a halogen atom, cyano group, hydroxyl group and C₁₋₆ alkoxy group).

46. The therapeutic drug according to Claim 40, wherein R² is group represented by Formula -CONHV^{a17} (wherein, V^{a17} represents a hydrogen atom, C₁₋₆ alkyl group or C₁₋₆ alkoxy group).

47. The therapeutic drug according to Claim 40, wherein R² is group represented by Formula -CONHV^{a18} (wherein, V^{a18} represents a hydrogen atom, methyl group or methoxy group).

48. The therapeutic drug according to Claim 40, wherein Y¹ is group represented by Formula 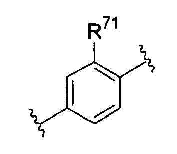 (wherein, R⁷¹ represents a hydrogen atom or a halogen atom).

49. The therapeutic drug according to Claim 40, wherein R³ and R⁴ represent a hydrogen atom.

50. The therapeutic drug according to Claim 40, wherein R⁵ is a hydrogen atom, C₁₋₆ alkyl group, C₃₋₈ cycloalkyl group or C₆₋₁₀ aryl group (where R⁵ may have at least one substituent selected from the group consisting of a halogen atom and methanesulfonyl group).

51. The therapeutic drug according to Claim 40, wherein R⁵ is methyl group, ethyl group or cyclopropyl group.

52. The therapeutic drug according to Claim 40, wherein the compound represented by General Formula (I), a pharmacologically acceptable salt thereof or a solvate thereof is at least one compound selected from the group consisting of:
N-(4-(6-cyano-7-(2-methoxyethoxy)-4-quinolyl)oxy-2-fluorophenyl)-N'-(4-fluorophenyl )urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl) -N'-(4-fluorophenyl)urea;
N-(4-((6-cyano-7-(((2R)-2-hydroxy-3-(1-pyrrolidino)propyl)oxy)-4-quinolyl)oxy)phenyl) -N'-(4-fluorophenyl)urea;
4-(3-chloro-4-(cyclopropylaminocarbonyl)aminophenoxy)-7-methoxy-6-quinolinecarbox amide;
4-(3-chloro-4-(cyclopropylaminocarbonyl)aminophenoxy)-7-(2-methoxyethoxy)-6-quino linecarboxamide;
N6-cyclopropyl-4-(3-chloro-4-(((cyclopropylamino)carbonyl)amino)phenoxy)-7-methox y-6-quinolinecarboxamide;
N6-(2-methoxyethyl)-4-(3-chloro-4-(((cyclopropylamino)carbonyl)amino)phenoxy)-7-m ethoxy-6-quinolinecarboxamide;
N6-(2-fluoroethyl)-4-(3-chloro-4-(((cyclopropylamino)carbonyl)amino)phenoxy)-7-meth oxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-q uinolinecarboxamide;
N6-ethyl-4-(3-chloro-4-(((cyclopropylamino)carbonyl)amino)phenoxy)-7-methoxy-6-qui nolinecarboxamide;
4-(3-fluoro-4-(cyclopropylaminocarbonyl)aminophenoxy)-7-(2-methoxyethoxy)-6-quinol inecarboxamide;
4-(3-chloro-4-(cyclopropylaminocarbonyl)aminophenoxy)-7-(2-hydroxyethoxy)-6-quinol inecarboxamide;
4-(3-chloro-4-(cyclopropylaminocarbonyl)aminophenoxy)-7-((2S)-2,3-dihydroxypropyl) oxy-6-quinolinecarboxamide;
4-(3-chloro-4-(methylaminocarbonyl)aminophenoxy)-7-methoxy-6-quinolinecarboxamid e;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinol inecarboxamide;
4-(3-chloro-4-(cyclopropylaminocarbonyl)aminophenoxy)-7-(2-ethoxyethoxy)-6-quinoli necarboxamide;
4-(4-((cyclopropylamino)carbonyl)aminophenoxy)-7-(2-methoxyethoxy)-6-quinolinecarb oxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-me thoxy-6-quinolinecarboxamide;
4-(3-chloro-4-(1-propylaminocarbonyl)aminophenoxy)-7-methoxy-6-quinolinecarboxami de;
4-(3-chloro-4-(cis-2-fluoro-cyclopropylaminocarbonyl)aminophenoxy)-7-methoxy-6-qui nolinecarboxamide;
N6-methyl-4-(3-chloro-4-(((cyclopropylamino)carbonyl)amino)phenoxy)-7-(2-methoxyet hoxy)-6-quinolinecarboxamide;
N6-methyl-4-(3-chloro-4-(((ethylamino)carbonyl)amino)phenoxy)-7-methoxy-6-quinolin ecarboxamide;
4-(3-chloro-4-(cyclopropylaminocarbonyl)aminophenoxy)-7-(2-(4-morpholino)ethoxy)-6 -quinolinecarboxamide;
4-(3-chloro-4-(2-fluoroethylaminocarbonyl)aminophenoxy)-7-methoxy-6-quinolinecarbo xamide;
N6-((2R)tetrahydro-2-furanylmethyl)-4-(3-chloro-4-(((methylamino)carbonyl)amino)phe noxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-py rrolidino)propoxy)-6-quinolinecarboxamide;
N6-methyl-4-(3-chloro-4-(((methylamino)carbonyl)amino)phenoxy)-7-((2R)-3-diethylam ino-2-hydroxypropoxy)-6-quinolinecarboxamide;
N6-methyl-4-(3-chloro-4-(((ethylamino)carbonyl)amino)phenoxy)-7-((2R)-3-diethylamin o-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-( 1-pyrrolidino)propoxy)-6-quinolinecarboxamide;
N6-methyl-4-(3-chloro-4-(((methylamino)carbonyl)amino)phenoxy)-7-((1-methyl-4-pipe ridyl)methoxy)-6-quinolinecarboxamide;
N6-methyl-4-(3-chloro-4-(((ethylamino)carbonyl)amino)phenoxy)-7-((1-methyl-4-piperi dyl)methoxy)-6-quinolinecarboxamide;
N-(4-(6-cyano-7-(2-methoxyethoxy)-4-quinolyl)oxy-2-fluorophenyl)-N'-cyclopropylurea ;
N-(4-(6-cyano-7-(3-(4-morpholino)propoxy)-4-quinolyl)oxyphenyl)-N'-(3-(methylsulfon yl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarb oxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopr opylurea,
a pharmacologically acceptable salt thereof or a solvate thereof.

53. The therapeutic drug according to Claim 40, wherein the compound represented by General Formula (I), a pharmacologically acceptable salt thereof or a solvate thereof is at least one compound selected from the group consisting of:
4-(3-chloro-4-(cyclopropylaminocarbonyl)aminophenoxy)-7-methoxy-6-quinolinecarbox amide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamid e; and
N6-methoxy-4-(3-chloro-4-(((ethylamino)carbonyl)amino)phenoxy)-7-methoxy-6-quinol inecarboxamide,
a pharmacologically acceptable salt thereof or a solvate thereof.

54. The therapeutic drug according to Claim 40, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarbox amide), a pharmacologically acceptable salt thereof or a solvate thereof.

55. The therapeutic drug according to Claim 40, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarbox amide.

56. The therapeutic drug according to any one of Claims 40-55, wherein the disease is a disease comprising at least one cell selected from the group consisting of a cell overexpressing FGFR3, a cell that has a t(4;14) translocation and a cell expressing mutant FGFR3.

57. The therapeutic drug according to Claim 56, wherein mutant FGFR3 comprises a mutation site where at least one amino acid selected from the group consisting of codons 248, 249, 370, 371, 373, 380, 384, 391 and 650 in the amino acid sequence represented by SEQ ID NO: 2 is substituted with other amino acid.

58. The therapeutic drug according to Claim 56, wherein mutant FGFR3 is a polypeptide comprising at least one mutation selected from the group consisting of R248C, S249C, G370C, S371C, Y373C, G380R, F384L, A391E, K650E, K650M, K650Q and K650T in the amino acid sequence represented by SEQ ID NO: 2.

59. A method for treating a disease comprising administering an effective amount of a compound represented by General Formula (I) below, a pharmacologically acceptable salt thereof or a solvate thereof to a living organism having at least one cell selected from the group consisting of a cell overexpressing FGFR3, a cell that has a t(4;14) translocation and a cell expressing mutant FGFR3
General Formula (I) 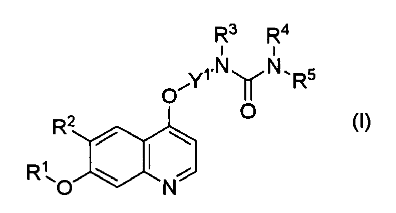 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, . C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 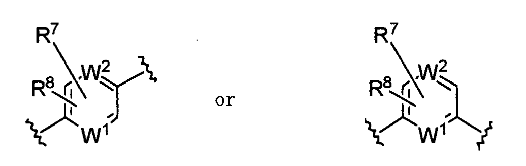 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent].

60. A method for treating multiple myeloma comprising administering an effective amount of a compound represented by General Formula (I) below, a pharmacologically acceptable salt thereof or a solvate thereof to a patient,
General Formula (I) 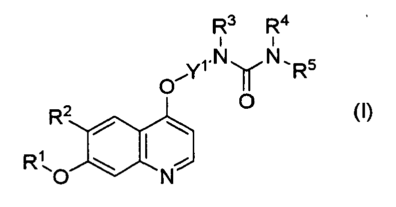 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁-₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 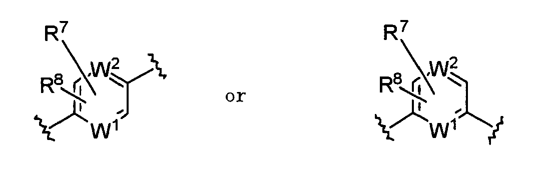 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

61. A method for treating at least one disease selected from the group consisting of bladder cancer, cervical cancer, hypochondroplasia, achondroplasia, thanatophoric dysplasia (TD) and skeletal dysplasia, comprising administering an effective amount of a compound represented by General Formula (I) below, a pharmacologically acceptable salt thereof or a solvate thereof to a patient
General Formula (I) 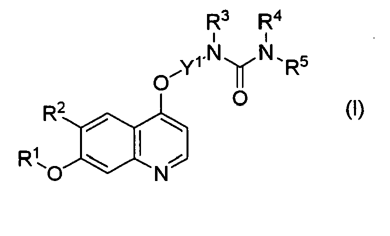 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 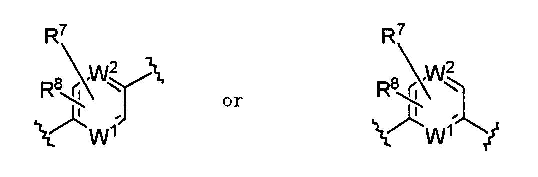 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

62. Use of a compound represented by General Formula (I) below, a pharmacologically acceptable salt thereof or a solvate thereof for producing a pharmaceutical composition which is to be administered to a living organism having at least one cell selected from the group consisting of a cell overexpressing FGFR3, a cell that has a t(4;14) translocation and a cell expressing mutant FGFR3,
General Formula (I) 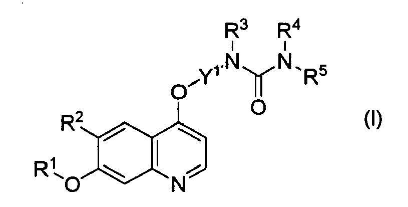 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 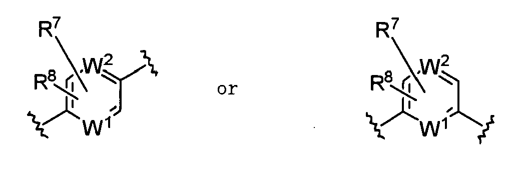 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

63. Use of a compound represented by General Formula (I) below, a pharmacologically acceptable salt thereof or a solvate thereof for producing a therapeutic drug for treating multiple myeloma
General Formula (I) 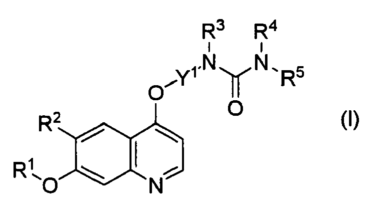 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 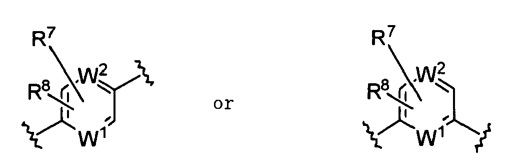 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

64. Use of a compound represented by General Formula (I) below, a pharmacologically acceptable salt thereof or a solvate thereof for producing a therapeutic drug for treating at least one disease selected from the group consisting of bladder cancer, cervical cancer, hypochondroplasia, achondroplasia, thanatophoric dysplasia and skeletal dysplasia,
General Formula (I) 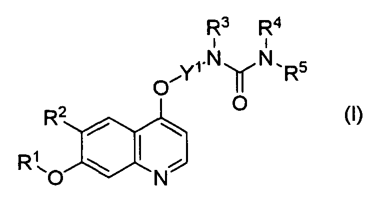 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 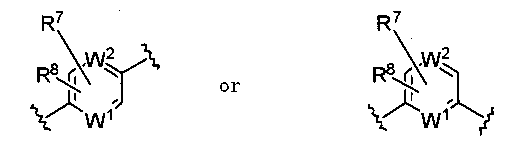 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

65. A compound represented by General Formula (I) below, a pharmacologically acceptable salt thereof or a solvate thereof for a pharmaceutical composition which is to be administered to a living organism having at least one cell selected from the group consisting of a cell overexpressing FGFR3, a cell that has a t(4;14) translocation and a cell expressing mutant FGFR3,
General Formula (I) 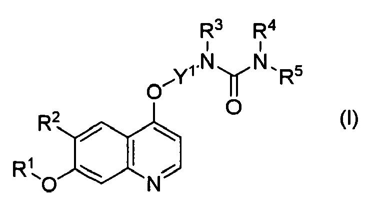 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 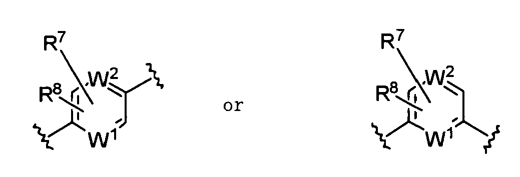 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CON-V^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

66. A compound represented by General Formula (I) below, a pharmacologically acceptable salt thereof or a solvate thereof for a therapeutic drug for treating multiple myeloma,
General Formula (I) 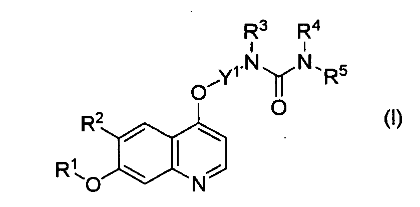 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 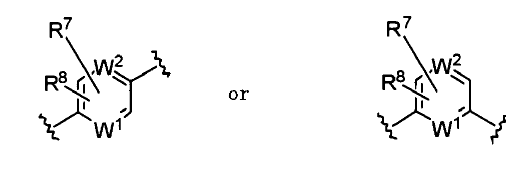 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁-₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

67. A compound represented by General Formula (I) below, a pharmacologically acceptable salt thereof or a solvate thereof for a therapeutic drug for treating at least one disease selected from the group consisting of bladder cancer, cervical cancer, hypochondroplasia, achondroplasia, thanatophoric dysplasia and skeletal dysplasia,
General Formula (I) 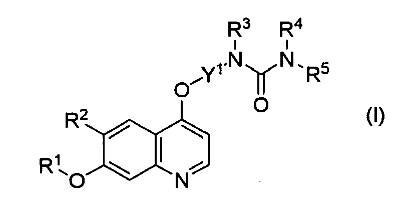 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 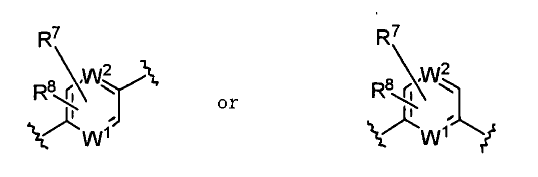 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

68. A method for predicting whether or not a patient is highly sensitive to a compound represented by General Formula (I) below, a pharmacologically acceptable salt thereof or a solvate thereof, the method comprising using at least one index selected from the group consisting of the FGFR3 expression level, the presence or the absence of a t(4;14) translocation and the presence or the absence of FGFR3 mutation in the cell, 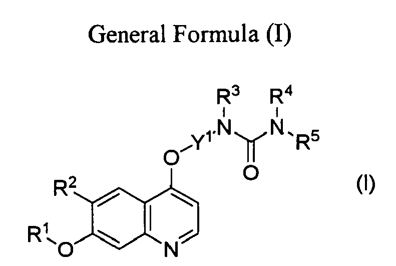 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 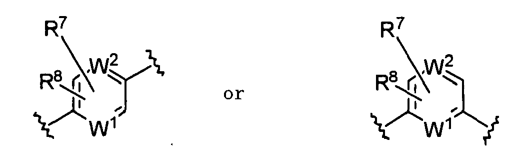 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

69. The method according to Claim 68, wherein R¹ is C₁₋₆ alkyl group (wherein, R¹ may have at least one substituent selected from the group consisting of 3-10-membered nonaromatic heterocyclic group, hydroxyl group, C₁₋₆ alkoxy group, amino group, mono-C₁₋₆ alkylamino group and di-C₁₋₆ alkylamino group which may have C₁₋₆ alkyl group).

70. The method according to Claim 68, wherein R¹ is methyl group or group represented by any one of the following Formulae 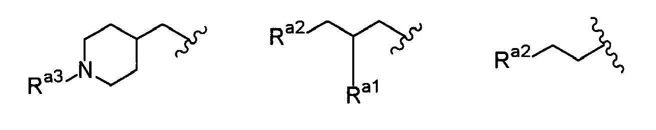 (wherein, R^{a3} represents methyl group; R^{a1} represents a hydrogen atom or hydroxyl group; R^{a2} represents methoxy group, ethoxy group, 1-pyrrolidinyl group, 1-piperidinyl group, 4-morpholinyl group, dimethylamino group or diethylamino group).

71. The method according to Claim 68, wherein R¹ is methyl group or 2-methoxyethyl group.

72. The method according to Claim 68, wherein R² is cyano group or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ acryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent).

73. The method according to Claim 68, wherein R² is cyano group or group represented by Formula -CONHV^{a16} (wherein, V^{a16} represents a hydrogen atom, C₁₋₆ alkyl group, C₃₋₈ cycloalkyl group, C₁₋₆ alkoxy group or C₃₋₈ cycloalkoxy group, where V^{a16} may have at least one substituent selected from the group consisting of a halogen atom, cyano group, hydroxyl group and C₁₋₆ alkoxy group).

74. The method according to Claim 68, wherein R² is group represented by Formula -CONHV^{a17} (wherein, V^{a17} represents a hydrogen atom, C₁₋₆ alkyl group or C₁₋₆ alkoxy group).

75. The method according to Claim 68, wherein R² is group represented by Formula -CONHV^{a18} (wherein, V^{a18} represents a hydrogen atom, methyl group or methoxy group).

76. The method according to Claim 68, wherein Y¹ is group represented by Formula 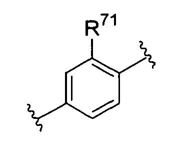 (wherein, R⁷¹ represents a hydrogen atom or a halogen atom).

77. The method according to Claim 68, wherein R³ and R⁴ represent a hydrogen atom.

78. The method according to Claim 68, wherein R⁵ is a hydrogen atom, C₁₋₆ alkyl group, C₃₋₈ cycloalkyl group or C₆₋₁₀ aryl group (where R⁵ may have at least one substituent selected from the group consisting of a halogen atom and methanesulfonyl group).

79. The method according to Claim 68, wherein R⁵ is methyl group, ethyl group or cyclopropyl group.

80. The method according to Claim 68, wherein the compound represented by General Formula (I), a pharmacologically acceptable salt thereof or a solvate thereof is at least one compound selected from the group consisting of:
N-(4-(6-cyano-7-(2-methoxyethoxy)-4-quinolyl)oxy-2-fluorophenyl)-N'-(4-fluorophenyl )urea;
N-(2-chloro-4-((6-cyano-7-((1-methyl-4-piperidyl)methoxy)-4-quinolyl)oxy)phenyl)-N'-cyclopropylurea;
N-(4-((6-cyano-7-(((2R)-3-(diethylamino)-2-hydroxypropyl)oxy)-4-quinolyl)oxy)phenyl) -N'-(4-fluorophenyl)urea;
N-(4-((6-cyano-7-(((2R)-2-hydroxy-3-(1-pyrrolidino)propyl)oxy)-4-quinolyl)oxy)phenyl) -N'-(4-fluorophenyl)urea;
4-(3-chloro-4-(cyclopropylaminocarbonyl)aminophenoxy)-7-methoxy-6-quinolinecarbox amide;
4-(3-chloro-4-(cyclopropylaminocarbonyl)aminophenoxy)-7-(2-methoxyethoxy)-6-quino linecarboxamide;
N6-cyclopropyl-4-(3-chloro-4-(((cyclopropylamino)carbonyl)amino)phenoxy)-7-methox y-6-quinolinecarboxamide;
N6-(2-methoxyethyl)-4-(3-chloro-4-(((cyclopropylamino)carbonyl)amino)phenoxy)-7-m ethoxy-6-quinolinecarboxamide;
N6-(2-fluoroethyl)-4-(3-chloro-4-(((cyclopropylamino)carbonyl)amino)phenoxy)-7-meth oxy-6-quinolinecarboxamide;
N6-methoxy-4-(3-chloro-4-(((cyclopropylamino)carbonyl)amino)phenoxy)-7-methoxy-6-quinolinecarboxamide;
N6-methyl-4-(3-chloro-4-(((cyclopropylamino)carbonyl)amino)phenoxy)-7-methoxy-6-q uinolinecarboxamide;
N6-ethyl-4-(3-chloro-4-(((cyclopropylamino)carbonyl)amino)phenoxy)-7-methoxy-6-qui nolinecarboxamide;
4-(3-fluoro-4-(cyclopropylaminocarbonyl)aminophenoxy)-7-(2-methoxyethoxy)-6-quinol inecarboxamide;
4-(3-chloro-4-(cyclopropylaminocarbonyl)aminophenoxy)-7-(2-hydroxyethoxy)-6-quinol inecarboxamide;
4-(3-chloro-4-(cyclopropylaminocarbonyl)aminophenoxy)-7-((2S)-2,3-dihydroxypropyl) oxy-6-quinolinecarboxamide;
4-(3-chloro-4-(methylaminocarbonyl)aminophenoxy)-7-methoxy-6-quinolinecarboxamid e;
4-(3-chloro-4-(ethylaminocarbonyl)aminophenoxy)-7-methoxy-6-quinolinecarboxamide;
N6-methoxy-4-(3-chloro-4-(((ethylamino)carbonyl)amino)phenoxy)-7-methoxy-6-quinol inecarboxamide;
4-(3-chloro-4-(cyclopropylaminocarbonyl)aminophenoxy)-7-(2-ethoxyethoxy)-6-quinoli necarboxamide;
4-(4-((cyclopropylamino)carbonyl)aminophenoxy)-7-(2-methoxyethoxy)-6-quinolinecarb oxamide;
N-(2-fluoro-4-((6-carbamoyl-7-methoxy-4-quinolyl)oxy)phenyl)-N'-cyclopropylurea;
N6-(2-hydroxyethyl)-4-(3-chloro-4-(((cyclopropylamino)carbonyl)amino)phenoxy)-7-me thoxy-6-quinolinecarboxamide;
4-(3-chloro-4-(1-propylaminocarbonyl)aminophenoxy)-7-methoxy-6-quinolinecarboxami de;
4-(3-chloro-4-(cis-2-fluoro-cyclopropylaminocarbonyl)aminophenoxy)-7-methoxy-6-qui nolinecarboxamide;
N6-methyl-4-(3-chloro-4-(((cyclopropylamino)carbonyl)amino)phenoxy)-7-(2-methoxyet hoxy)-6-quinolinecarboxamide;
N6-methyl-4-(3-chloro-4-(((ethylamino)carbonyl)amino)phenoxy)-7-methoxy-6-quinolin ecarboxamide;
4-(3-chloro-4-(cyclopropylaminocarbonyl)aminophenoxy)-7-(2-(4-morpholino)ethoxy)-6 -quinolinecarboxamide;
4-(3-chloro-4-(2-fluoroethylaminocarbonyl)aminophenoxy)-7-methoxy-6-quinolinecarbo xamide;
N6-((2R)tetrahydro-2-furanylmethyl)-4-(3-chloro-4-(((methylamino)carbonyl)amino)phe noxy)-7-methoxy-6-quinolinecarboxamide;
4-(3-fluoro-4-(ethylaminocarbonyl)aminophenoxy)-7-methoxy-6-quinolinecarboxamide;
4-(3-chloro-4-(((cyclopropylamino)carbonyl)amino)phenoxy)-7-((2R)-2-hydroxy-3-(1-py rrolidino)propoxy)-6-quinolinecarboxamide;
N6-methyl-4-(3-chloro-4-(((methylamino)carbonyl)amino)phenoxy)-7-((2R)-3-diethylam ino-2-hydroxypropoxy)-6-quinolinecarboxamide;
N6-methyl-4-(3-chloro-4-(((ethylamino)carbonyl)amino)phenoxy)-7-((2R)-3-diethylamin o-2-hydroxypropoxy)-6-quinolinecarboxamide;
N6-methyl-4-(3-chloro-4-(((methylamino)carbonyl)amino)phenoxy)-7-((2R)-2-hydroxy-3-(1-pyrrolidino)propoxy)-6-quinolinecarboxamide;
N6-methyl-4-(3-chloro-4-(((ethylamino)carbonyl)amino)phenoxy)-7-((2R)-2-hydroxy-3-( 1-pyrrolidino)propoxy)-6-quinolinecarboxamide;
N6-methyl-4-(3-chloro-4-(((methylamino)carbonyl)amino)phenoxy)-7-((1-methyl-4-pipe ridyl)methoxy)-6-quinolinecarboxamide;
N6-methyl-4-(3-chloro-4-(((ethylamino)carbonyl)amino)phenoxy)-7-((1-methyl-4-piperi dyl)methoxy)-6-quinolinecarboxamide;
N-(4-(6-cyano-7-(2-methoxyethoxy)-4-quinolyl)oxy-2-fluorophenyl)-N'-cyclopropylurea
N-(4-(6-cyano-7-(3-(4-morpholino)propoxy)-4-quinolyl)oxyphenyl)-N'-(3-(methylsulfon yl)phenyl)urea;
4-(4-((cyclopropylamino)carbonyl)aminophenoxy)-7-methoxy-6-quinolinecarboxamide;
4-(3-fluoro-4-((2-fluoroethylamino)carbonyl)aminophenoxy)-7-methoxy-6-quinolinecarb oxamide;
N6-(2-ethoxyethyl)-4-(3-chloro-4-(((methylamino)carbonyl)amino)phenoxy)-7-methoxy-6-quinolinecarboxamide;
4-(4-(3-ethylureido)-3-fluoro-phenoxy)-7-methoxyquinoline-6-carboxylic acid (2-cyanoethyl)amide; and
N-(4-(6-(2-cyanoethyl)carbamoyl-7-methoxy-4-quinolyl)oxy-2-fluorophenyl)-N'-cyclopr opylurea,
a pharmacologically acceptable salt thereof or a solvate thereof.

81. The method according to Claim 68, wherein the compound represented by General Formula (I), a pharmacologically acceptable salt thereof or a solvate thereof is at least one compound selected from the group consisting of:
4-(3-chloro-4-(cyclopropylaminocarbonyl)aminophenoxy)-7-methoxy-6-quinolinecarbox amide;
4-(3-chloro-4-(ethylaminocarbonyl)aminophenoxy)-7-methoxy-6-quinolinecarboxamide;
N6-methoxy-4-(3-chloro-4-(((cyclopropylamino)carbonyl)amino)phenoxy)-7-methoxy-6-quinolinecarboxamide;
4-(3-chloro-4-(methylaminocarbonyl)aminophenoxy)-7-methoxy-6-quinolinecarboxamid e; and
N6-methoxy-4-(3-chloro-4-(((ethylamino)carbonyl)amino)phenoxy)-7-methoxy-6-quinol inecarboxamide,
a pharmacologically acceptable salt thereof or a solvate thereof.

82. The method according to Claim 68, wherein the compound represented by General Formula (I), a pharmacologically acceptable salt thereof or a solvate thereof is 4-(3-chloro-4-(cyclopropylaminocarbonyl)aminophenoxy)-7-methoxy-6-quinolinecarbox amide), a pharmacologically acceptable salt thereof or a solvate thereof.

83. The method according to Claim 68, wherein the compound represented by General Formula (I), a pharmacologically acceptable salt thereof or a solvate thereof is methanesulfonate of 4-(3-chloro-4-(cyclopropylaminocarbonyl)aminophenoxy)-7-methoxy-6-quinolinecarbox amide.

84. The method according to any one of Claims 68-83, wherein the mutation of FGFR3 is substitution of at least one amino acid selected from the group consisting of codons 248, 249, 370, 371, 373, 380, 384, 391 and 650 in the amino acid sequence represented by SEQ ID NO: 2 with other amino acid.

85. The method according to any one of Claims 68-83, wherein the mutation of FGFR3 is at least one mutation selected from the group consisting of R248C, S249C, G370C, S371C, Y373C, G380R, F384L, A391E, K650E, K650M, K650Q and K650T in the amino acid sequence represented by SEQ ID NO: 2.

86. The method according to any one of Claims 68-83, wherein the cell is a multiple myeloma cell.

87. The method according to any one of Claims 68-83, wherein the patient is a patient suffering from at least one disease selected from the group consisting of multiple myeloma, bladder cancer, cervical cancer, hypochondroplasia, achondroplasia, thanatophoric dysplasia and skeletal dysplasia.

88. The method according to any one of Claims 68-83, wherein the method for predicting comprises the steps of: determining at least one selected from the group consisting of the FGFR3 expression level, the presence or the absence of a t(4; 14) translocation and the presence or the absence of FGFR3 mutation in the cell; and predicting whether or not the patient is highly sensitive to the compound represented by General Formula (I), a pharmacologically acceptable salt thereof or a solvate thereof using the result from the determination as an index.

89. The method according to Claim 88, wherein the determination of the FGFR3 expression level, the presence or the absence of a t(4;14) translocation or the presence or the absence of FGFR3 mutation in the cell is carried out by an immunochemical technique.

90. The method according to Claim 88, wherein the determination of the presence or the absence of a t(4; 14) translocation in the cell is carried out by FISH method.

91. A FGFR3 inhibitor comprising a compound represented by General Formula (I) below, a pharmacologically acceptable salt thereof or a solvate thereof, 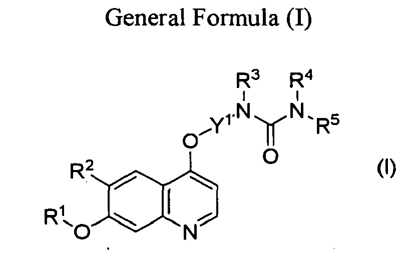 [wherein, R¹ represents group represented by Formula -V¹-V²-V³ (wherein, V¹ represents C₁₋₆ alkylene group that may have a substituent; V² represents a single bond, an oxygen atom, a sulfur atom, carbonyl group, sulfinyl group, sulfonyl group, group represented by Formula -CONR⁶-, group represented by Formula -SO₂NR⁶-, group represented by Formula -NR⁶SO₂-, group represented by Formula -NR⁶CO- or group represented by Formula -NR⁶- (wherein, R⁶ represents a hydrogen atom, C₁₋₆ alkyl group that may have a substituent or C₃₋₈ cycloalkyl group that may have a substituent); V³ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent);
R² represents cyano group, C₁₋₆ alkoxy group that may have a substituent, carboxyl group, C₂₋₇ alkoxycarbonyl group that may have a substituent or group represented by Formula -CONV^{a11}V^{a12} (wherein, V^{a11} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent; V^{a12} represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent, 3-10-membered nonaromatic heterocyclic group that may have a substituent, hydroxyl group, C₁₋₆ alkoxy group that may have a substituent or C₃₋₈ cycloalkoxy group that may have a substituent);
Y¹ represents group represented by Formula 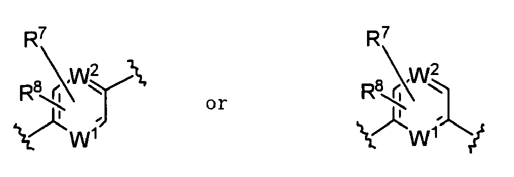 (wherein, R⁷ and R⁸ each independently represent a hydrogen atom, a halogen atom, cyano group, nitro group, amino group, C₁₋₆ alkyl group that may have a substituent, C₃₋₈ cycloalkyl group that may have a substituent, C₁₋₆ alkoxy group that may have a substituent, C₁₋₆ alkylthio group that may have a substituent, formyl group, C₂₋₇ acyl group that may have a substituent, C₂₋₇ alkoxycarbonyl group that may have a substituent or group represented by Formula -CONV^{d1}V^{d2} (wherein, V^{d1} and V^{d2} each independently represent a hydrogen atom or C₁₋₆ alkyl group that may have a substituent);
W¹ and W² each independently represent a carbon atom or a nitrogen atom that may have a substituent);
R³ and R⁴ each independently represent a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₂₋₇ acyl group that may have a substituent or C₂₋₇ alkoxycarbonyl group that may have a substituent;
R⁵ represents a hydrogen atom, C₁₋₆ alkyl group that may have a substituent, C₂₋₆ alkenyl group that may have a substituent, C₂₋₆ alkynyl group that may have a substituent, C₃₋₈ cycloalkyl group that may have a substituent, C₆₋₁₀ aryl group that may have a substituent, 5-10-membered heteroaryl group that may have a substituent or 3-10-membered nonaromatic heterocyclic group that may have a substituent].

92. The pharmaceutical composition according to Claim 1, wherein the compound represented by General Formula (I), a pharmacologically acceptable salt thereof or a solvate thereof has a FGFR3-inhibiting activity.

93. The therapeutic drug according to Claim 21, wherein the compound represented by General Formula (I), a pharmacologically acceptable salt thereof or a solvate thereof has a FGFR3-inhibiting activity.

94. The therapeutic drug according to Claim 40, wherein the compound represented by General Formula (I), a pharmacologically acceptable salt thereof or a solvate thereof has a FGFR3-inhibiting activity.

95. The method according to Claim 68, wherein the compound represented by General Formula (I), a pharmacologically acceptable salt thereof or a solvate thereof has a FGFR3-inhibiting activity.


SEQUENCE LISTING

<110>  Eisai R&D Management Co., Ltd.

<120>  Antitumor agents for multiple myeloma

<130>  PCT06-0157

<150>  JP2005-337772
<151>  2005-11-22

<150>  US60/803,450
<151>  2006-05-30

<160>  3

<170>  PatentIn version 3.3

<210>  1
<211>  4093
<212>  DNA
<213>  Homo sapiens


<220>
<221>  CDS
<222>  (40)..(2460)

<400>  1
cgcgcgctgc ctgaggacgc cgcggccccc gcccccgcc atg ggc gcc cct gcc      54
                                           Met Gly Ala Pro Ala
                                           1               5

tgc gcc ctc gcg ctc tgc gtg gcc gtg gcc atc gtg gcc ggc gcc tcc      102
Cys Ala Leu Ala Leu Cys Val Ala Val Ala Ile Val Ala Gly Ala Ser
                10                  15                  20

tcg gag tcc ttg ggg acg gag cag cgc gtc gtg ggg cga gcg gca gaa      150
Ser Glu Ser Leu Gly Thr Glu Gln Arg Val Val Gly Arg Ala Ala Glu
            25                  30                  35

gtc ccg ggc cca gag ccc ggc cag cag gag cag ttg gtc ttc ggc agc      198
Val Pro Gly Pro Glu Pro Gly Gln Gln Glu Gln Leu Val Phe Gly Ser
        40                  45                  50

ggg gat gct gtg gag ctg agc tgt ccc ccg ccc ggg ggt ggt ccc atg      246
Gly Asp Ala Val Glu Leu Ser Cys Pro Pro Pro Gly Gly Gly Pro Met
    55                  60                  65

ggg ccc act gtc tgg gtc aag gat ggc aca ggg ctg gtg ccc tcg gag      294
Gly Pro Thr Val Trp Val Lys Asp Gly Thr Gly Leu Val Pro Ser Glu
70                  75                  80                  85

cgt gtc ctg gtg ggg ccc cag cgg ctg cag gtg ctg aat gcc tcc cac      342
Arg Val Leu Val Gly Pro Gln Arg Leu Gln Val Leu Asn Ala Ser His
                90                  95                  100

gag gac tcc ggg gcc tac agc tgc cgg cag cgg ctc acg cag cgc gta      390

Glu Asp Ser Gly Ala Tyr Ser Cys Arg Gln Arg Leu Thr Gln Arg Val
            105                 110                 115

ctg tgc cac ttc agt gtg cgg gtg aca gac gct cca tcc tcg gga gat      438
Leu Cys His Phe Ser Val Arg Val Thr Asp Ala Pro Ser Ser Gly Asp
        120                 125                 130

gac gaa gac ggg gag gac gag gct gag gac aca ggt gtg gac aca ggg      486
Asp Glu Asp Gly Glu Asp Glu Ala Glu Asp Thr Gly Val Asp Thr Gly
    135                 140                 145

gcc cct tac tgg aca cgg ccc gag cgg atg gac aag aag ctg ctg gcc      534
Ala Pro Tyr Trp Thr Arg Pro Glu Arg Met Asp Lys Lys Leu Leu Ala
150                 155                 160                 165

gtg ccg gcc gcc aac acc gtc cgc ttc cgc tgc cca gcc gct ggc aac      582
Val Pro Ala Ala Asn Thr Val Arg Phe Arg Cys Pro Ala Ala Gly Asn
                170                 175                 180

ccc act ccc tcc atc tcc tgg ctg aag aac ggc agg gag ttc cgc ggc      630
Pro Thr Pro Ser Ile Ser Trp Leu Lys Asn Gly Arg Glu Phe Arg Gly
            185                 190                 195

gag cac cgc att gga ggc atc aag ctg cgg cat cag cag tgg agc ctg      678
Glu His Arg Ile Gly Gly Ile Lys Leu Arg His Gln Gln Trp Ser Leu
        200                 205                 210

gtc atg gaa agc gtg gtg ccc tcg gac cgc ggc aac tac acc tgc gtc      726
Val Met Glu Ser Val Val Pro Ser Asp Arg Gly Asn Tyr Thr Cys Val
    215                 220                 225

gtg gag aac aag ttt ggc agc atc cgg cag acg tac acg ctg gac gtg      774
Val Glu Asn Lys Phe Gly Ser Ile Arg Gln Thr Tyr Thr Leu Asp Val
230                 235                 240                 245

ctg gag cgc tcc ccg cac cgg ccc atc ctg cag gcg ggg ctg ccg gcc      822
Leu Glu Arg Ser Pro His Arg Pro Ile Leu Gln Ala Gly Leu Pro Ala
                250                 255                 260

aac cag acg gcg gtg ctg ggc agc gac gtg gag ttc cac tgc aag gtg      870
Asn Gln Thr Ala Val Leu Gly Ser Asp Val Glu Phe His Cys Lys Val
            265                 270                 275

tac agt gac gca cag ccc cac atc cag tgg ctc aag cac gtg gag gtg      918
Tyr Ser Asp Ala Gln Pro His Ile Gln Trp Leu Lys His Val Glu Val
        280                 285                 290

aac ggc agc aag gtg ggc ccg gac ggc aca ccc tac gtt acc gtg ctc      966
Asn Gly Ser Lys Val Gly Pro Asp Gly Thr Pro Tyr Val Thr Val Leu
    295                 300                 305

aag acg gcg ggc gct aac acc acc gac aag gag cta gag gtt ctc tcc      1014
Lys Thr Ala Gly Ala Asn Thr Thr Asp Lys Glu Leu Glu Val Leu Ser
310                 315                 320                 325

ttg cac aac gtc acc ttt gag gac gcc ggg gag tac acc tgc ctg gcg      1062
Leu His Asn Val Thr Phe Glu Asp Ala Gly Glu Tyr Thr Cys Leu Ala
                330                 335                 340

ggc aat tct att ggg ttt tct cat cac tct gcg tgg ctg gtg gtg ctg 1110
Gly Asn Ser Ile Gly Phe Ser His His Ser Ala Trp Leu Val Val Leu
            345                 350                 355

cca gcc gag gag gag ctg gtg gag gct gac gag gcg ggc agt gtg tat 1158
Pro Ala Glu Glu Glu Leu Val Glu Ala Asp Glu Ala Gly Ser Val Tyr
        360                 365                 370

gca ggc atc ctc agc tac ggg gtg ggc ttc ttc ctg ttc atc ctg gtg 1206
Ala Gly Ile Leu Ser Tyr Gly Val Gly Phe Phe Leu Phe Ile Leu Val
    375                 380                 385

gtg gcg gct gtg acg ctc tgc cgc ctg cgc agc ccc ccc aag aaa ggc 1254
Val Ala Ala Val Thr Leu Cys Arg Leu Arg Ser Pro Pro Lys Lys Gly
390                 395                 400                 405

ctg ggc tcc ccc acc gtg cac aag atc tcc cgc ttc ccg ctc aag cga 1302
Leu Gly Ser Pro Thr Val His Lys Ile Ser Arg Phe Pro Leu Lys Arg
                410                 415                 420

cag gtg tcc ctg gag tcc aac gcg tcc atg agc tcc aac aca cca ctg 1350
Gln Val Ser Leu Glu Ser Asn Ala Ser Met Ser Ser Asn Thr Pro Leu
            425                 430                 435

gtg cgc atc gca agg ctg tcc tca ggg gag ggc ccc acg ctg gcc aat 1398
Val Arg Ile Ala Arg Leu Ser Ser Gly Glu Gly Pro Thr Leu Ala Asn
        440                 445                 450

gtc tcc gag ctc gag ctg cct gcc gac ccc aaa tgg gag ctg tct cgg 1446
Val Ser Glu Leu Glu Leu Pro Ala Asp Pro Lys Trp Glu Leu Ser Arg
    455                 460                 465

gcc cgg ctg acc ctg ggc aag ccc ctt ggg gag ggc tgc ttc ggc cag 1494
Ala Arg Leu Thr Leu Gly Lys Pro Leu Gly Glu Gly Cys Phe Gly Gln
470                 475                 480                 485

gtg gtc atg gcg gag gcc atc ggc att gac aag gac cgg gcc gcc aag 1542
Val Val Met Ala Glu Ala Ile Gly Ile Asp Lys Asp Arg Ala Ala Lys
                490                 495                 500

cct gtc acc gta gcc gtg aag atg ctg aaa gac gat gcc act gac aag 1590
Pro Val Thr Val Ala Val Lys Met Leu Lys Asp Asp Ala Thr Asp Lys
            505                 510                 515

gac ctg tcg gac ctg gtg tct gag atg gag atg atg aag atg atc ggg 1638
Asp Leu Ser Asp Leu Val Ser Glu Met Glu Met Met Lys Met Ile Gly
        520                 525                 530

aaa cac aaa aac atc atc aac ctg ctg ggc gcc tgc acg cag ggc ggg 1686
Lys His Lys Asn Ile Ile Asn Leu Leu Gly Ala Cys Thr Gln Gly Gly
    535                 540                 545

ccc ctg tac gtg ctg gtg gag tac gcg gcc aag ggt aac ctg cgg gag 1734
Pro Leu Tyr Val Leu Val Glu Tyr Ala Ala Lys Gly Asn Leu Arg Glu
550                 555                 560                 565

ttt ctg cgg gcg cgg cgg ccc ccg ggc ctg gac tac tcc ttc gac acc 1782

Phe Leu Arg Ala Arg Arg Pro Pro Gly Leu Asp Tyr Ser Phe Asp Thr
                570                 575                 580

tgc aag ccg ccc gag gag cag ctc acc ttc aag gac ctg gtg tcc tgt 1830
Cys Lys Pro Pro Glu Glu Gln Leu Thr Phe Lys Asp Leu Val Ser Cys
            585                 590                 595

gcc tac cag gtg gcc cgg ggc atg gag tac ttg gcc tcc cag aag tgc 1878
Ala Tyr Gln Val Ala Arg Gly Met Glu Tyr Leu Ala Ser Gln Lys Cys
        600                 605                 610

atc cac agg gac ctg gct gcc cgc aat gtg ctg gtg acc gag gac aac 1926
Ile His Arg Asp Leu Ala Ala Arg Asn Val Leu Val Thr Glu Asp Asn
    615                 620                 625

gtg atg aag atc gca gac ttc ggg ctg gcc cgg gac gtg cac aac ctc 1974
Val Met Lys Ile Ala Asp Phe Gly Leu Ala Arg Asp Val His Asn Leu
630                 635                 640                 645

gac tac tac aag aag aca acc aac ggc cgg ctg ccc gtg aag tgg atg 2022
Asp Tyr Tyr Lys Lys Thr Thr Asn Gly Arg Leu Pro Val Lys Trp Met
                650                 655                 660

gcg cct gag gcc ttg ttt gac cga gtc tac act cac cag agt gac gtc 2070
Ala Pro Glu Ala Leu Phe Asp Arg Val Tyr Thr His Gln Ser Asp Val
            665                 670                 675

tgg tcc ttt ggg gtc ctg ctc tgg gag atc ttc acg ctg ggg ggc tcc 2118
Trp Ser Phe Gly Val Leu Leu Trp Glu Ile Phe Thr Leu Gly Gly Ser
        680                 685                 690

ccg tac ccc ggc atc cct gtg gag gag ctc ttc aag ctg ctg aag gag 2166
Pro Tyr Pro Gly Ile Pro Val Glu Glu Leu Phe Lys Leu Leu Lys Glu
    695                 700                 705

ggc cac cgc atg gac aag ccc gcc aac tgc aca cac gac ctg tac atg 2214
Gly His Arg Met Asp Lys Pro Ala Asn Cys Thr His Asp Leu Tyr Met
710                 715                 720                 725

atc atg cgg gag tgc tgg cat gcc gcg ccc tcc cag agg ccc acc ttc 2262
Ile Met Arg Glu Cys Trp His Ala Ala Pro Ser Gln Arg Pro Thr Phe
                730                 735                 740

aag cag ctg gtg gag gac ctg gac cgt gtc ctt acc gtg acg tcc acc 2310
Lys Gln Leu Val Glu Asp Leu Asp Arg Val Leu Thr Val Thr Ser Thr
            745                 750                 755

gac gag tac ctg gac ctg tcg gcg cct ttc gag cag tac tcc ccg ggt 2358
Asp Glu Tyr Leu Asp Leu Ser Ala Pro Phe Glu Gln Tyr Ser Pro Gly
        760                 765                 770

ggc cag gac acc ccc agc tcc agc tcc tca ggg gac gac tcc gtg ttt 2406
Gly Gln Asp Thr Pro Ser Ser Ser Ser Ser Gly Asp Asp Ser Val Phe
    775                 780                 785

gcc cac gac ctg ctg ccc ccg gcc cca ccc agc agt ggg ggc tcg cgg 2454
Ala His Asp Leu Leu Pro Pro Ala Pro Pro Ser Ser Gly Gly Ser Arg
790                 795                 800                 805

acg tga agggccactg gtccccaaca atgtgagggg tccctagcag ccctccctgc 2510
Thr

tgctggtgca cagccactcc ccggcatgag actcagtgca gatggagaga cagctacaca 2570

gagctttggt ctgtgtgtgt gtgtgtgcgt gtgtgtgtgt gtgtgcacat ccgcgtgtgc 2630

ctgtgtgcgt gcgcatcttg cctccaggtg cagaggtacc ctgggtgtcc ccgctgctgt 2690

gcaacggtct cctgactggt gctgcagcac cgaggggcct ttgttctggg gggacccagt 2750

gcagaatgta agtgggccca cccggtggga ccccgtgggg cagggagctg ggcccgacat 2810

ggctcggcct ctgcctttgc accacgggac atcacagggt gcgctcggcc cctcccacac 2870

ccaaagctga gcctgcaggg aagccccaca tgtccagcac cttgtgcctg gggtgttagt 2930

ggcaccgcct ccccacctcc aggctttccc acttcccacc ctgcccctca gagactgaaa 2990

ttacgggtac ctgaagatgg gagcctttac cttttatgca aaaggtttat tccggaaact 3050

agtgtacatt tctataaata gatgctgtgt atatggtata tatacatata tatatataac 3110

atatatggaa gaggaaaagg ctggtacaac ggaggcctgc gaccctgggg gcacaggagg 3170

caggcatggc cctgggcggg gcgtgggggg gcgtggaggg aggccccagg ggtctcaccc 3230

atgcaagcag aggaccaggg ctttttctgg caccgcagtt ttgttttaaa actggacctg 3290

tatatttgta aagctattta tgggcccctg gcactcttgt tcccacaccc caacacttcc 3350

agcatttagc tggccacatg gcggagagtt ttaattttta acttattgac aaccgagaag 3410

gtttatcccg ccgatagagg gacggccaag aatgtacgtc cagcctgccc cggagctgga 3470

ggatcccctc caagcctaaa aggttgttaa tagttggagg tgattccagt gaagatattt 3530

tatttgcttt gtcctttttc aggagaatta gatttctata ggatttttct ttaggagatt 3590

tattttttgg acttcaaagc aagctggtat tttcatacaa attcttctaa ttgctgtgtg 3650

tcccaggcag ggagacggtt tccagggagg ggccggccct gtgtgcaggt tccgatgtta 3710

ttagatgtta caagtttata tatatctata tatataattt attgagtttt tacaagatgt 3770

atttgttgta gacttaacac ttcttacgca atgcttctag agttttatag cctggactgc 3830

tacctttcaa agcttggagg gaagccgtga attcagttgg ttcgttctgt actgttactg 3890

ggccctgagt ctgggcagct gtcccttgct tgcctgcagg gccatggctc agggtggtct 3950

cttcttgggg cccagtgcat ggtggccaga ggtgtcaccc aaaccggcag gtgcgatttt 4010

gttaacccag cgacgaactt tccgaaaaat aaagacacct ggttgctaac ctgaaaaaaa 4070

aaaaaaaaaa aaaaaaaaaa aaa 4093

<210> 2
<211> 806
<212> PRT
<213> Homo sapiens

<400> 2

Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His

195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr

660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly
    770                 775                 780

Asp Asp Ser Val Phe Ala His Asp Leu Leu Pro Pro Ala Pro Pro Ser
785                 790                 795                 800

Ser Gly Gly Ser Arg Thr
                805

<210>  3
<211>  784
<212>  PRT
<213>  Homo sapiens

<400>  3

Glu Ser Leu Gly Thr Glu Gln Arg Val Val Gly Arg Ala Ala Glu Val
1               5                   10                  15

Pro Gly Pro Glu Pro Gly Gln Gln Glu Gln Leu Val Phe Gly Ser Gly
            20                  25                  30

Asp Ala Val Glu Leu Ser Cys Pro Pro Pro Gly Gly Gly Pro Met Gly
        35                  40                  45

Pro Thr Val Trp Val Lys Asp Gly Thr Gly Leu Val Pro Ser Glu Arg
    50                  55                  60

Val Leu Val Gly Pro Gln Arg Leu Gln Val Leu Asn Ala Ser His Glu
65                  70                  75                  80

Asp Ser Gly Ala Tyr Ser Cys Arg Gln Arg Leu Thr Gln Arg Val Leu
                85                  90                  95

Cys His Phe Ser Val Arg Val Thr Asp Ala Pro Ser Ser Gly Asp Asp
            100                 105                 110

Glu Asp Gly Glu Asp Glu Ala Glu Asp Thr Gly Val Asp Thr Gly Ala
        115                 120                 125

Pro Tyr Trp Thr Arg Pro Glu Arg Met Asp Lys Lys Leu Leu Ala Val
    130                 135                 140

Pro Ala Ala Asn Thr Val Arg Phe Arg Cys Pro Ala Ala Gly Asn Pro
145                 150                 155                 160

Thr Pro Ser Ile Ser Trp Leu Lys Asn Gly Arg Glu Phe Arg Gly Glu
                165                 170                 175

His Arg Ile Gly Gly Ile Lys Leu Arg His Gln Gln Trp Ser Leu Val
            180                 185                 190

Met Glu Ser Val Val Pro Ser Asp Arg Gly Asn Tyr Thr Cys Val Val
        195                 200                 205

Glu Asn Lys Phe Gly Ser Ile Arg Gln Thr Tyr Thr Leu Asp Val Leu
    210                 215                 220

Glu Arg Ser Pro His Arg Pro Ile Leu Gln Ala Gly Leu Pro Ala Asn
225                 230                 235                 240

Gln Thr Ala Val Leu Gly Ser Asp Val Glu Phe His Cys Lys Val Tyr
                245                 250                 255

Ser Asp Ala Gln Pro His Ile Gln Trp Leu Lys His Val Glu Val Asn
            260                 265                 270

Gly Ser Lys Val Gly Pro Asp Gly Thr Pro Tyr Val Thr Val Leu Lys
        275                 280                 285

Thr Ala Gly Ala Asn Thr Thr Asp Lys Glu Leu Glu Val Leu Ser Leu
    290                 295                 300

His Asn Val Thr Phe Glu Asp Ala Gly Glu Tyr Thr Cys Leu Ala Gly
305                 310                 315                 320

Asn Ser Ile Gly Phe Ser His His Ser Ala Trp Leu Val Val Leu Pro
                325                 330                 335

Ala Glu Glu Glu Leu Val Glu Ala Asp Glu Ala Gly Ser Val Tyr Ala
            340                 345                 350

Gly Ile Leu Ser Tyr Gly Val Gly Phe Phe Leu Phe Ile Leu Val Val
        355                 360                 365

Ala Ala Val Thr Leu Cys Arg Leu Arg Ser Pro Pro Lys Lys Gly Leu
    370                 375                 380

Gly Ser Pro Thr Val His Lys Ile Ser Arg Phe Pro Leu Lys Arg Gln
385                 390                 395                 400

Val Ser Leu Glu Ser Asn Ala Ser Met Ser Ser Asn Thr Pro Leu Val
                405                 410                 415

Arg Ile Ala Arg Leu Ser Ser Gly Glu Gly Pro Thr Leu Ala Asn Val
            420                 425                 430

Ser Glu Leu Glu Leu Pro Ala Asp Pro Lys Trp Glu Leu Ser Arg Ala
        435                 440                 445

Arg Leu Thr Leu Gly Lys Pro Leu Gly Glu Gly Cys Phe Gly Gln Val
    450                 455                 460

Val Met Ala Glu Ala Ile Gly Ile Asp Lys Asp Arg Ala Ala Lys Pro
465                 470                 475                 480

Val Thr Val Ala Val Lys Met Leu Lys Asp Asp Ala Thr Asp Lys Asp
                485                 490                 495

Leu Ser Asp Leu Val Ser Glu Met Glu Met Met Lys Met Ile Gly Lys
            500                 505                 510

His Lys Asn Ile Ile Asn Leu Leu Gly Ala Cys Thr Gln Gly Gly Pro
        515                 520                 525

Leu Tyr Val Leu Val Glu Tyr Ala Ala Lys Gly Asn Leu Arg Glu Phe
    530                 535                 540

Leu Arg Ala Arg Arg Pro Pro Gly Leu Asp Tyr Ser Phe Asp Thr Cys
545                 550                 555                 560

Lys Pro Pro Glu Glu Gln Leu Thr Phe Lys Asp Leu Val Ser Cys Ala
                565                 570                 575

Tyr Gln Val Ala Arg Gly Met Glu Tyr Leu Ala Ser Gln Lys Cys Ile
            580                 585                 590

His Arg Asp Leu Ala Ala Arg Asn Val Leu Val Thr Glu Asp Asn Val
        595                 600                 605

Met Lys Ile Ala Asp Phe Gly Leu Ala Arg Asp Val His Asn Leu Asp
    610                 615                 620

Tyr Tyr Lys Lys Thr Thr Asn Gly Arg Leu Pro Val Lys Trp Met Ala
625                 630                 635                 640

Pro Glu Ala Leu Phe Asp Arg Val Tyr Thr His Gln Ser Asp Val Trp
                645                 650                 655

Ser Phe Gly Val Leu Leu Trp Glu Ile Phe Thr Leu Gly Gly Ser Pro
            660                 665                 670

Tyr Pro Gly Ile Pro Val Glu Glu Leu Phe Lys Leu Leu Lys Glu Gly
        675                 680                 685

His Arg Met Asp Lys Pro Ala Asn Cys Thr His Asp Leu Tyr Met Ile
    690                 695                 700

Met Arg Glu Cys Trp His Ala Ala Pro Ser Gln Arg Pro Thr Phe Lys
705                 710                 715                 720

Gln Leu Val Glu Asp Leu Asp Arg Val Leu Thr Val Thr Ser Thr Asp
                725                 730                 735

Glu Tyr Leu Asp Leu Ser Ala Pro Phe Glu Gln Tyr Ser Pro Gly Gly
            740                 745                 750

Gln Asp Thr Pro Ser Ser Ser Ser Ser Gly Asp Asp Ser Val Phe Ala
        755                 760                 765

His Asp Leu Leu Pro Pro Ala Pro Pro Ser Ser Gly Gly Ser Arg Thr
    770                 775                 780

SEQUENCE LISTING

<110>  Eisai R&D Management Co., Ltd.

<120>  Antitumor agents for multiple myeloma

<130>  N.104615

<140>  06833681.7
<141>  2006-11-22

<150>  PCT/JP06/323878
<151>  2006-11-22

<150>  JP2005-337772
<151>  2005-11-22

<150>  US60/803,450
<151>  2006-05-30

<160>  3

<170>  PatentIn version 3.3

<210>  1
<211>  4093
<212>  DNA
<213>  Homo sapiens


<220>
<221>  CDS
<222>  (40)..(2460)

<400>  1
cgcgcgctgc ctgaggacgc cgcggccccc gcccccgcc atg ggc gcc cct gcc        54
                                           Met Gly Ala Pro Ala
                                           1               5

tgc gcc ctc gcg ctc tgc gtg gcc gtg gcc atc gtg gcc ggc gcc tcc      102
Cys Ala Leu Ala Leu Cys Val Ala Val Ala Ile Val Ala Gly Ala Ser
                10                  15                  20

tcg gag tcc ttg ggg acg gag cag cgc gtc gtg ggg cga gcg gca gaa      150
Ser Glu Ser Leu Gly Thr Glu Gln Arg Val Val Gly Arg Ala Ala Glu
            25                  30                  35

gtc ccg ggc cca gag ccc ggc cag cag gag cag ttg gtc ttc ggc agc      198
Val Pro Gly Pro Glu Pro Gly Gln Gln Glu Gln Leu Val Phe Gly Ser
        40                  45                  50

ggg gat gct gtg gag ctg agc tgt ccc ccg ccc ggg ggt ggt ccc atg      246
Gly Asp Ala Val Glu Leu Ser Cys Pro Pro Pro Gly Gly Gly Pro Met
    55                  60                  65

ggg ccc act gtc tgg gtc aag gat ggc aca ggg ctg gtg ccc tcg gag      294
Gly Pro Thr Val Trp Val Lys Asp Gly Thr Gly Leu Val Pro Ser Glu
70                  75                  80                  85

cgt gtc ctg gtg ggg ccc cag cgg ctg cag gtg ctg aat gcc tcc cac      342

Arg Val Leu Val Gly Pro Gln Arg Leu Gln Val Leu Asn Ala Ser His
                90                  95                  100

gag gac tcc ggg gcc tac agc tgc cgg cag cgg ctc acg cag cgc gta      390
Glu Asp Ser Gly Ala Tyr Ser Cys Arg Gln Arg Leu Thr Gln Arg Val
        105                 110                 115

ctg tgc cac ttc agt gtg cgg gtg aca gac gct cca tcc tcg gga gat      438
Leu Cys His Phe Ser Val Arg Val Thr Asp Ala Pro Ser Ser Gly Asp
        120                 125                 130

gac gaa gac ggg gag gac gag gct gag gac aca ggt gtg gac aca ggg      486
Asp Glu Asp Gly Glu Asp Glu Ala Glu Asp Thr Gly Val Asp Thr Gly
    135                 140                 145

gcc cct tac tgg aca cgg ccc gag cgg atg gac aag aag ctg ctg gcc      534
Ala Pro Tyr Trp Thr Arg Pro Glu Arg Met Asp Lys Lys Leu Leu Ala
150                 155                 160                 165

gtg ccg gcc gcc aac acc gtc cgc ttc cgc tgc cca gcc gct ggc aac      582
Val Pro Ala Ala Asn Thr Val Arg Phe Arg Cys Pro Ala Ala Gly Asn
                170                 175                 180

ccc act ccc tcc atc tcc tgg ctg aag aac ggc agg gag ttc cgc ggc      630
Pro Thr Pro Ser Ile Ser Trp Leu Lys Asn Gly Arg Glu Phe Arg Gly
        185                 190                 195

gag cac cgc att gga ggc atc aag ctg cgg cat cag cag tgg agc ctg      678
Glu His Arg Ile Gly Gly Ile Lys Leu Arg His Gln Gln Trp Ser Leu
        200                 205                 210

gtc atg gaa agc gtg gtg ccc tcg gac cgc ggc aac tac acc tgc gtc      726
Val Met Glu Ser Val Val Pro Ser Asp Arg Gly Asn Tyr Thr Cys Val
    215                 220                 225

gtg gag aac aag ttt ggc agc atc cgg cag acg tac acg ctg gac gtg      774
Val Glu Asn Lys Phe Gly Ser Ile Arg Gln Thr Tyr Thr Leu Asp Val
230                 235                 240                 245

ctg gag cgc tcc ccg cac cgg ccc atc ctg cag gcg ggg ctg ccg gcc      822
Leu Glu Arg Ser Pro His Arg Pro Ile Leu Gln Ala Gly Leu Pro Ala
                250                 255                 260

aac cag acg gcg gtg ctg ggc agc gac gtg gag ttc cac tgc aag gtg      870
Asn Gln Thr Ala Val Leu Gly Ser Asp Val Glu Phe His Cys Lys Val
        265                 270                 275

tac agt gac gca cag ccc cac atc cag tgg ctc aag cac gtg gag gtg      918
Tyr Ser Asp Ala Gln Pro His Ile Gln Trp Leu Lys His Val Glu Val
        280                 285                 290

aac ggc agc aag gtg ggc ccg gac ggc aca ccc tac gtt acc gtg ctc      966
Asn Gly Ser Lys Val Gly Pro Asp Gly Thr Pro Tyr Val Thr Val Leu
    295                 300                 305

aag acg gcg ggc gct aac acc acc gac aag gag cta gag gtt ctc tcc      1014
Lys Thr Ala Gly Ala Asn Thr Thr Asp Lys Glu Leu Glu Val Leu Ser
310                 315                 320                 325

ttg cac aac gtc acc ttt gag gac gcc ggg gag tac acc tgc ctg gcg      1062

Leu His Asn Val Thr Phe Glu Asp Ala Gly Glu Tyr Thr Cys Leu Ala
                330                 335                 340

ggc aat tct att ggg ttt tct cat cac tct gcg tgg ctg gtg gtg ctg      1110
Gly Asn Ser Ile Gly Phe Ser His His Ser Ala Trp Leu Val Val Leu
            345                 350                 355

cca gcc gag gag gag ctg gtg gag gct gac gag gcg ggc agt gtg tat      1158
Pro Ala Glu Glu Glu Leu Val Glu Ala Asp Glu Ala Gly Ser Val Tyr
        360                 365                 370

gca ggc atc ctc agc tac ggg gtg ggc ttc ttc ctg ttc atc ctg gtg      1206
Ala Gly Ile Leu Ser Tyr Gly Val Gly Phe Phe Leu Phe Ile Leu Val
    375                 380                 385

gtg gcg gct gtg acg ctc tgc cgc ctg cgc agc ccc ccc aag aaa ggc      1254
Val Ala Ala Val Thr Leu Cys Arg Leu Arg Ser Pro Pro Lys Lys Gly
390                 395                 400                 405

ctg ggc tcc ccc acc gtg cac aag atc tcc cgc ttc ccg ctc aag cga      1302
Leu Gly Ser Pro Thr Val His Lys Ile Ser Arg Phe Pro Leu Lys Arg
                410                 415                 420

cag gtg tcc ctg gag tcc aac gcg tcc atg agc tcc aac aca cca ctg      1350
Gln Val Ser Leu Glu Ser Asn Ala Ser Met Ser Ser Asn Thr Pro Leu
            425                 430                 435

gtg cgc atc gca agg ctg tcc tca ggg gag ggc ccc acg ctg gcc aat      1398
Val Arg Ile Ala Arg Leu Ser Ser Gly Glu Gly Pro Thr Leu Ala Asn
        440                 445                 450

gtc tcc gag ctc gag ctg cct gcc gac ccc aaa tgg gag ctg tct cgg      1446
Val Ser Glu Leu Glu Leu Pro Ala Asp Pro Lys Trp Glu Leu Ser Arg
    455                 460                 465

gcc cgg ctg acc ctg ggc aag ccc ctt ggg gag ggc tgc ttc ggc cag      1494
Ala Arg Leu Thr Leu Gly Lys Pro Leu Gly Glu Gly Cys Phe Gly Gln
470                 475                 480                 485

gtg gtc atg gcg gag gcc atc ggc att gac aag gac cgg gcc gcc aag      1542
Val Val Met Ala Glu Ala Ile Gly Ile Asp Lys Asp Arg Ala Ala Lys
                490                 495                 500

cct gtc acc gta gcc gtg aag atg ctg aaa gac gat gcc act gac aag      1590
Pro Val Thr Val Ala Val Lys Met Leu Lys Asp Asp Ala Thr Asp Lys
            505                 510                 515

gac ctg tcg gac ctg gtg tct gag atg gag atg atg aag atg atc ggg      1638
Asp Leu Ser Asp Leu Val Ser Glu Met Glu Met Met Lys Met Ile Gly
        520                 525                 530

aaa cac aaa aac atc atc aac ctg ctg ggc gcc tgc acg cag ggc ggg      1686
Lys His Lys Asn Ile Ile Asn Leu Leu Gly Ala Cys Thr Gln Gly Gly
    535                 540                 545

ccc ctg tac gtg ctg gtg gag tac gcg gcc aag ggt aac ctg cgg gag      1734
Pro Leu Tyr Val Leu Val Glu Tyr Ala Ala Lys Gly Asn Leu Arg Glu
550                 555                 560                 565

ttt ctg cgg gcg cgg cgg ccc ccg ggc ctg gac tac tcc ttc gac acc      1782

Phe Leu Arg Ala Arg Arg Pro Pro Gly Leu Asp Tyr Ser Phe Asp Thr
                570                 575                 580

tgc aag ccg ccc gag gag cag ctc acc ttc aag gac ctg gtg tcc tgt 1830
Cys Lys Pro Pro Glu Glu Gln Leu Thr Phe Lys Asp Leu Val Ser Cys
            585                 590                 595

gcc tac cag gtg gcc cgg ggc atg gag tac ttg gcc tcc cag aag tgc 1878
Ala Tyr Gln Val Ala Arg Gly Met Glu Tyr Leu Ala Ser Gln Lys Cys
        600                 605                 610

atc cac agg gac ctg gct gcc cgc aat gtg ctg gtg acc gag gac aac 1926
Ile His Arg Asp Leu Ala Ala Arg Asn Val Leu Val Thr Glu Asp Asn
    615                 620                 625

gtg atg aag atc gca gac ttc ggg ctg gcc cgg gac gtg cac aac ctc 1974
Val Met Lys Ile Ala Asp Phe Gly Leu Ala Arg Asp Val His Asn Leu
630                 635                 640                 645

gac tac tac aag aag aca acc aac ggc cgg ctg ccc gtg aag tgg atg 2022
Asp Tyr Tyr Lys Lys Thr Thr Asn Gly Arg Leu Pro Val Lys Trp Met
                650                 655                 660

gcg cct gag gcc ttg ttt gac cga gtc tac act cac cag agt gac gtc 2070
Ala Pro Glu Ala Leu Phe Asp Arg Val Tyr Thr His Gln Ser Asp Val
            665                 670                 675

tgg tcc ttt ggg gtc ctg ctc tgg gag atc ttc acg ctg ggg ggc tcc 2118
Trp Ser Phe Gly Val Leu Leu Trp Glu Ile Phe Thr Leu Gly Gly Ser
        680                 685                 690

ccg tac ccc ggc atc cct gtg gag gag ctc ttc aag ctg ctg aag gag 2166
Pro Tyr Pro Gly Ile Pro Val Glu Glu Leu Phe Lys Leu Leu Lys Glu
    695                 700                 705

ggc cac cgc atg gac aag ccc gcc aac tgc aca cac gac ctg tac atg 2214
Gly His Arg Met Asp Lys Pro Ala Asn Cys Thr His Asp Leu Tyr Met
710                 715                 720                 725

atc atg cgg gag tgc tgg cat gcc gcg ccc tcc cag agg ccc acc ttc 2262
Ile Met Arg Glu Cys Trp His Ala Ala Pro Ser Gln Arg Pro Thr Phe
                730                 735                 740

aag cag ctg gtg gag gac ctg gac cgt gtc ctt acc gtg acg tcc acc 2310
Lys Gln Leu Val Glu Asp Leu Asp Arg Val Leu Thr Val Thr Ser Thr
            745                 750                 755

gac gag tac ctg gac ctg tcg gcg cct ttc gag cag tac tcc ccg ggt 2358
Asp Glu Tyr Leu Asp Leu Ser Ala Pro Phe Glu Gln Tyr Ser Pro Gly
        760                 765                 770

ggc cag gac acc ccc agc tcc agc tcc tca ggg gac gac tcc gtg ttt 2406
Gly Gln Asp Thr Pro Ser Ser Ser Ser Ser Gly Asp Asp Ser Val Phe
    775                 780                 785

gcc cac gac ctg ctg ccc ccg gcc cca ccc agc agt ggg ggc tcg cgg 2454
Ala His Asp Leu Leu Pro Pro Ala Pro Pro Ser Ser Gly Gly Ser Arg
790                 795                 800                 805

acg tga agggccactg gtccccaaca atgtgagggg tccctagcag ccctccctgc 2510

Thr

tgctggtgca cagccactcc ccggcatgag actcagtgca gatggagaga cagctacaca 2570

gagctttggt ctgtgtgtgt gtgtgtgcgt gtgtgtgtgt gtgtgcacat ccgcgtgtgc 2630

ctgtgtgcgt gcgcatcttg cctccaggtg cagaggtacc ctgggtgtcc ccgctgctgt 2690

gcaacggtct cctgactggt gctgcagcac cgaggggcct ttgttctggg gggacccagt 2750

gcagaatgta agtgggccca cccggtggga ccccgtgggg cagggagctg ggcccgacat 2810

ggctcggcct ctgcctttgc accacgggac atcacagggt gcgctcggcc cctcccacac 2870

ccaaagctga gcctgcaggg aagccccaca tgtccagcac cttgtgcctg gggtgttagt 2930

ggcaccgcct ccccacctcc aggctttccc acttcccacc ctgcccctca gagactgaaa 2990

ttacgggtac ctgaagatgg gagcctttac cttttatgca aaaggtttat tccggaaact 3050

agtgtacatt tctataaata gatgctgtgt atatggtata tatacatata tatatataac 3110

atatatggaa gaggaaaagg ctggtacaac ggaggcctgc gaccctgggg gcacaggagg 3170

caggcatggc cctgggcggg gcgtgggggg gcgtggaggg aggccccagg ggtctcaccc 3230

atgcaagcag aggaccaggg ctttttctgg caccgcagtt ttgttttaaa actggacctg 3290

tatatttgta aagctattta tgggcccctg gcactcttgt tcccacaccc caacacttcc 3350

agcatttagc tggccacatg gcggagagtt ttaattttta acttattgac aaccgagaag 3410

gtttatcccg ccgatagagg gacggccaag aatgtacgtc cagcctgccc cggagctgga 3470

ggatcccctc caagcctaaa aggttgttaa tagttggagg tgattccagt gaagatattt 3530

tatttgcttt gtcctttttc aggagaatta gatttctata ggatttttct ttaggagatt 3590

tattttttgg acttcaaagc aagctggtat tttcatacaa attcttctaa ttgctgtgtg 3650

tcccaggcag ggagacggtt tccagggagg ggccggccct gtgtgcaggt tccgatgtta 3710

ttagatgtta caagtttata tatatctata tatataattt attgagtttt tacaagatgt 3770

atttgttgta gacttaacac ttcttacgca atgcttctag agttttatag cctggactgc 3830

tacctttcaa agcttggagg gaagccgtga attcagttgg ttcgttctgt actgttactg 3890

ggccctgagt ctgggcagct gtcccttgct tgcctgcagg gccatggctc agggtggtct 3950

cttcttgggg cccagtgcat ggtggccaga ggtgtcaccc aaaccggcag gtgcgatttt 4010

gttaacccag cgacgaactt tccgaaaaat aaagacacct ggttgctaac ctgaaaaaaa 4070

aaaaaaaaaa aaaaaaaaaa aaa 4093

<210> 2
<211> 806

<212>  PRT
<213>  Homo sapiens

<400>  2

Met Gly Ala Pro Ala Cys Ala Leu Ala Leu Cys Val Ala Val Ala Ile
1               5                   10                  15

Val Ala Gly Ala Ser Ser Glu Ser Leu Gly Thr Glu Gln Arg Val Val
            20                  25                  30

Gly Arg Ala Ala Glu Val Pro Gly Pro Glu Pro Gly Gln Gln Glu Gln
        35                  40                  45

Leu Val Phe Gly Ser Gly Asp Ala Val Glu Leu Ser Cys Pro Pro Pro
    50                  55                  60

Gly Gly Gly Pro Met Gly Pro Thr Val Trp Val Lys Asp Gly Thr Gly
65                  70                  75                  80

Leu Val Pro Ser Glu Arg Val Leu Val Gly Pro Gln Arg Leu Gln Val
                85                  90                  95

Leu Asn Ala Ser His Glu Asp Ser Gly Ala Tyr Ser Cys Arg Gln Arg
            100                 105                 110

Leu Thr Gln Arg Val Leu Cys His Phe Ser Val Arg Val Thr Asp Ala
        115                 120                 125

Pro Ser Ser Gly Asp Asp Glu Asp Gly Glu Asp Glu Ala Glu Asp Thr
    130                 135                 140

Gly Val Asp Thr Gly Ala Pro Tyr Trp Thr Arg Pro Glu Arg Met Asp
145                 150                 155                 160

Lys Lys Leu Leu Ala Val Pro Ala Ala Asn Thr Val Arg Phe Arg Cys
                165                 170                 175

Pro Ala Ala Gly Asn Pro Thr Pro Ser Ile Ser Trp Leu Lys Asn Gly
            180                 185                 190

Arg Glu Phe Arg Gly Glu His Arg Ile Gly Gly Ile Lys Leu Arg His
        195                 200                 205

Gln Gln Trp Ser Leu Val Met Glu Ser Val Val Pro Ser Asp Arg Gly
    210                 215                 220

Asn Tyr Thr Cys Val Val Glu Asn Lys Phe Gly Ser Ile Arg Gln Thr
225                 230                 235                 240

Tyr Thr Leu Asp Val Leu Glu Arg Ser Pro His Arg Pro Ile Leu Gln
                245                 250                 255

Ala Gly Leu Pro Ala Asn Gln Thr Ala Val Leu Gly Ser Asp Val Glu
            260                 265                 270

Phe His Cys Lys Val Tyr Ser Asp Ala Gln Pro His Ile Gln Trp Leu
        275                 280                 285

Lys His Val Glu Val Asn Gly Ser Lys Val Gly Pro Asp Gly Thr Pro
    290                 295                 300

Tyr Val Thr Val Leu Lys Thr Ala Gly Ala Asn Thr Thr Asp Lys Glu
305                 310                 315                 320

Leu Glu Val Leu Ser Leu His Asn Val Thr Phe Glu Asp Ala Gly Glu
                325                 330                 335

Tyr Thr Cys Leu Ala Gly Asn Ser Ile Gly Phe Ser His His Ser Ala
            340                 345                 350

Trp Leu Val Val Leu Pro Ala Glu Glu Glu Leu Val Glu Ala Asp Glu
        355                 360                 365

Ala Gly Ser Val Tyr Ala Gly Ile Leu Ser Tyr Gly Val Gly Phe Phe
    370                 375                 380

Leu Phe Ile Leu Val Val Ala Ala Val Thr Leu Cys Arg Leu Arg Ser
385                 390                 395                 400

Pro Pro Lys Lys Gly Leu Gly Ser Pro Thr Val His Lys Ile Ser Arg
                405                 410                 415

Phe Pro Leu Lys Arg Gln Val Ser Leu Glu Ser Asn Ala Ser Met Ser
            420                 425                 430

Ser Asn Thr Pro Leu Val Arg Ile Ala Arg Leu Ser Ser Gly Glu Gly
        435                 440                 445

Pro Thr Leu Ala Asn Val Ser Glu Leu Glu Leu Pro Ala Asp Pro Lys
    450                 455                 460

Trp Glu Leu Ser Arg Ala Arg Leu Thr Leu Gly Lys Pro Leu Gly Glu
465                 470                 475                 480

Gly Cys Phe Gly Gln Val Val Met Ala Glu Ala Ile Gly Ile Asp Lys
                485                 490                 495

Asp Arg Ala Ala Lys Pro Val Thr Val Ala Val Lys Met Leu Lys Asp
            500                 505                 510

Asp Ala Thr Asp Lys Asp Leu Ser Asp Leu Val Ser Glu Met Glu Met
        515                 520                 525

Met Lys Met Ile Gly Lys His Lys Asn Ile Ile Asn Leu Leu Gly Ala
    530                 535                 540

Cys Thr Gln Gly Gly Pro Leu Tyr Val Leu Val Glu Tyr Ala Ala Lys
545                 550                 555                 560

Gly Asn Leu Arg Glu Phe Leu Arg Ala Arg Arg Pro Pro Gly Leu Asp
                565                 570                 575

Tyr Ser Phe Asp Thr Cys Lys Pro Pro Glu Glu Gln Leu Thr Phe Lys
            580                 585                 590

Asp Leu Val Ser Cys Ala Tyr Gln Val Ala Arg Gly Met Glu Tyr Leu
        595                 600                 605

Ala Ser Gln Lys Cys Ile His Arg Asp Leu Ala Ala Arg Asn Val Leu
    610                 615                 620

Val Thr Glu Asp Asn Val Met Lys Ile Ala Asp Phe Gly Leu Ala Arg
625                 630                 635                 640

Asp Val His Asn Leu Asp Tyr Tyr Lys Lys Thr Thr Asn Gly Arg Leu
                645                 650                 655

Pro Val Lys Trp Met Ala Pro Glu Ala Leu Phe Asp Arg Val Tyr Thr
            660                 665                 670

His Gln Ser Asp Val Trp Ser Phe Gly Val Leu Leu Trp Glu Ile Phe
        675                 680                 685

Thr Leu Gly Gly Ser Pro Tyr Pro Gly Ile Pro Val Glu Glu Leu Phe
    690                 695                 700

Lys Leu Leu Lys Glu Gly His Arg Met Asp Lys Pro Ala Asn Cys Thr
705                 710                 715                 720

His Asp Leu Tyr Met Ile Met Arg Glu Cys Trp His Ala Ala Pro Ser
                725                 730                 735

Gln Arg Pro Thr Phe Lys Gln Leu Val Glu Asp Leu Asp Arg Val Leu
            740                 745                 750

Thr Val Thr Ser Thr Asp Glu Tyr Leu Asp Leu Ser Ala Pro Phe Glu
        755                 760                 765

Gln Tyr Ser Pro Gly Gly Gln Asp Thr Pro Ser Ser Ser Ser Ser Gly
    770                 775                 780

Asp Asp Ser Val Phe Ala His Asp Leu Leu Pro Pro Ala Pro Pro Ser
785                 790                 795                 800

Ser Gly Gly Ser Arg Thr
                805

<210>  3
<211>  784
<212>  PRT
<213>  Homo sapiens

<400>  3

Glu Ser Leu Gly Thr Glu Gln Arg Val Val Gly Arg Ala Ala Glu Val
1               5                   10                  15

Pro Gly Pro Glu Pro Gly Gln Gln Glu Gln Leu Val Phe Gly Ser Gly
            20                  25                  30

Asp Ala Val Glu Leu Ser Cys Pro Pro Pro Gly Gly Gly Pro Met Gly
        35                  40                  45

Pro Thr Val Trp Val Lys Asp Gly Thr Gly Leu Val Pro Ser Glu Arg
    50                  55                  60

Val Leu Val Gly Pro Gln Arg Leu Gln Val Leu Asn Ala Ser His Glu
65                  70                  75                  80

Asp Ser Gly Ala Tyr Ser Cys Arg Gln Arg Leu Thr Gln Arg Val Leu
                85                  90                  95

Cys His Phe Ser Val Arg Val Thr Asp Ala Pro Ser Ser Gly Asp Asp
            100                 105                 110

Glu Asp Gly Glu Asp Glu Ala Glu Asp Thr Gly Val Asp Thr Gly Ala
        115                 120                 125

Pro Tyr Trp Thr Arg Pro Glu Arg Met Asp Lys Lys Leu Leu Ala Val
    130                 135                 140

Pro Ala Ala Asn Thr Val Arg Phe Arg Cys Pro Ala Ala Gly Asn Pro
145                 150                 155                 160

Thr Pro Ser Ile Ser Trp Leu Lys Asn Gly Arg Glu Phe Arg Gly Glu
                165                 170                 175

His Arg Ile Gly Gly Ile Lys Leu Arg His Gln Gln Trp Ser Leu Val
            180                 185                 190

Met Glu Ser Val Val Pro Ser Asp Arg Gly Asn Tyr Thr Cys Val Val
        195                 200                 205

Glu Asn Lys Phe Gly Ser Ile Arg Gln Thr Tyr Thr Leu Asp Val Leu
    210                 215                 220

Glu Arg Ser Pro His Arg Pro Ile Leu Gln Ala Gly Leu Pro Ala Asn
225                 230                 235                 240

Gln Thr Ala Val Leu Gly Ser Asp Val Glu Phe His Cys Lys Val Tyr
                245                 250                 255

Ser Asp Ala Gln Pro His Ile Gln Trp Leu Lys His Val Glu Val Asn
            260                 265                 270

Gly Ser Lys Val Gly Pro Asp Gly Thr Pro Tyr Val Thr Val Leu Lys
        275                 280                 285

Thr Ala Gly Ala Asn Thr Thr Asp Lys Glu Leu Glu Val Leu Ser Leu
    290                 295                 300

His Asn Val Thr Phe Glu Asp Ala Gly Glu Tyr Thr Cys Leu Ala Gly
305                 310                 315                 320

Asn Ser Ile Gly Phe Ser His His Ser Ala Trp Leu Val Val Leu Pro
                325                 330                 335

Ala Glu Glu Glu Leu Val Glu Ala Asp Glu Ala Gly Ser Val Tyr Ala
            340                 345                 350

Gly Ile Leu Ser Tyr Gly Val Gly Phe Phe Leu Phe Ile Leu Val Val
        355                 360                 365

Ala Ala Val Thr Leu Cys Arg Leu Arg Ser Pro Pro Lys Lys Gly Leu
    370                 375                 380

Gly Ser Pro Thr Val His Lys Ile Ser Arg Phe Pro Leu Lys Arg Gln
385                 390                 395                 400

Val Ser Leu Glu Ser Asn Ala Ser Met Ser Ser Asn Thr Pro Leu Val
                405                 410                 415

Arg Ile Ala Arg Leu Ser Ser Gly Glu Gly Pro Thr Leu Ala Asn Val
            420                 425                 430

Ser Glu Leu Glu Leu Pro Ala Asp Pro Lys Trp Glu Leu Ser Arg Ala
        435                 440                 445

Arg Leu Thr Leu Gly Lys Pro Leu Gly Glu Gly Cys Phe Gly Gln Val
    450                 455                 460

Val Met Ala Glu Ala Ile Gly Ile Asp Lys Asp Arg Ala Ala Lys Pro
465                 470                 475                 480

Val Thr Val Ala Val Lys Met Leu Lys Asp Asp Ala Thr Asp Lys Asp
                485                 490                 495

Leu Ser Asp Leu Val Ser Glu Met Glu Met Met Lys Met Ile Gly Lys
            500                 505                 510

His Lys Asn Ile Ile Asn Leu Leu Gly Ala Cys Thr Gln Gly Gly Pro
        515                 520                 525

Leu Tyr Val Leu Val Glu Tyr Ala Ala Lys Gly Asn Leu Arg Glu Phe
    530                 535                 540

Leu Arg Ala Arg Arg Pro Pro Gly Leu Asp Tyr Ser Phe Asp Thr Cys
545                 550                 555                 560

Lys Pro Pro Glu Glu Gln Leu Thr Phe Lys Asp Leu Val Ser Cys Ala
                565                 570                 575

Tyr Gln Val Ala Arg Gly Met Glu Tyr Leu Ala Ser Gln Lys Cys Ile
            580                 585                 590

His Arg Asp Leu Ala Ala Arg Asn Val Leu Val Thr Glu Asp Asn Val
        595                 600                 605

Met Lys Ile Ala Asp Phe Gly Leu Ala Arg Asp Val His Asn Leu Asp
    610                 615                 620

Tyr Tyr Lys Lys Thr Thr Asn Gly Arg Leu Pro Val Lys Trp Met Ala
625                 630                 635                 640

Pro Glu Ala Leu Phe Asp Arg Val Tyr Thr His Gln Ser Asp Val Trp
                645                 650                 655

Ser Phe Gly Val Leu Leu Trp Glu Ile Phe Thr Leu Gly Gly Ser Pro
            660                 665                 670

Tyr Pro Gly Ile Pro Val Glu Glu Leu Phe Lys Leu Leu Lys Glu Gly
        675                 680                 685

His Arg Met Asp Lys Pro Ala Asn Cys Thr His Asp Leu Tyr Met Ile
    690                 695                 700

Met Arg Glu Cys Trp His Ala Ala Pro Ser Gln Arg Pro Thr Phe Lys
705                 710                 715                 720

Gln Leu Val Glu Asp Leu Asp Arg Val Leu Thr Val Thr Ser Thr Asp
                725                 730                 735

Glu Tyr Leu Asp Leu Ser Ala Pro Phe Glu Gln Tyr Ser Pro Gly Gly
            740                 745                 750

Gln Asp Thr Pro Ser Ser Ser Ser Ser Gly Asp Asp Ser Val Phe Ala
        755                 760                 765

His Asp Leu Leu Pro Pro Ala Pro Pro Ser Ser Gly Gly Ser Arg Thr
    770                 775                 780